# EUROPEAN PATENT SPECIFICATION

(11) **EP 4 305 203 B1**
(45) Date of publication and mention of the grant of the patent: **14.01.2026**
(21) Application number: 22768125.1
(22) Date of filing: 11.03.2022
(51) Int. Cl.: C12Q 1/6874, C12Q 1/6869, C08F 20/56, C08F 220/56, C08F 222/38, C12Q 1/6813, C12Q 1/6841

(54) **HIGH-RESOLUTION SPATIAL TRANSCRIPTOME**
HOCHAUFLÖSENDES RÄUMLICHES TRANSKRIPTOM
TRANSCRIPTOME SPATIAL À HAUTE RÉSOLUTION

(30) Priority: 12.03.2021 US 202163160576 P
(43) Date of publication of application: 17.01.2024
(73) Proprietor: University of Washington, Seattle, Washington 98105-4721 (US)
(72) Inventor: GU, Liangcai, Seattle, Washington 98105-4721 (US); FU, Xiaonan, Seattle, Washington 98105-4721 (US); LIN, Shin, Seattle, Washington 98105-4721 (US); SUN, Li, Seattle, Washington 98105-4721 (US)
(74) Representative: Hobson, David James
(86) International application number: PCT/US2022/020028
(87) International publication number: WO 2022/192721

(56) References cited:
- WO-A1-2019/213254
- WO-A1-2020/076976
- US-A1- 2007 087 362
- US-A1- 2016 253 584
- US-A1- 2016 256 846
- US-A1- 2020 002 764
- AO CHEN: "Large field of view-spatially resolved transcriptomics at nanoscale resolution", BIORXIV, 24 January 2021 (2021-01-24), XP093159106, Retrieved from the Internet <URL:https://www.biorxiv.org/content/10.1101/2021.01.17.427004v2.full.pdf> DOI: 10.1101/2021.01.17.427004
- FU XIAONAN ET AL: "Polony gels enable amplifiable DNA stamping and spatial transcriptomics of chronic pain", vol. 185, no. 24, 1 November 2022 (2022-11-01), Amsterdam NL, pages 4621 - 4633.e17, XP093233792, ISSN: 0092-8674, Retrieved from the Internet <URL:https://pdf.sciencedirectassets.com/272196/1-s2.0-S0092867421X00256/1-s2.0-S0092867422013678/main.pdf?hash=df43dc8ee7233fe775aafe7d34d6d89408a4f489f341e127811a162744346861&host=68042c943591013ac2b2430a89b270f6af2c76d8dfd086a07176afe7c76c2c61&pii=S0092867422013678&tid=spdf-58e6c70d-745c-44d6-89c3-3d2> DOI: 10.1016/j.cell.2022.10.021
- FU XIAONAN, SUN LI, CHEN JANE Y., DONG RUNZE, LIN YIING, PALMITER RICHARD D., LIN SHIN, GU LIANGCAI: "Continuous Polony Gels for Tissue Mapping with High Resolution and RNA Capture Efficiency", BIORXIV, 17 March 2021 (2021-03-17), XP055983312, Retrieved from the Internet <URL:https://www.biorxiv.org/content/10.1101/2021.03.17.435795v1.full> [retrieved on 20221121], DOI: 10.1101/2021.03.17.435795

## Description

### REFERENCE TO SEQUENCE LISTING

The Sequence Listing associated with this application is provided in text format in lieu of a paper copy . The name of the text file containing the Sequence Listing is W149-0007PCT_ST25.txt. The text file is 5.25 KB, was created on March 11, 2022 and is being submitted electronically via EFS-Web.

### TECHNICAL FIELD

This application relates to systems, devices, and methods for generating high-resolution spatial transcriptomes with high ribonucleic acid (RNA) capture efficiency.

### BACKGROUND

The spatial organization and dynamics of cell-specific gene expression in tissues are fundamental to the understanding of anatomy, physiology, and pathology. In situ capture of spatially resolved omics data of individual cells is important for understanding the structure and function of heterogeneous tissues such as the brain.

Preceded by dissociative single-cell RNA sequencing (scRNA-seq) (Tang, F. C. et al., Nat. Methods 6, 377-82 (2009); Ramskold, D. et al., Nat. Biotechnol. 30, 777-82 (2012); Jaitin, D. A. et al., Science 343, 776-79 (2014); Macosko, E. Z. et al., Cell 161, 1202-1214 (2015)), technologies enabling global, single-cell gene expression profiling in morphologically intact tissues would further reveal the heterogeneity of cellular compositions, functional dynamics, and responses to diseases and drugs. Spatially resolved transcriptome profiling has been achieved by imaging-based in situ RNA hybridization (see, e.g., E. Lubeck et al., Nat. Methods 9, 743-48 (2012); K. H. Chen et al., Science 348, aaa6090 (2015); S. Shah et al., Neuron 92, 42-357 (2016)) and sequencing (R. Ke et al., Nat. Methods 10, 857-60 (2013); J. H. Lee et al., Science 343, 1360-63 (2014)). Current technologies for acquiring spatial transcript information from tissue sections rely on either RNA probes (e.g., MERFISH (Chen, K. H., et al., Science 348, aaa6090 (2015)), seqFISH (Shah, S., et al., Neuron 92, 342-57 (2016)), and FISSEQ (Lee, J. H. et al., Science 343, 1360-63 (2014))) or spatial barcodes (e.g., Spatial Transcriptomics (Stahl, P. L. et al., Science 353, 78-82 (2016); Vickovic, S. et al., Nat. Methods 16, 987-90 (2019)), Slide-seq (Rodriques, S. G. et al., Science 363, 1463-67 (2019); Stickels, R. R. et al., Nat. Biotechnol. 39, 313-19 (2021)), and DBiT-seq (Liu, Y. et al., Cell 183, 1665-81 (2020))). The former methods typically require a priori knowledge for probeset formulation; the latter have yet to achieve a single-cell resolution and/or transcript capture efficiencies approaching the dissociative methods.

To map single-cell transcriptomes, a two-step approach was to perform dissociative single-cell RNA sequencing (scRNA-seq) and then probe-based imaging of cells in a tissue context (Moffitt, J. R. et al., Science 362, aau5324 (2018)). Although it has been validated on some tissue types, the data from two assays were collected with different samples and rare cell types or functional states tend to be lost during tissue dissociation.

Spatially barcoded RNA sequencing technologies have rapidly advanced by enhancing the resolution and sensitivity of transcript detection. Previous methods use oligonucleotide arrayindexed (or spatially barcoded) RNA sequencing (P. L. Stahl et al., Science 353, 78-82 (2016); S. G. Rodriques et al., Science 363, 1463-67 (2019); S. Vickovic et al., Nature Methods 16, 987-90 (2019); Y. Liu et al., Cell 183, 1665-81 (2020)). They employ arrayed oligos with spatially resolved indices (e.g., spotted (Stahl, P. L. et al., Science 353, 78-82 (2016)), self-assembled bead (Vickovic, S. et al., Nat. Methods 16, 987-90 (2019); Rodriques, S. G. et al., Science 363, 1463-67 (2019)), and in situ synthesized (Liu, Y. et al., Cell 183, 1665-81 (2020)) arrays) as primers which are incorporated into in situ synthesized complementary DNAs (cDNAs) derived from a tissue section placed atop the array. Subsequent sequencing of the indexed cDNAs reveals the transcripts and their spatial locations. Among various oligo arrays, those formed with polymerase colonies (known as "polonies" (Mitra, R. D. & Church, G. M., Nucleic Acids Res. 27, e34-e39 (1999); Gu, L. et al., Nature 30 515, 554-57 (2014)) or "DNA clusters" (Bentley, D. R. et al., Nature 456, 53-59 (2008))) and rolling circle colonies ("rolonies" or "DNA nanoballs" (Drmanac, R. et al., Science 327, 78-81 (2010))) with diameters of ≤ 1 µm offer a sufficient resolution to delineate cell boundaries to segregate mapped transcripts into individual cells. Recently, such arrays formed in commercial sequencing flowcells were repurposed for tissue mapping to demonstrate improved resolutions (Chen, A. et al. Large field of view-spatially resolved transcriptomics at nanoscale resolution, Preprint at https://www.biorxiv.org/content/10.1101/2021.01.17.427004v2 (2021); Cho, C. S. et al., Cell 184, 3559-72 (2021)). However, the reported data were mainly aggregated in regular bins, and the feature resolution was not translated to single-cell resolution. Heretofore, simple, robust, tissue dissociation-independent scRNA-seq has not been demonstrated for demanding applications such as brain mapping.

### SUMMARY OF INVENTION

The present invention is defined by the claims.

Accordingly, in one aspect the invention provides a method for identifying the location and type of single cells within a tissue sample, the method comprising:
generating a crosslinked polyacrylamide (PAA) gel comprising 3-20% acrylamide/ bis, 0.01-1% ammonium persulfate, and 0.01-1% N,N,N',N'-tetramethylethylenediamine (TEMED);
generating a polony gel by generating a first polony and a second polony on a surface of the crosslinked PAA gel, the first polony comprising a first template, the second polony comprising a second template, the first polony bordering the second polony on the surface of the crosslinked PAA gel;
generating a polony map by sequencing the first template in the first polony and sequencing the second template in the second polony;
capturing, by the first template and the second template, RNA from a cell of a tissue sample, the cell overlapping the first polony and the second polony;
generating cDNA based on the captured RNA, the first template, and the second template;
generating a sequencing library by sequencing the cDNA; and
identifying the location of the cell on the polony gel by comparing the sequencing library to the polony map.

In another aspect, the invention provides a polony gel for single cell RNA sequencing, the polony gel comprising:
a hydrogel comprising crosslinked PAA;
a first polony on a surface of the hydrogel, the first polony comprising a first template that comprises a first capture sequence and a first index sequence; and
a second polony on the surface of the hydrogel, the second polony bordering the first polony and comprising a second template that comprises a second capture sequence and a second index sequence.

### BRIEF DESCRIPTION OF THE DRAWINGS

Some of the drawings submitted herewith may be better understood in color. Applicant considers the color versions of the drawings as part of the original submission and reserves the right to present color images of the drawings in later proceedings.

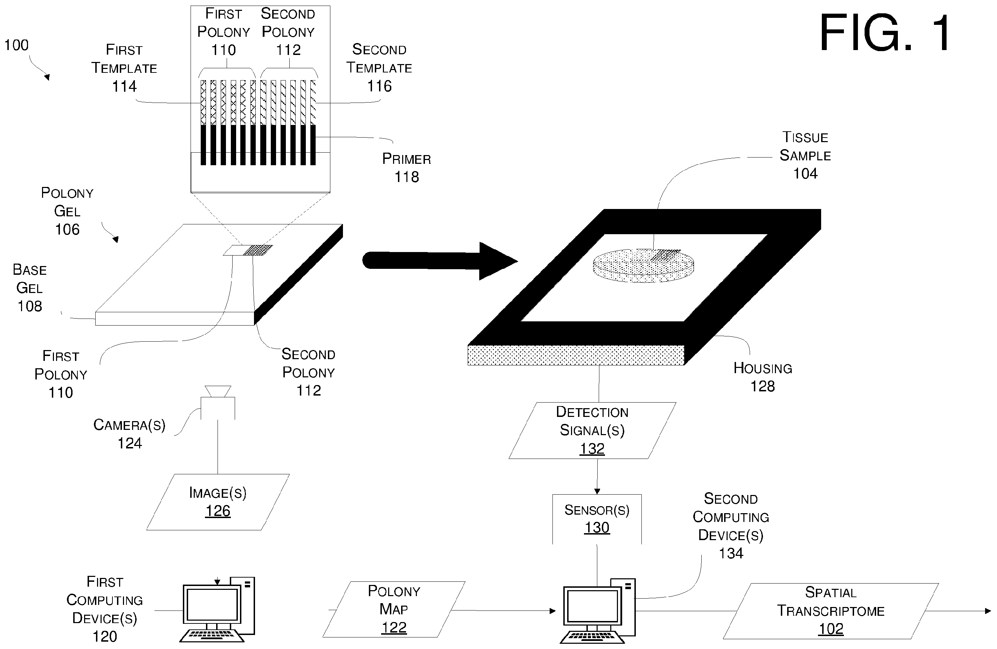

FIG. 1 illustrates an example system for generating a spatial transcriptome of a tissue sample using a polony gel.

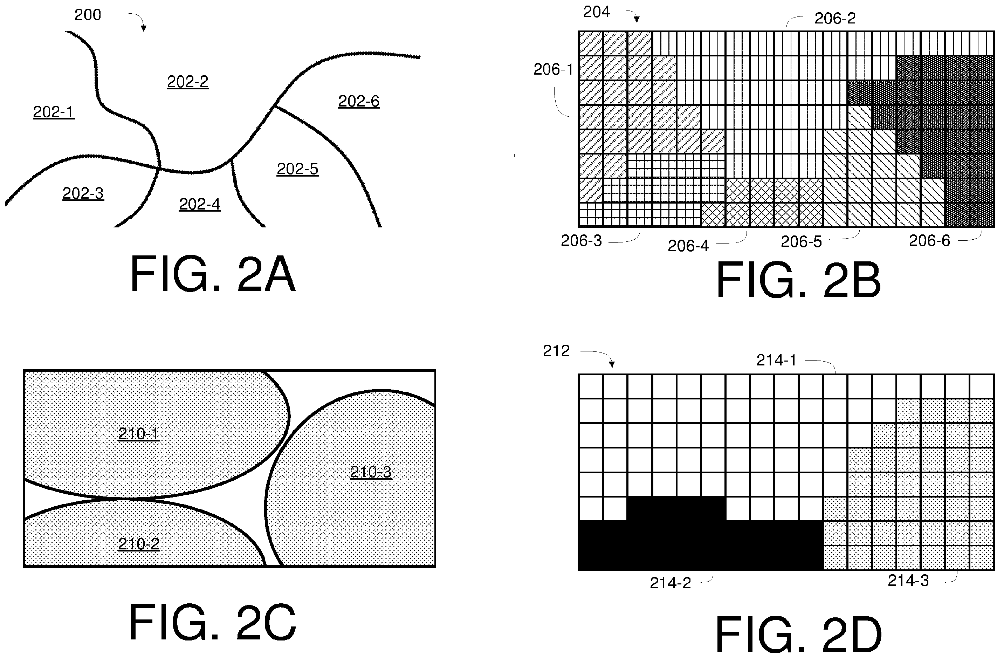

FIGS. 2A to 2D illustrate example techniques for generating a spatial transcriptome using polonies. FIG. 2A illustrates an example of polonies of a polony gel. FIG. 2B illustrates an example polony map representing the polony gel. FIG. 2C illustrates an image of a tissue sample that includes a first cell, a second cell, and a third cell. FIG. 2D illustrates an example of a spatial transcriptome of the tissue sample illustrated in FIG. 2C.

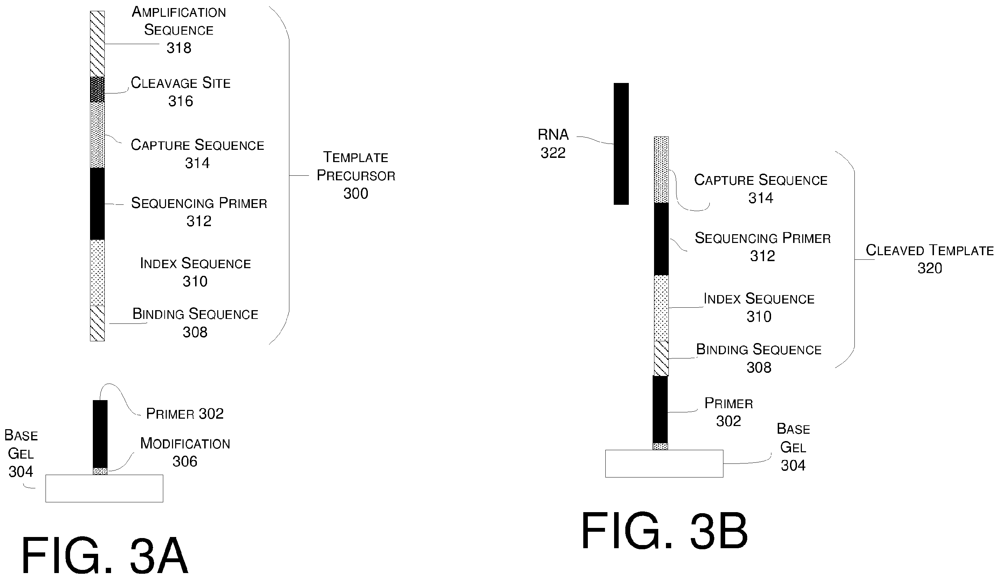

FIGS. 3A to 3B illustrate examples of various constituents used to generate polony gels and to identify spatial transcriptomes. FIG. 3A illustrates a template precursor to be attached to a primer during polony gel fabrication. FIG. 3B illustrates an example of a cleaved template that captures an RNA.

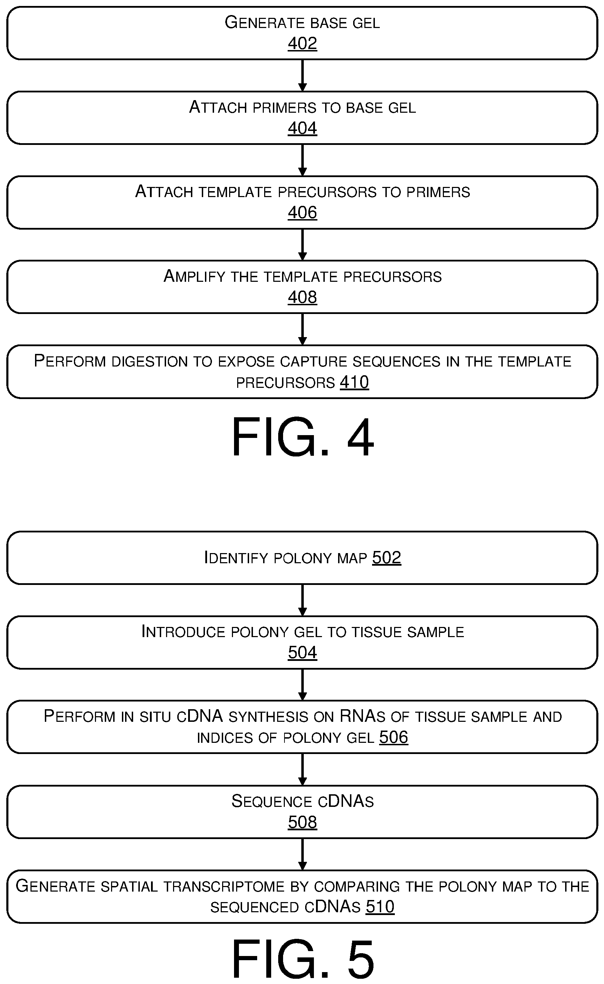

FIG. 4 illustrates an example process for synthesizing a polony gel.
FIG. 5 illustrates an example process for generating a spatial transcriptome using a polony gel.

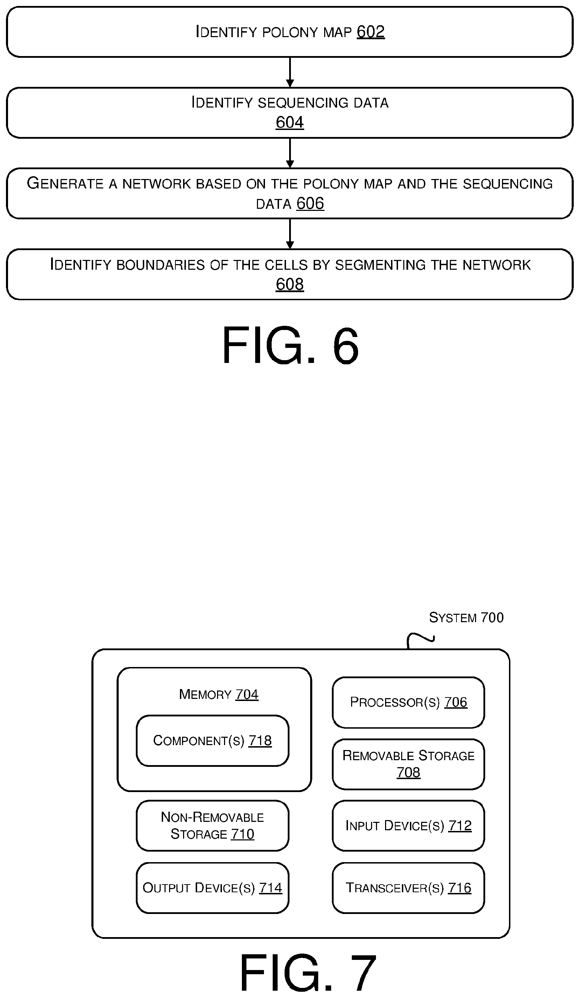

FIG. 6 illustrates an example process for segmenting cells based on sequencing data representing mRNA from the cells bound to templates of a polony gel.
FIG. 7 illustrates an example of a system for performing various functions described herein.

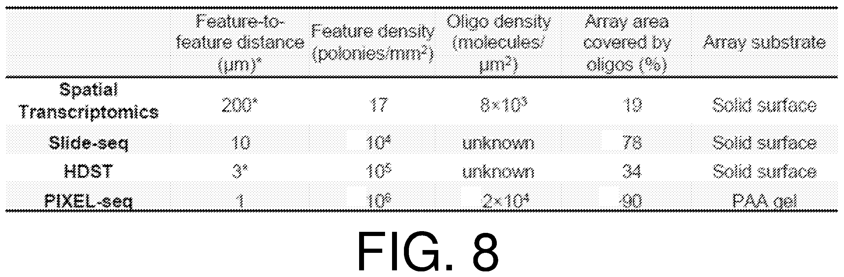

FIG. 8 illustrates a table comparing example spatial transcriptomics techniques described herein to other reported methods.

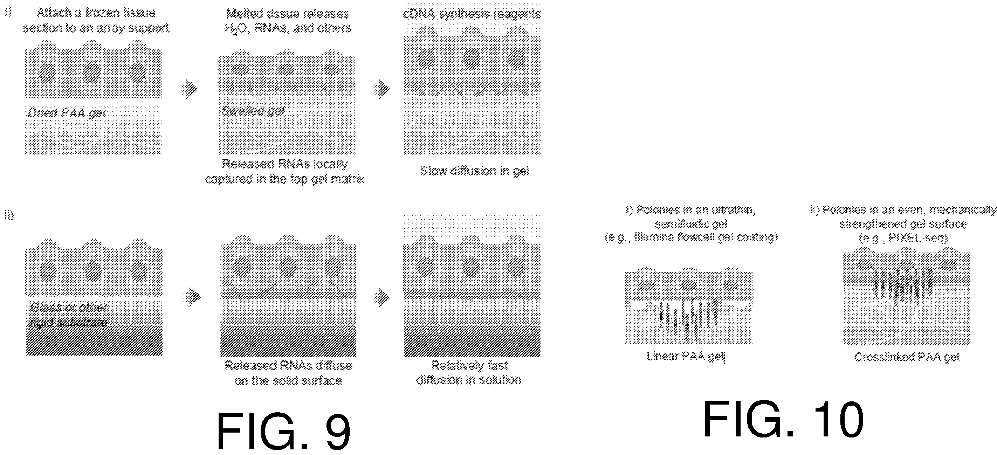

FIG. 9 illustrates an example of a benefit of crosslinked PAA-gel on in-situ cDNA synthesis.
FIG. 10 illustrates an example structural difference of polonies on a linear PAA-gel and acrosslinked PAA-gel.

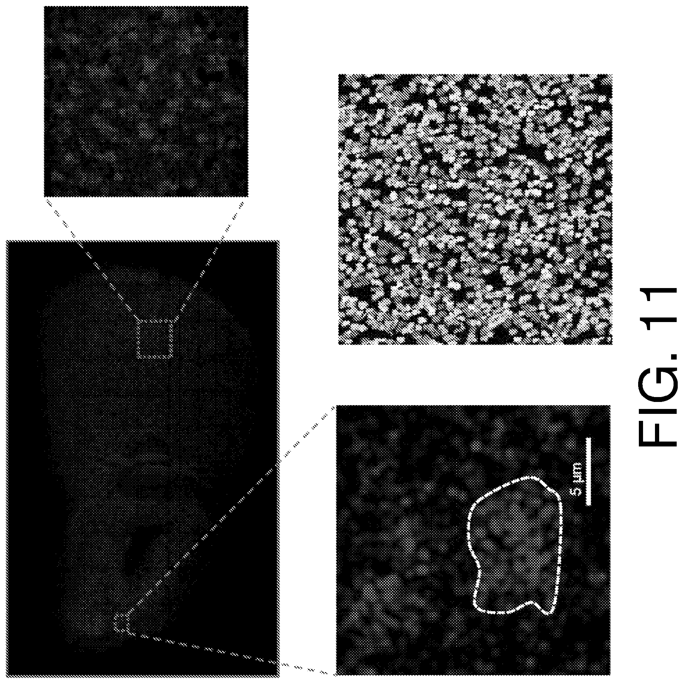

FIG. 11 illustrates an example of a spatial distribution of cDNA read density per polony (1 pixel = 0.325 * 0.325 um). FIG. 11 also illustrates a close visualization of cDNA density distribution showing a subcellular resolution (right panel).

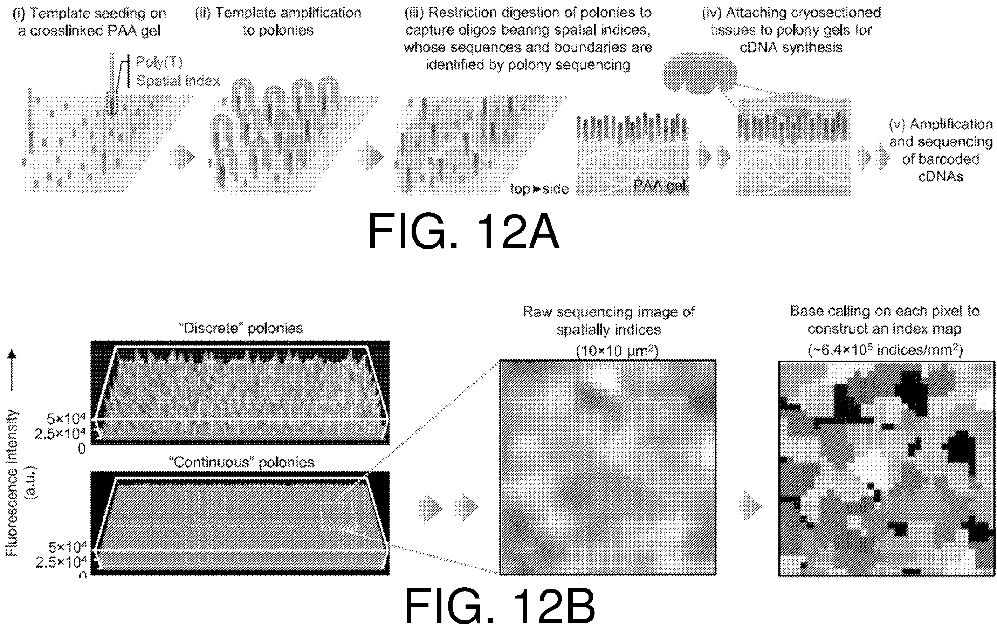

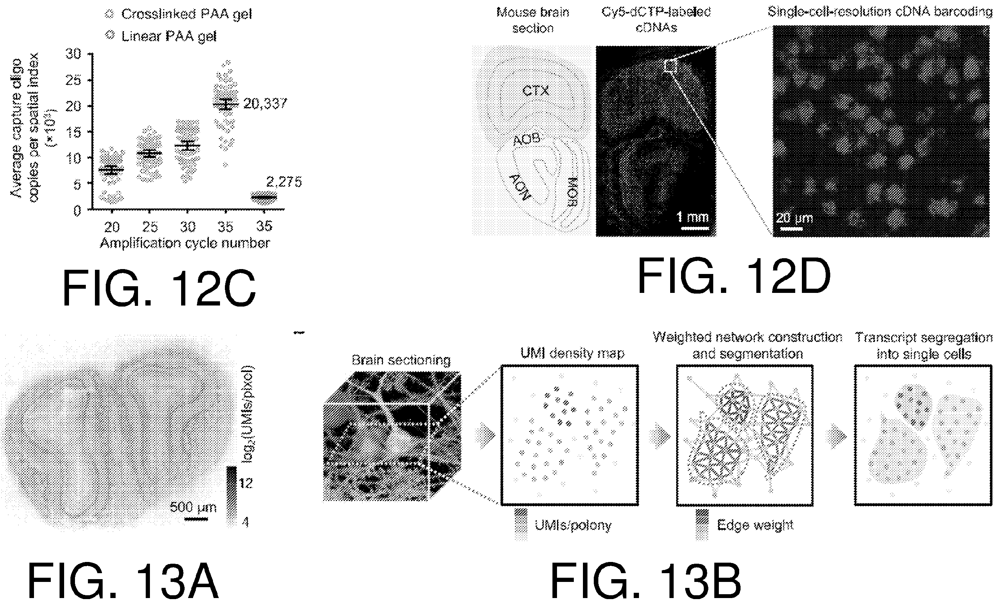

FIGS. 12A to 12D illustrate principles of example spatial transcriptomics techniques. FIG. 12A illustrates a schematic of polony-based spatial barcoding of tissue transcripts. 370-bp DNA templates bearing 24-bp random spatial indices, a 20-bp poly-T probe, and two Taql restriction sites are amplified to polonies on a PAA gel surface. Spatial indices and their coordinates in the gel are determined by polony sequencing. A cryosectioned tissue is placed onto the gel surface for transcript capturing and cDNA synthesis. Barcoded cDNAs are amplified for sequencing (e.g. next generation sequences (NGS)) to associate transcripts to their gel locations. FIG. 12B illustrates 3D intensity profiles of SYBR Green-stained discrete and continuous polonies amplified from templates at the same density by 35 cycles (left). A merged four-color sequencing image (middle) was converted to a spatial index map by pixel-level base calling (right). FIG. 12C illustrates a comparison of polony amplification with different PAA gel structures. Oligo copies were estimated by real-time image quantification using DNA-coated beads as standards. The gel was a linear PAA gel. FIG. 12D illustrates Cy5-labeling of cDNAs synthesized in a mouse olfactory bulb-isocortex section showing single-cell resolution. AON, anterior olfactory nucleus; AOB, accessory olfactory bulb; MOB: main olfactory bulb; CTX, cerebral cortex.

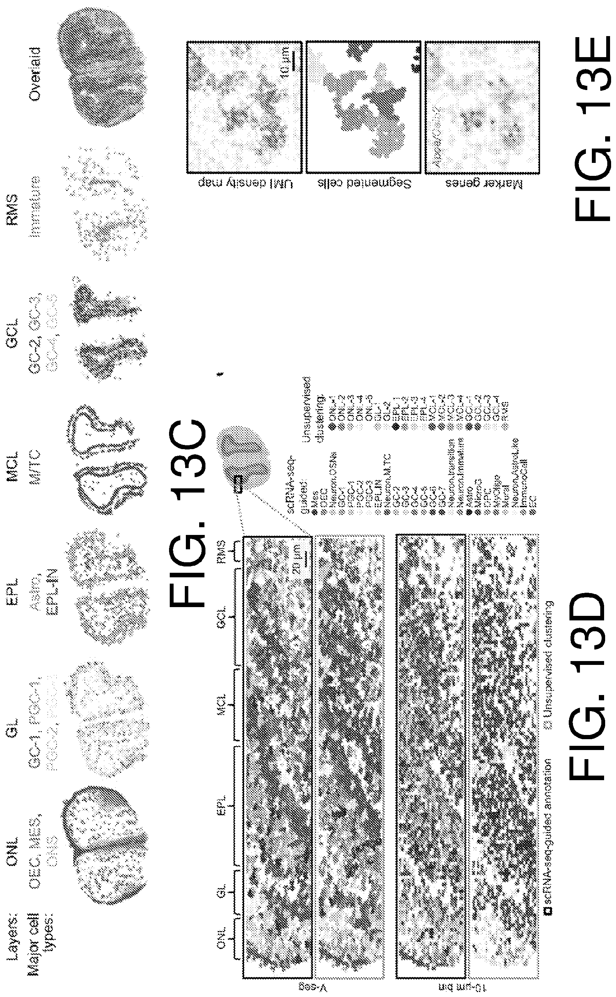

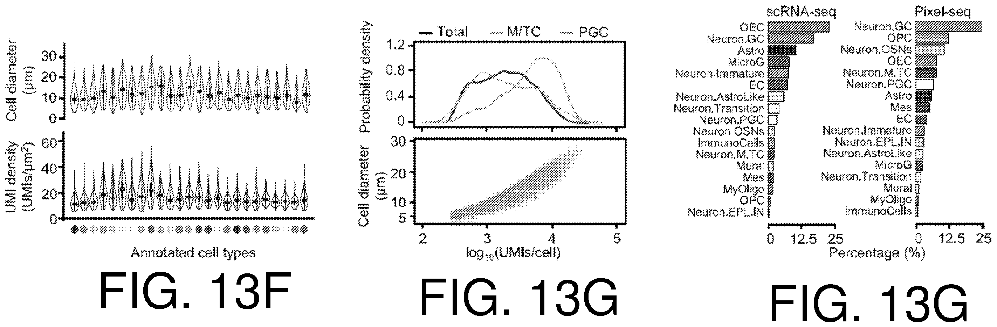

FIG. 13A to 13H illustrate non-dissociative scRNA-seq of the MOB. FIG. 13A shows a representative UMI density map of a coronal MOB section. UMIs were counted on each image pixel (0.325 × 0.325 µm2). FIG. 13B illustrates the principle of V-seg. A k-nearest neighbor network is built on a spatial index map of transcripts where an index represents a node. Edge weights are assigned as a function of the UMI counts, the distance and the transcript similarity between two connected indices. The graph-based segmentation of the network aggregates transcripts into single cells. FIG. 13C shows spatial localization of annotated cell types in the major MOB layers. ONL, olfactory neuron layer; GL, glomerular layer; EPL, external plexiform layer; GCL, granule cell layer; MCL, mitral and tufted cell layers; RMS, rostral migratory stream. FIG. 13D illustrates a comparison of cell annotation outcomes of transcript segmentation by V-seg and a regular bin of 10 × 10 µm². Aggregated transcripts were analyzed by scRNA-seq-guided annotation or unsupervised clustering. FIG. 13E shows a comparison of the UMI density map as input (top), the V-seg outcome (middle), and the marker gene locations (bottom) for validation. FIG. 13F shows plots of cell diameters (top) and UMI densities (bottom) of annotated cell types. Irregular cells were converted to round shapes with corresponding sizes to calculate diameters. Data for 5,565 to 135 cells for each type were plotted. FIG. 13G illustrates a correlation between the cell size and UMI counts per cell. Data for 248 PGC, 1,723 M/TC, and 22,830 total cells were plotted. FIG. 13H shows a comparison of relative abundances of MOB cell types detected by scRNA-seq and example spatial transcriptomics techniques.

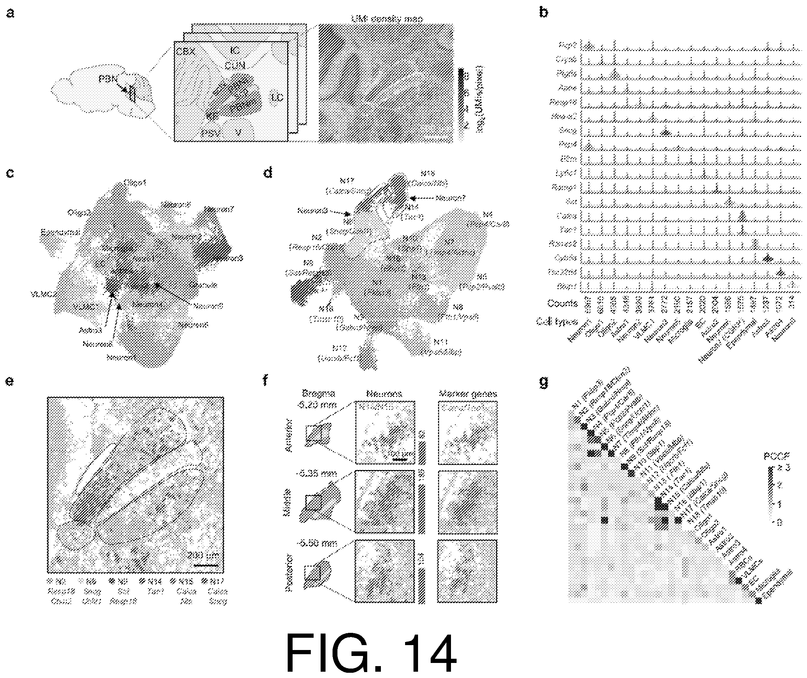

FIGS. 14A to 14G show examples of spatial, single-cell transcriptome mapping of the PBN. FIG. 14A shows an anatomical structure and UMI density map of a middle coronal section comprising the PBN (red) and neighboring regions. CBX, cerebellar cortex; IC, inferior colliculus; CUN, cuneiform nucleus; sctv, ventral spinocerebellar tract; scp, superior cerebellar peduncle; KF, Kölliker-Fuse subnucleus; PBNI, lateral PBN; PBNm, medial PBN; PSV, principal trigeminal sensory nucleus; V, trigeminal motor nucleus; LC, locus ceruleus. FIG. 14B shows violin plots of selected top genes showing differential expression in each cluster. FIG. 14C illustrates uniform Manifold Approximation and Projection (UMAP) embedding of example spatial transcriptomics techniques data of ~60,000 cells passing quality control metrics from four middle PBN sections. Astro, astrocyte; Oligo, oligodendrocyte; EC, endothelial cell; VLMC, vascular and leptomeningeal cell. FIG. 14D shows UMAP embedding of the data of 31,505 neuronal cells isolated from FIG. 14c with defined marker gene(s) for each cluster. Selected subclusters separated from corresponding clusters in FIG. 14C are highlighted by dotted lines. FIG. 14E illustrates a spatial distribution of major neuronal subtypes in the PBN and V. FIG. 14F illustrates 3D mapping of PBN Tac1 and Calca neurons. Stacked bars denote cell counts. FIG. 14G illustrates a cell-cell contact heatmap of found clusters in FIGS. 14C and 14D. Cell contacts were quantified by a PCCF colocalization statistic.

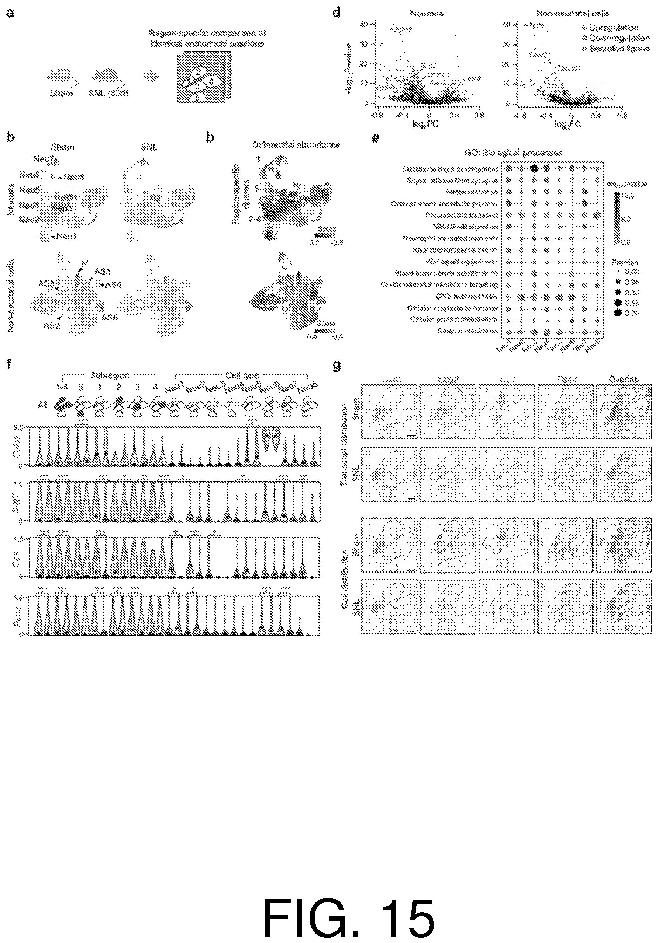

FIGS. 15A to 15G show an example of chronic pain-regulated gene expression in the PBN subnuclei. FIG. 15A shows a comparison of the transcriptome data of sham and pain mice at anatomically identical PBN subregions. SNL (30d) 30 days post partial sciatic nerve ligation. FIG. 15B illustrates UMAP analysis to compare clusters in two middle coronal PBN sections from sham and SNL mice. 15,833 neurons and 16,473 non-neuronal cells are plotted. Major neurons, astrocytes, and microglia in the PBN and V are labelled. P-value, Kolmogorov-Smirnov (KS) test. FIG. 15C shows a differential abundance analysis of the data in FIG. 15B. PBN and V region-specific neuronal clusters are highlighted by dotted lines. d, Differential gene expression analysis of cells in FIG. 15B. FC, fold change. Colored genes, |log2FC| ≥ 0.25 and P < 0.05, Wilcoxon rank-sum test. FIG. 15E illustrates GO enrichment analysis of biological processes for major PBN and V neuronal clusters. P-values were calculated by a Fisher's exact test. FIG. 15F illustrates region- and cell-type-specific differential expression of selected neuropeptide precursors. Data represent mean values of ≥ 217 cells; error bars, standard error of mean. *P < 0.05, **P < 0.01, ***P < 0.001, Wilcoxon rank-sum test. FIG. 15G shows a spatial differential expression of the neuropeptide precursors in f and the distribution of corresponding cells. Scale bar, 100 µm.

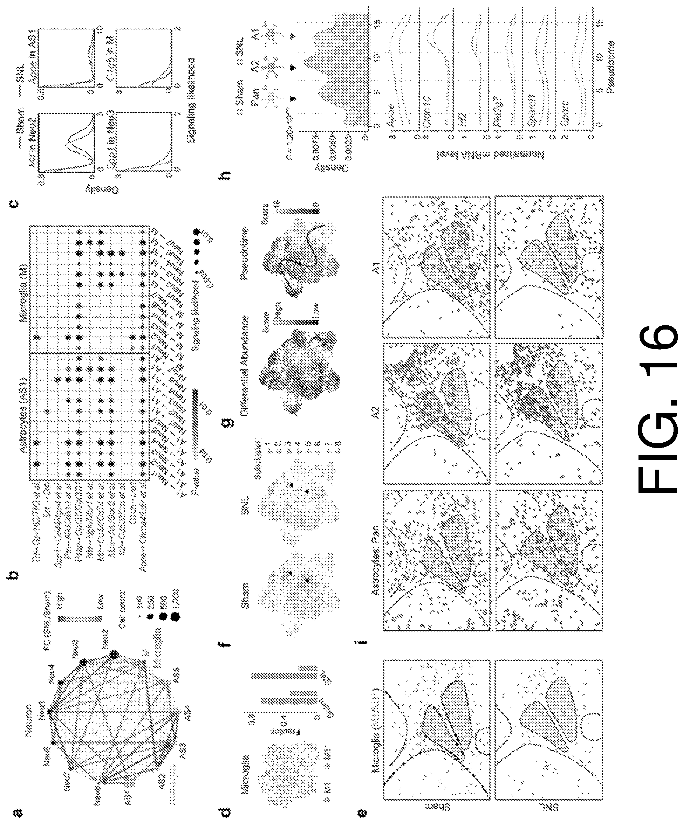

FIGS. 16A to 16I show an example of chronic pain-associated cell-cell communication changes and glial transcriptome dynamics. FIG. 16A provides a network representation of cell signaling changes for the neuronal, astrocyte and microglial clusters with significant distributions in the PBN. Signaling likelihoods were calculated by SpaOTsc (Zhao, J. et al., Proc. Natl. Acad. Sci. U. S. A. 118, e2100293118 (2021)). FIG. 16B is a dot plot showing contributions of paired ligand and receptor genes to the changed signaling in the most abundant astrocyte subtype, AS1, and microglia. P-value, KS test. FIG. 16C shows a density distribution of signaling likelihoods of selected ligand genes in sender clusters. FIG. 16D shows a UMAP plot showing re-clustered microglia and the subtype proportions under the sham and pain conditions. FIG. 16E illustrates a spatial distribution of microglial subclusters in the PBN and neighboring regions. 584 cells are plotted. FIG. 16F illustrates a UMAP plot of re-clustered astrocytes. 4,471 cells are plotted. The subclusters 2 and 3 are denoted by triangles. FIG. 16G illustrates a differential abundance (left) and pseudotime (right) analyses of the data in FIG. 15F. A pseudotime trajectory was inferred by Slingshot. FIG. 16h illustrates a density distribution of astrocytes and normalized expression of selected genes along the pseudotime trajectory in g. P- value, KS test. FIG. 16i shows a spatial distribution of astrocyte subclusters in the PBN.

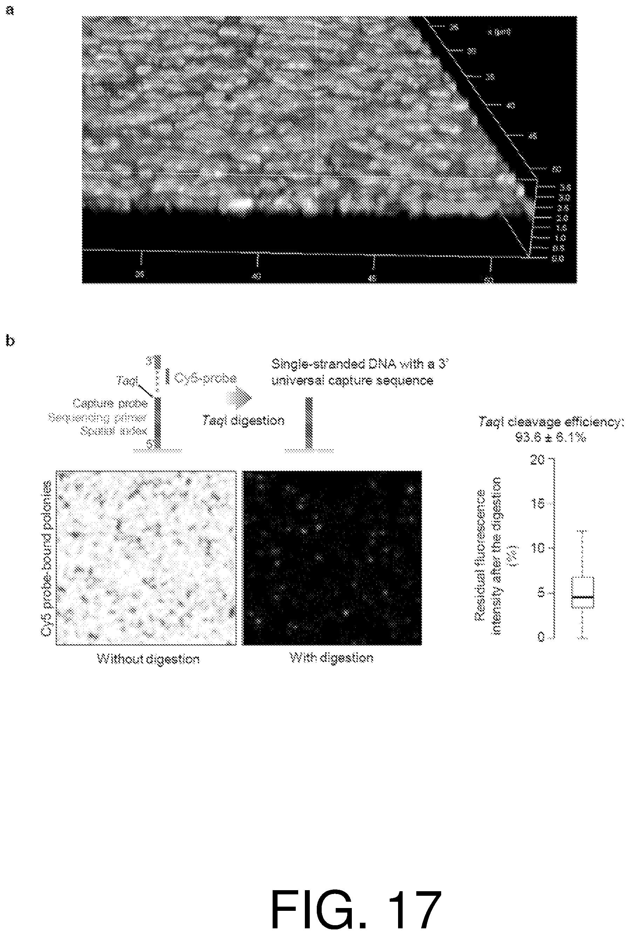

FIG. 17A shows a depth distribution of continuous polony DNAs in a PAA gel. Fluorescent sequencing signals were Z-axis scanned by confocal fluorescence microscopy; most of the fluorescence signals were found at the gel surface in a 400-nm thickness. FIG. 17B shows an analysis of the cleavage efficiency of polony double-stranded DNAs (dsDNAs) by restriction digestion. (Left) dsDNAs in a polony gel were before and after treated with Taql and non-cleaved DNAs were detected by hybridization to a Cy5-labelled oligo. (Right) Taql cleavage efficiency was quantified by comparing fluorescence signals for eight field-of-views (FOVs; 1,024 × 1,024 pixels, 1 pixel = 0.325 × 0.325 µm²).

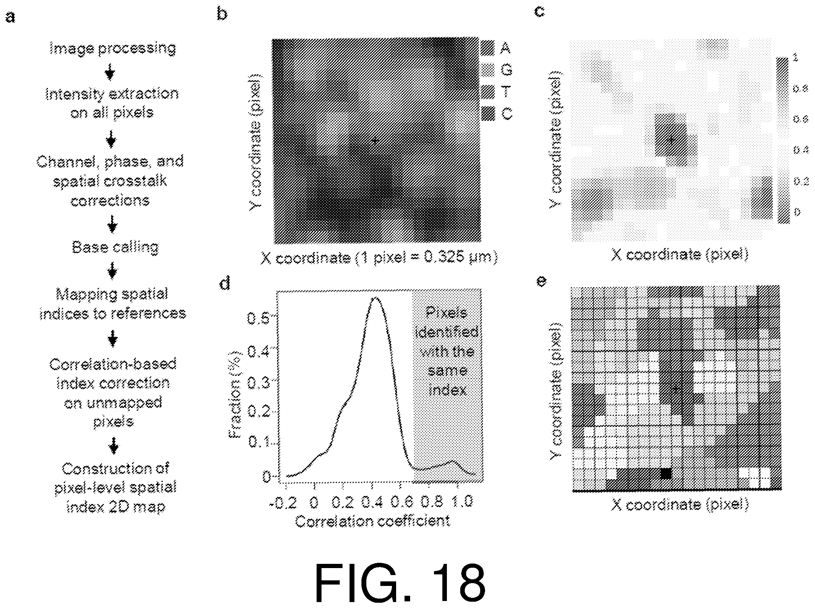

FIGS. 18A to 18E illustrate an example construction of a spatial index map at an image pixel level. FIG. 18A shows a pipeline of the index map construction. FIG. 18B shows a representative merged raw image of the four-channel polony sequencing. FIG. 18C shows a spatial heatmap of the correlation coefficient of extracted intensities from all sequencing channels and cycles between the center pixel and other neighboring pixels in the image. FIG. 18D shows a density plot of the correlation coefficient for all pixels in FIG. 18C. A cutoff of 0.7 was used to group pixels with an identical index. FIG. 18E shows a final constructed index map.

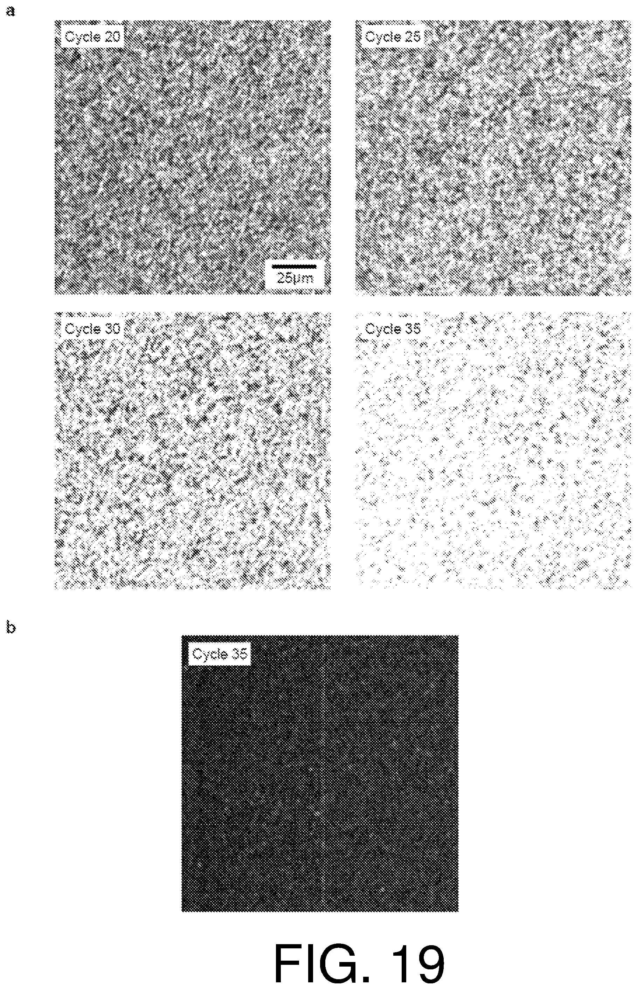

FIG. 19A shows a representative image of polonies amplified in a crosslinked PAA gel. FIG. 19B shows a representative image of polonies amplified in a linear PAA gel. The linear PAA gel was fabricated following a method reported in Bentley, D. R. et al., Nature 456, 53-59 (2008). DNA templates of the same density on different gel surfaces were amplified with the indicated cycle numbers before staining polonies by SYBR Green. All images were acquired with the same imaging setting. Total fluorescence signals subtracted by the background are compared in FIG. 17c.

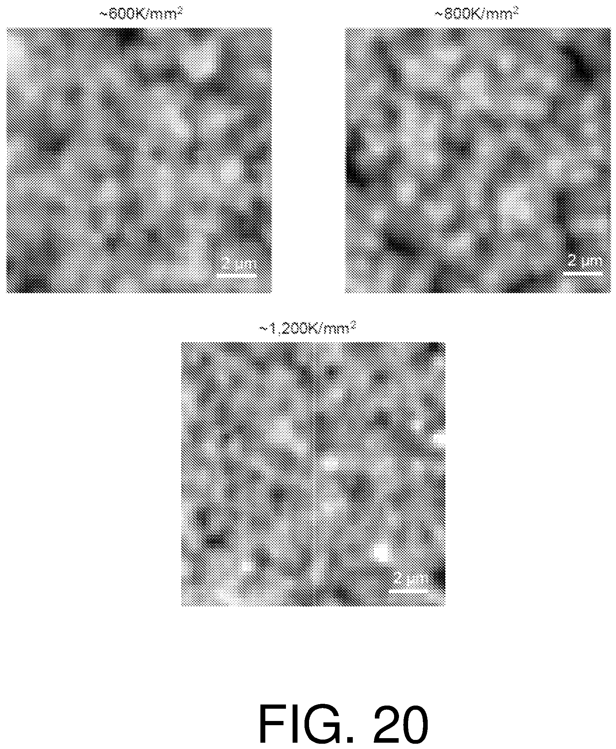

FIG. 20 illustrates raw sequencing images showing increased polony densities and decreased sizes. Polonies were grown at different template densities with 35 amplification cycles. Polonies densities were estimated by template seeding concentrations and polony sizes. Due to a polony exclusion effect (described in Aach, J. & Church, G. M., J. Theor. Biol. 228, 31-46 (2004)), polonies arising from adjacent DNA templates often cannot interpenetrate, resulting in clear boundaries. A higher polony density leads to smaller polony sizes. At the polony density of 1,200K/mm², the average polony size is 0.5 µm. Many dark regions in the images were associated with polonies with no sequencing signals because their spatial indices were cleaved by Taql.

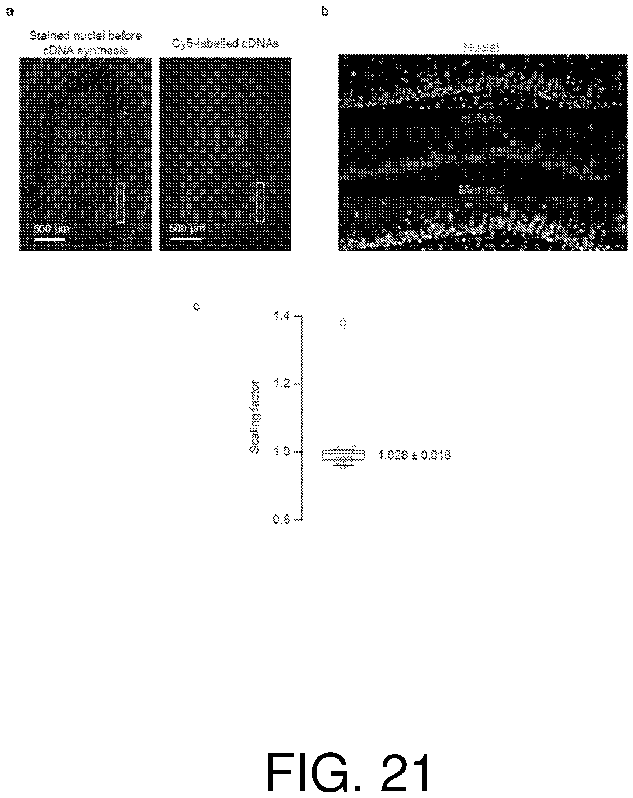

FIGS. 21A to 21C illustrate an analysis of lateral template diffusion during transcript capturing on polony gels. FIG. 21A illustrates images of a 10-µm-thick SYTOX Green-stained, non-fixed mouse MOB section on a dry polony gel pre-soaked with the dye (left) and Cy5-dCTP labelled cDNAs after cDNA synthesis (right). FIG. 21B shows a zoom-in visualization of registered nucleus and cDNA images. Nucleus signals are significantly more than cDNA signals because the former are from a whole tissue depth, but the latter were only generated on the exposed tissue cutting surface. FIG. 21C illustrates a scaling factor, an indicator of the lateral diffusion, which was calculated for 10 randomly selected positions in the images.

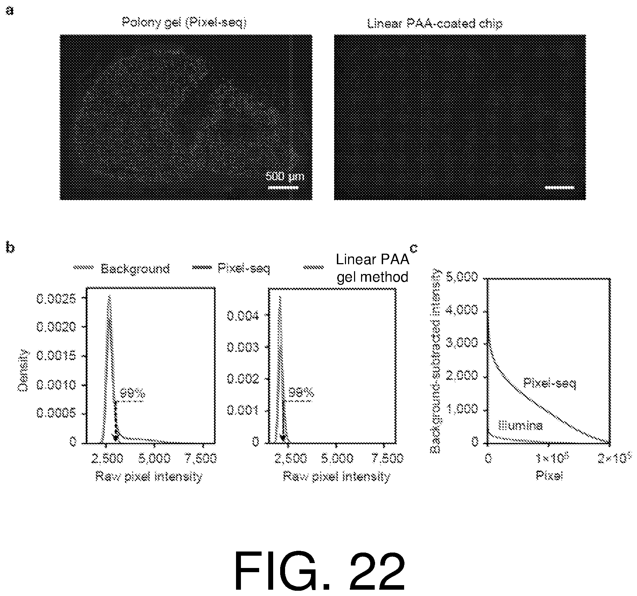

FIGS. 22A to 22C show a comparison of cDNA yields on a crosslinked polony gel and a linear PAA gel. FIG. 22A shows images of Cy5-dCTP-labelled cDNAs in mouse olfactory bulb-isocortex sections placed on a crosslinked and a linear PAA gels. The linear PAA gel was fabricated following a method reported in Bentley, D. R. et al., Nature 456, 53-59 (2008). Two adjacent brain sections were attached to the gels and then assayed at the same condition. Quantitative comparison of the Cy5 signals (FIG. 22B) before and (FIG. 22C) after background signal subtraction. The subtracted Cy5 signal of barcoded cDNAs in cell bodies, represented by the top 1% of pixels in the polony gel, is 17.58-fold higher than that on the linear PAA gel.

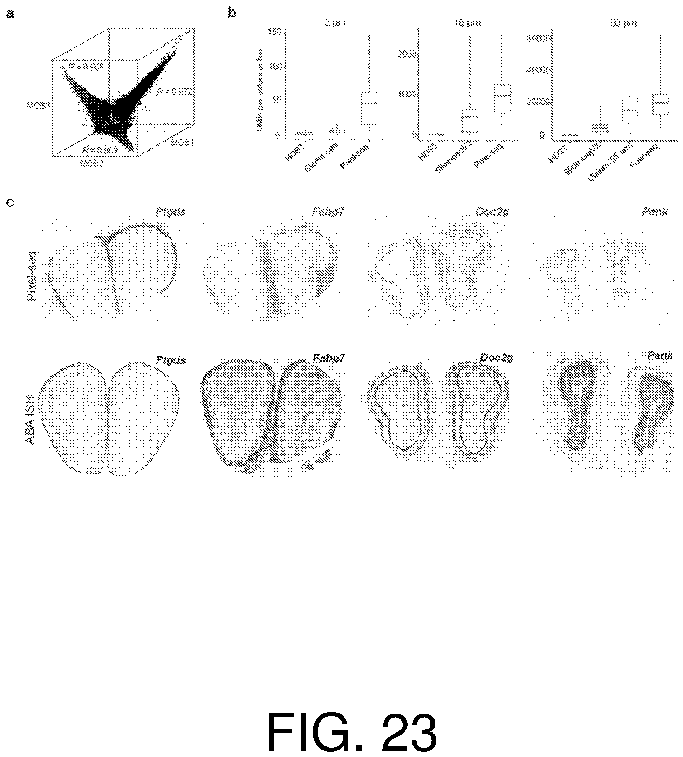

FIG. 23A shows a comparison of three example spatial transcriptomics techniques datasets on the mouse MOB. The Pearson correlation coefficients (Rs) between two of the three datasets were calculated. FIG. 23B shows a comparison of transcript capture efficiencies and resolution of example spatial transcriptomics techniques and other spatial barcoding methods using their most recent MOB datasets. Boxplots show total UMIs captured by example spatial transcriptomics techniques and the other methods at their specific feature resolutions. For example spatial transcriptomics techniques, UMIs/feature was calculated by grouping UMIs on regular bins of 7 × 7 (2 µm), 33 × 33 (10 µm), or 154 × 154 (50 µm) pixels (1 pixel = 0.325 µm). Feature-to-feature gaps in the arrays used by the other methods were not considered in this comparison. FIG. 23C show a validation of selected spatially patterned genes detected by example spatial transcriptomics techniques by the Allen Brain Atlas (ABA) In Situ Hybridization (ISH) data.

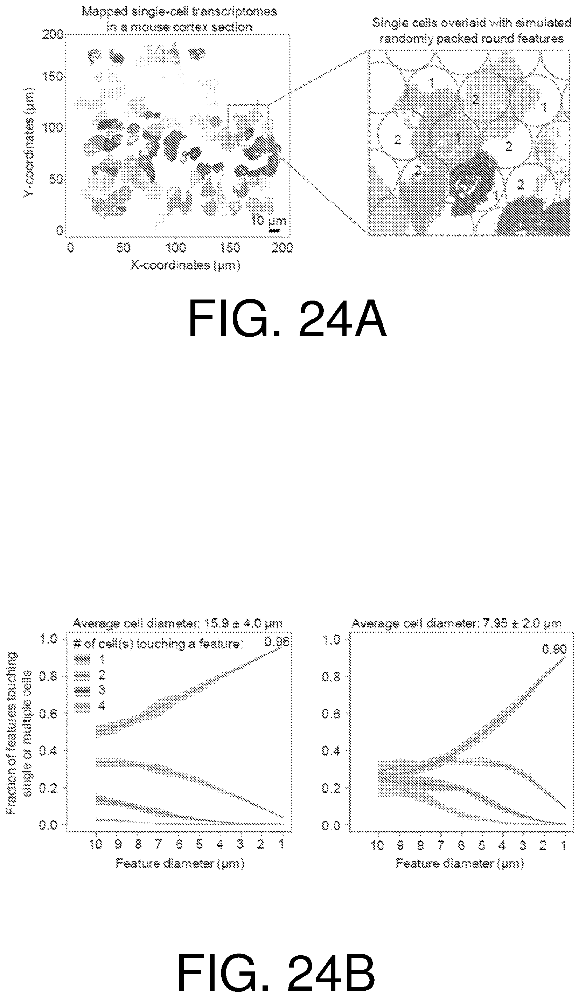

FIGS. 24A and 24B illustrate a computational simulation of how the feature size impacts single-cell resolution of the spatial barcoding. FIG. 24A shows that the simulation used a published seqFISH+ mouse cortex dataset (Eng, C. H. L. et al., 45 Nature 568, 235-239 (2019)) (left) with a pixel size of 0.103 µm. It used round features with a selected diameter size (10 to 1 µm) following a Gaussian distribution and a 5% size variation. In a 2D plane, simulated features were progressively packed and then overlaid with the cortex transcriptome data to calculate the number of cells touched by a feature (right) and group touched transcripts by feature. 10 simulations were run for each diameter size. FIG. 24B shows a fraction of features that can be confidently assigned to single cells with a cutoff of 0.7 (i.e., 70% of transcripts in a feature that came from the same cell). Original and resized cortex cells with average diameters of 15.9 (left) and 7.95 µm (right), respectively, were used for the simulation.

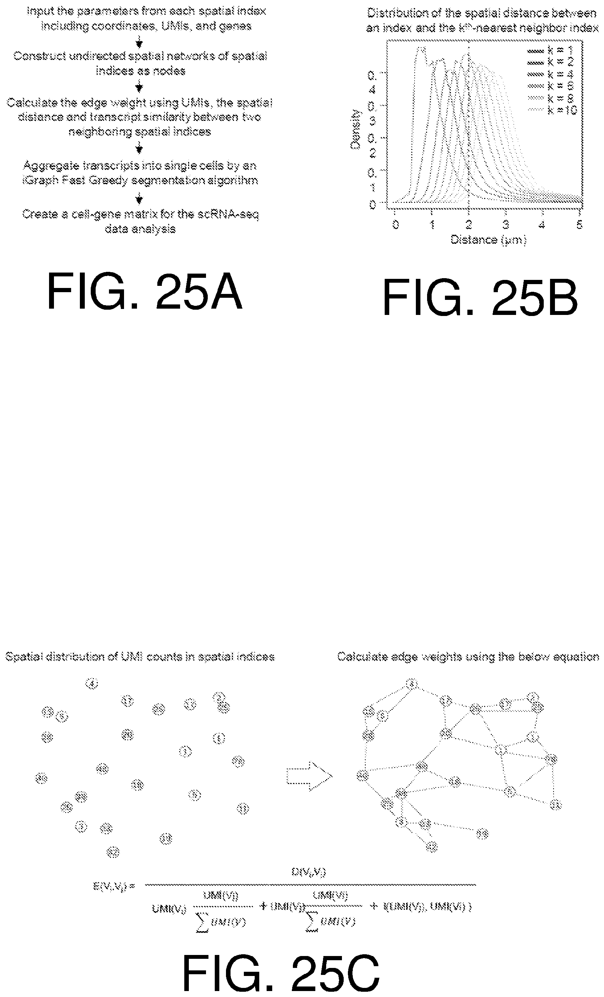

FIGS. 25A to 25C illustrate the principle of V-seg. FIG. 25A shows a pipeline of V-seg. ED FIG. 25B shows a distribution of the kth-nearest neighbor distances for each spatial index. Based on the distribution, a cutoff edge distance of 2 µm (dotted line) was chosen to ensure every index was connected to at least one different index and avoid generating many redundant edges. FIG. 25C illustrates an example of network edge weight calculation. The edge weight calculation considers the distance, UMIs and similarity of connected spatial indices. In the edge weight equation, V is a node or a spatial index, E(Vi,Vj) is an edge weight between Vi and Vj, UMI(Vi) is the UMI count in Vi; I(UMI(Vj), UMI(Vi)) is the UMI count of shared transcripts between Vj and Vi. In UMI Vi (UMI(Vj)/ ,L UMI(V)), ,L UMI(V) is the sum of UMIs on all nodes connected to the node Vi.

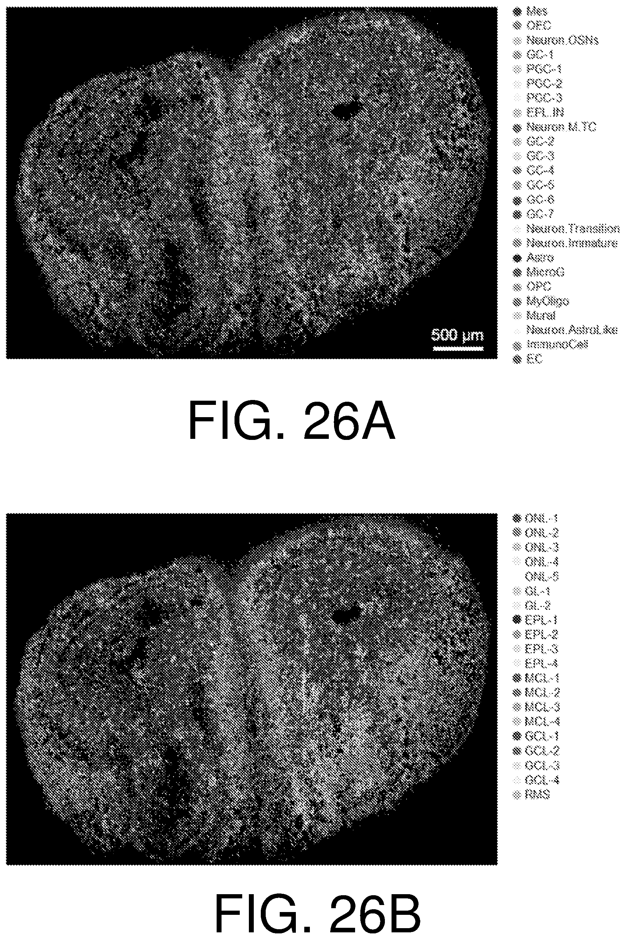

FIGS. 26A and 26B illustrate spatial distribution of V-seg-processed MOB single cells annotated by (FIG. 26A) MOB scRNA-seq data and (FIG. 26B) unsupervised clustering. Zoom-in visualization of segmented cells are in FIG. 13D (top two). Mes, mesenchymal cell; OEC, olfactory ensheathing cell; OSN, olfactory sensory neuron; GC, granule cell; PGC, periglomerular cell; Transition, transitional neuron; Immature, immature neuron; Astro, astrocyte; MicroG, microglia; OPC, oligodendrocyte precursor; MyOligo, myelinating-oligodendrocyte; Mural, mural cell; EC, endothelial cell; ONL, olfactory neuron layer; GL, glomerular layer; EPL, external plexiform layer; GCL, granule cell layer; MCL: mitral and tufted cell layers; RMS: rostral migratory stream.

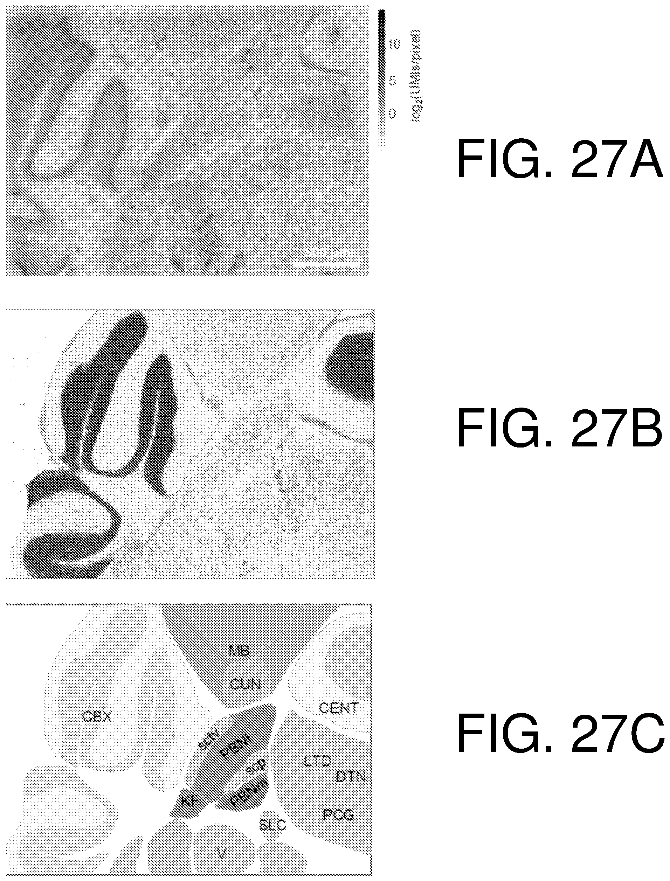

FIGS. 27A to 27C illustrate a comparison of (FIG. 27A) a UMI density map of a middle, coronal PBN section with (FIG. 27B) the corresponding ABA's Nissl-stained image and (FIG. 27C) the anatomical structure from the ABA reference atlas. CBX, cerebellar cortex; MB, midbrain; CUN, cuneiform nucleus; sctv, ventral spinocerebellar tract; PBNI, parabrachial nucleus, lateral division; PBNm, parabrachial nucleus, medial division; KF, Koelliker-Fuse subnucleus, scp, superior cerebellar peduncles; V, motor nucleus of trigeminal; SLC, subceruleus nucleus; LTD, laterodorsal tegmental nucleus; DTN: dorsal tegmental nucleus; PCG, pontine central gray; CENT: central lobule.

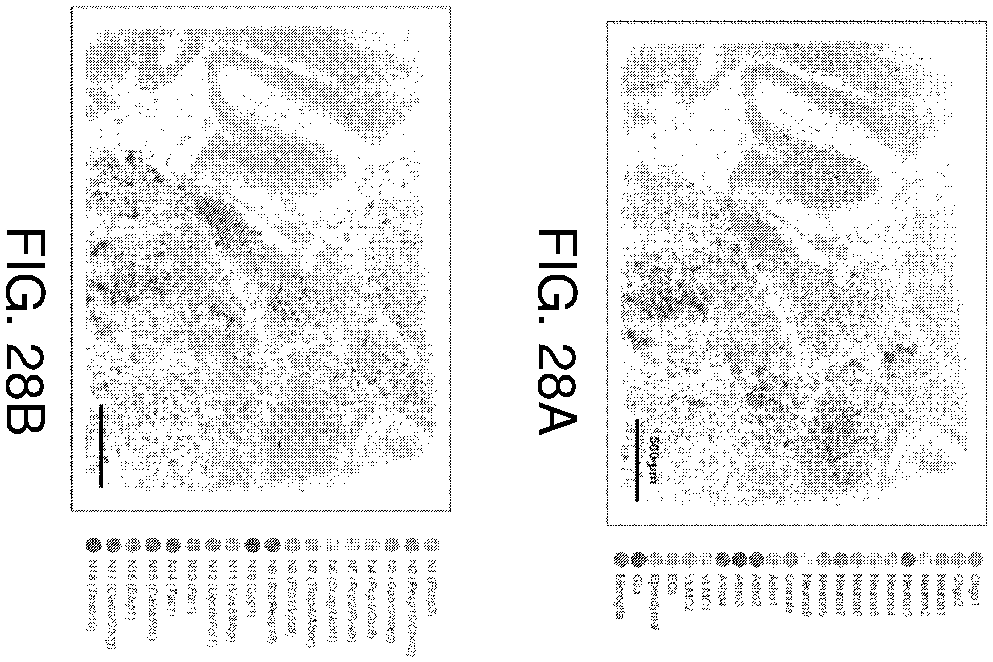

FIGS. 28A and 28B illustrate spatial distributions of cell types in the PBN and neighboring regions. FIG. 28A shows cells in the middle, coronal PBN section were clustered by Seurat to 22 types which are spatially mapped in different colors. Two oligodendrocyte clusters (Oligo1 and Oligo2), ten neuronal clusters (Neuron1-Neuron9 and Granule), four astrocyte clusters (Astro1-Astro4), and two vascular leptomeningeal clusters (VLMC1 and VLMC2), one endothelial clusters (ECs), one ependymal cluster (Ependymal), one glial cluster (Glia), and one microglia cluster (Microglia) were identified. FIG. 28b shows all neurons were re-clustered to identify 18 subclusters (N1-N18) with defined markers. Clustered neurons are mapped with other cells in grey.

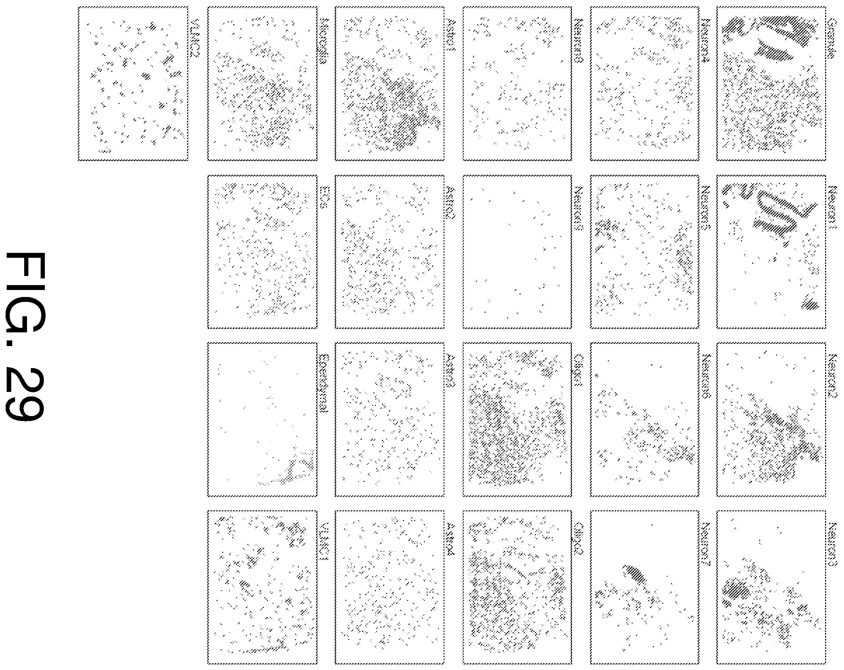

FIG. 29 illustrates spatial distributions of cell types in the middle, coronal PBN section. All cells were clustered by Seurat to identify 10 neuronal clusters (Neuron1-9 and Granule), two oligodendrocyte clusters (Oligo1 and Oligo2), four astrocyte clusters (Astro1-4), one microglial cluster (Microglia), one endothelial cluster (ECs), one ependymal cluster (Ependymal), and two vascular leptomeningeal clusters (VLMC1 and VLMC2). Cells are denoted by dots at their geometric centers. Refer to FIGS. 27,12 for the anatomical reference point.

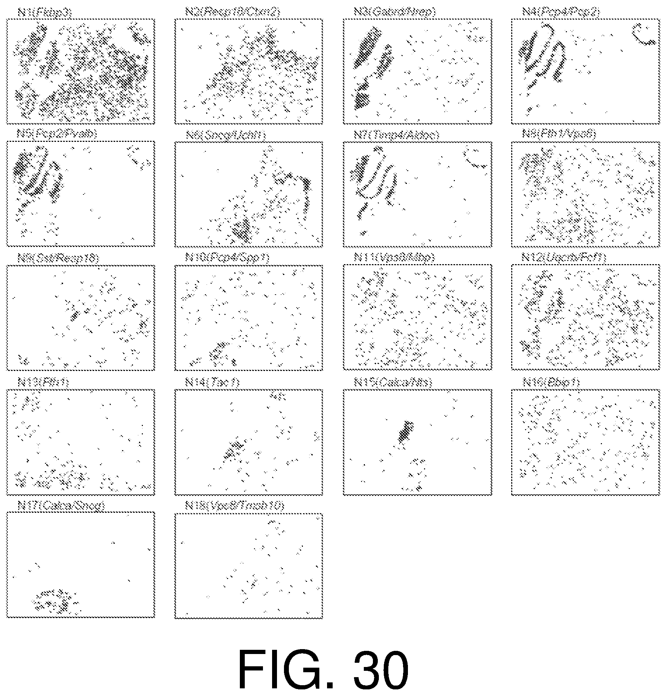

FIG. 30 illustrates spatial distributions of neuronal subtypes in the middle, coronal PBN section. Neurons were clustered by Seurat to identify 18 neuronal subclusters including the Tac1 (N14) and Calca neurons (N15). Cells are denoted by dots at their geometric centers. Refer to FIGS. 27,12 for the anatomical reference point.

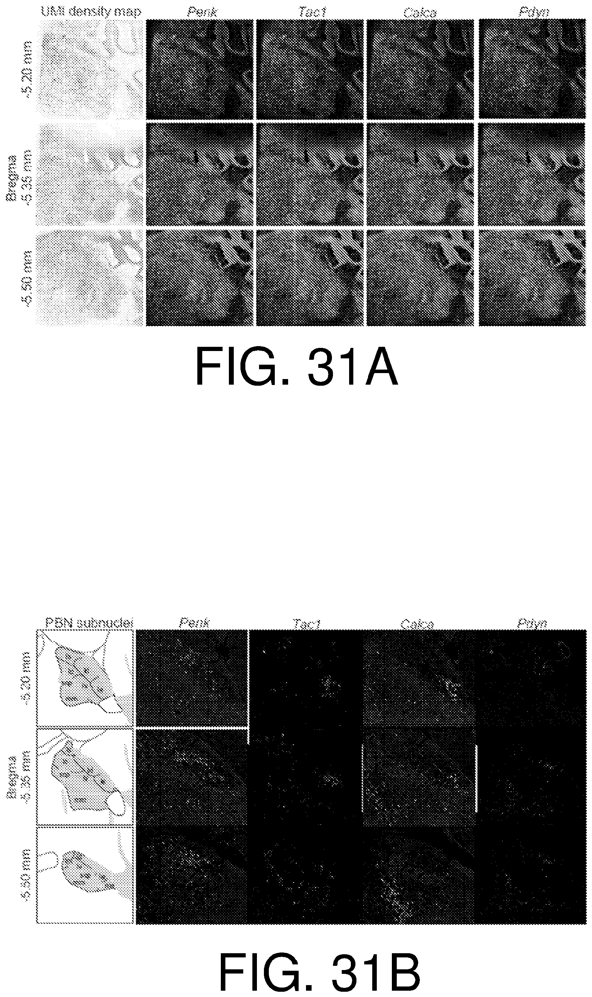

FIGS. 31A and 31B illustrate spatial distributions of selected neuropeptide genes in the PBN and neighboring regions detected by (FIG. 31A) example spatial transcriptomics techniques and (FIG. 31B) hybridization chain reaction (HCR) FISH. For the example spatial transcriptomics techniques data, neuropeptide transcripts (in red) are plotted atop the total transcripts (in gray). Subnuclei of lateral PBN: lateral (Is), dorsal (Id), center (Ic), ventral (Iv), and centeral (Ic), and external (le); subnucleus of medial PBN: medial (mm); superior cerebellar peduncles (scp).

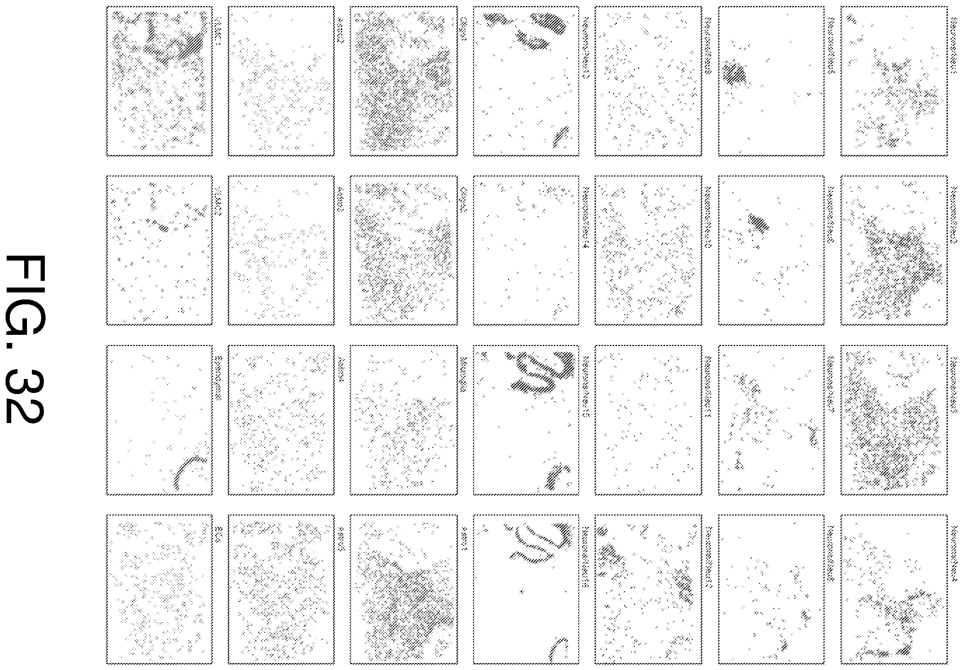

FIG. 32 illustrates spatial distributions of cell types under the sham condition. Neurons and non-neuronal cells of the middle PBN section at the sham condition were separately clustered by Seurat to identify 16 neuronal clusters (Neu1-Neu16), 12 non-neuronal clusters (two oligodendrocyte (Oligo1 and Oligo2), one microglia (Microglia), five astrocyte (Astro1-5), two vascular leptomeningeal clusters (VLMC1 and VLMC2), one ependymal cluster (Ependymal), and one endothelial clusters (ECs). Cells are plotted at their geometric centers. The anatomical reference point is shown in FIGS. 27 and 28.

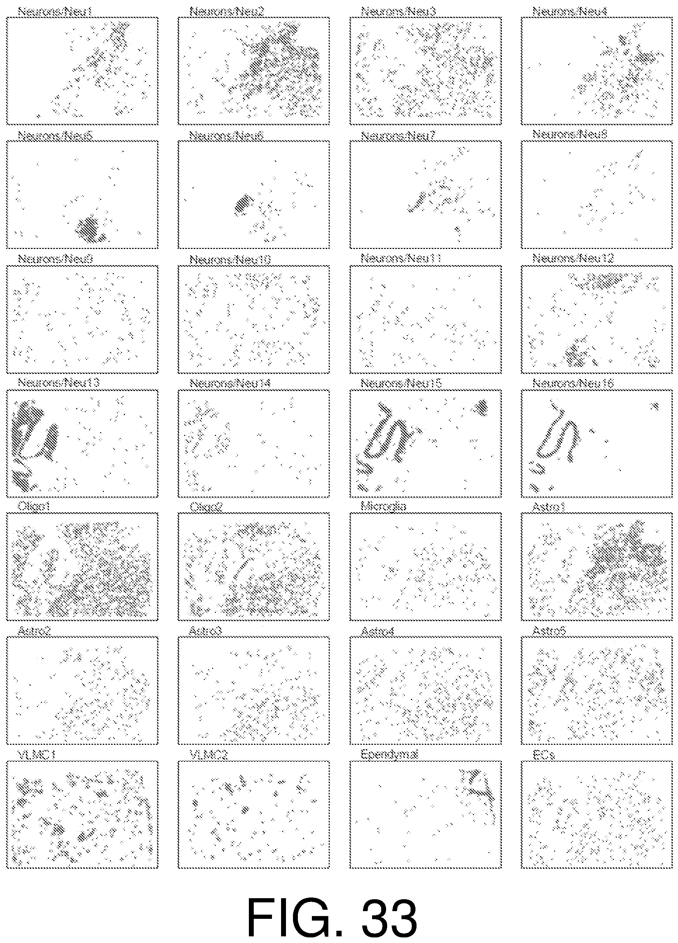

FIG. 33 shows spatial distributions of cell types under the SNL condition. Neurons and non-neuronal cells of the middle PBN section at the SNL condition were separately clustered by Seurat to identify 16 neuronal clusters (Neu1-Neu16), 12 non-neuronal clusters (two oligodendrocyte (Oligo1 and Oligo2), one microglia (Microglia), five astrocyte (Astro1-5), two vascular leptomeningeal clusters (VLMC1 and VLMC2), one ependymal cluster (Ependymal), and one endothelial clusters (ECs). Cells are plotted at their geometric centers. The anatomical reference point is shown in FIGS. 27 and 28.

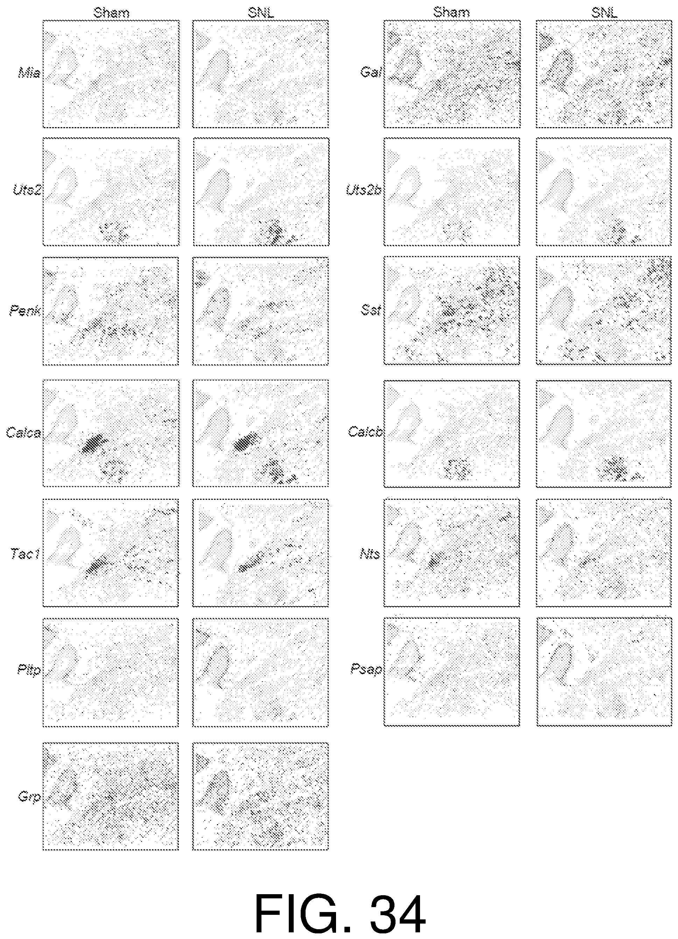

FIG. 34 illustrates a comparison of spatial distributions of selected genes associated with secreted ligands. Transcripts are plotted to a UMI density map. Each dot represents a spatial index with corresponding detected genes. The relative expression of transcripts was scaled to the gray and purple gradient. The anatomical reference point is shown in FIGS. 27 and 28.

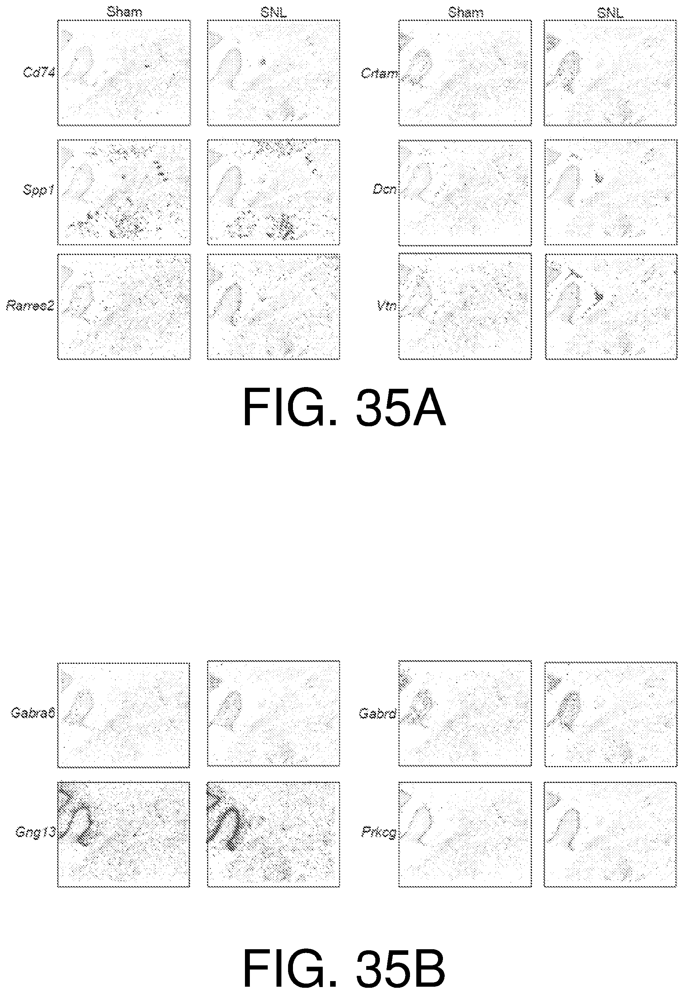

FIGS. 35A and 35B illustrate a comparison of spatial distributions of selected genes associated with (FIG. 35A) other ligand genes and (FIG. 35B) GABAergic synapse genes. Transcripts are plotted to a UMI density map. Each dot represents a spatial index with corresponding detected genes. The relative expression of transcripts was scaled to the gray and purple gradient. The anatomical reference point is shown in FIGS. 27 and 28.

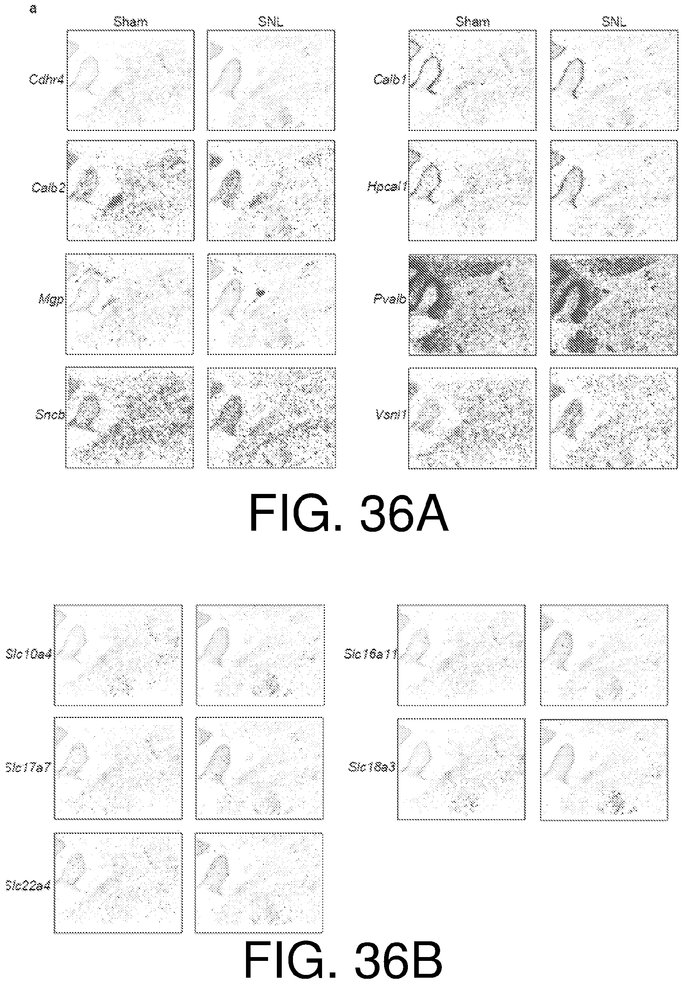

FIGS. 36A and 36B illustrate a comparison of spatial distributions of selected genes associated with (FIG. 36A) the calcium ion binding and (FIG. 36B) the solute carrier superfamily. Transcripts are plotted to a UMI density map. Each dot represents a spatial index with corresponding detected genes. The relative expression of transcripts was scaled to the gray and purple gradient. The anatomical reference point is shown in FIGS. 27 and 28.

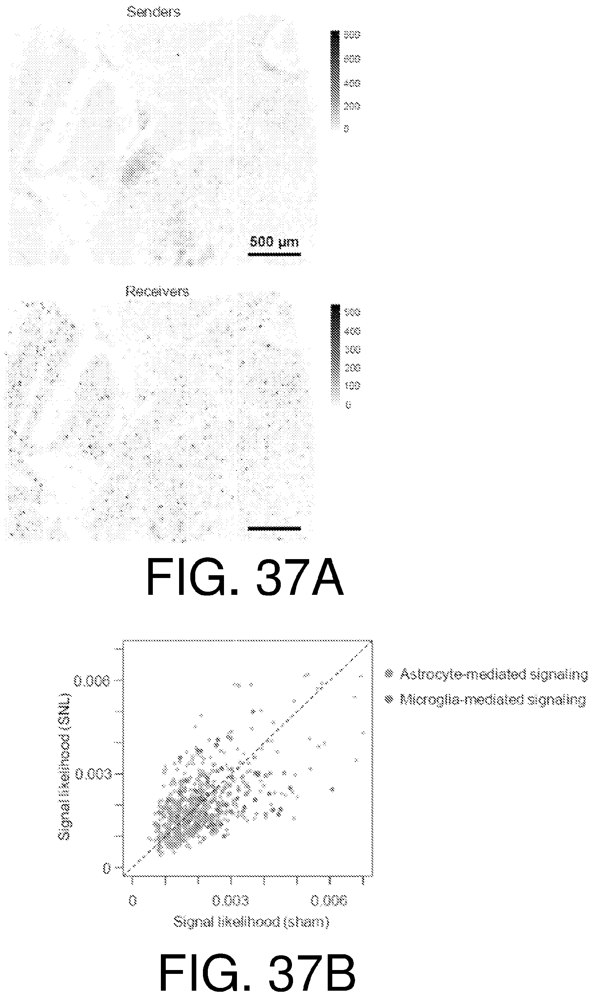

FIGS. 37A and 37B illustrate spatial cell signaling in the PBN and neighboring regions. FIG. 37A shows spatial distributions of signaling likelihoods of sender and receiver cells in the same PBN section. The signaling likelihoods of each cell as a sender (top) and a receiver (bottom) were calculated by SpaOTsc (described in Cang, et al., Nature Communications 11.1, pp. 1-13 (2020)). Cells are denoted by filled circles at their geometric centers in the 2D atlas. FIG. 37B illustrates a comparison of the signaling between different cell types under the sham and SNL conditions. Astrocyte and microglial clusters are highlighted in orange and green, respectively, and others are in gray.

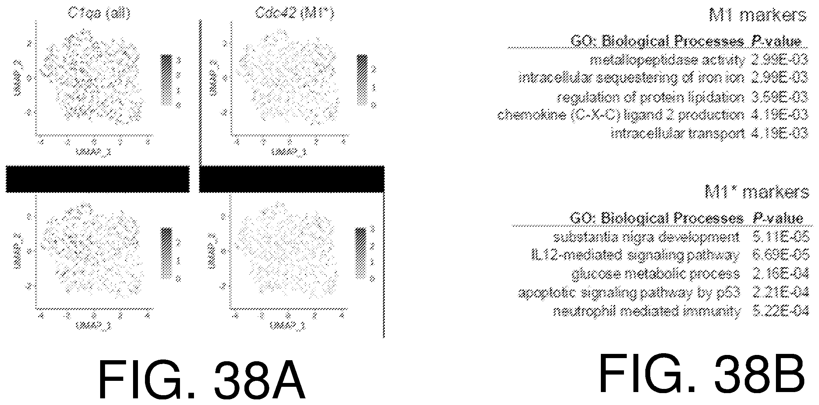

FIGS. 38A and 38B illustrate an analysis of microglial subclusters. FIG. 38A illustrates feature plots of selected microglial marker genes. FIG. 38B illustrates GO term enrichment analysis of marker genes of the microglial subclusters. P-values were calculated from a Fisher's exact test.

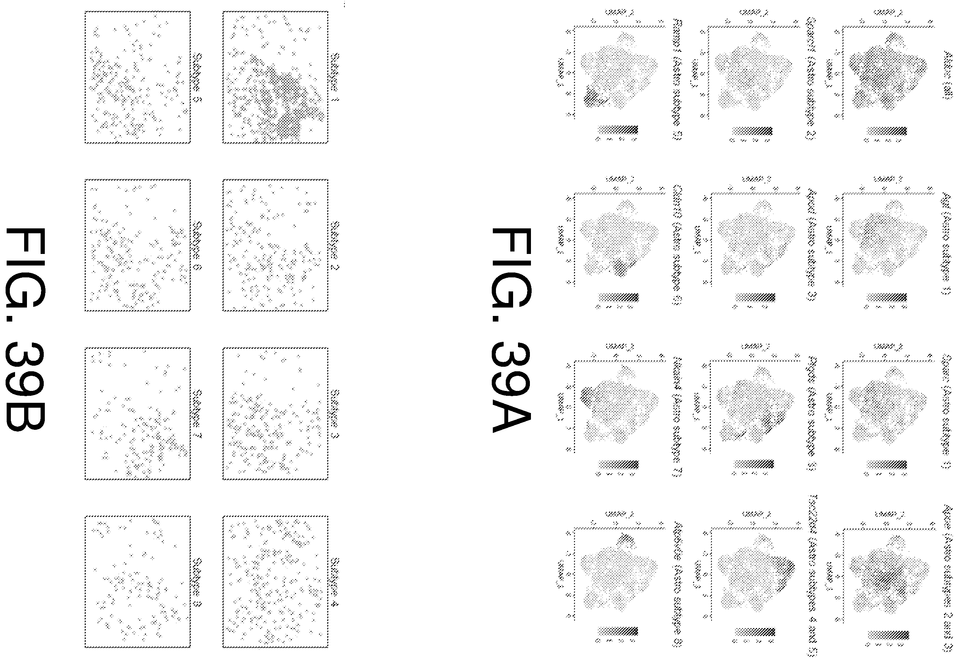

FIGS. 39A and 39B illustrate an analysis of astrocyte subclusters. FIG. 39A illustrates feature plots of astrocyte marker genes for each subcluster. FIG. 39B illustrates spatial distributions of astrocyte subclusters in the PBN region. Astrocytes are denoted by filled circles at their geometric centers in the 2D atlas.

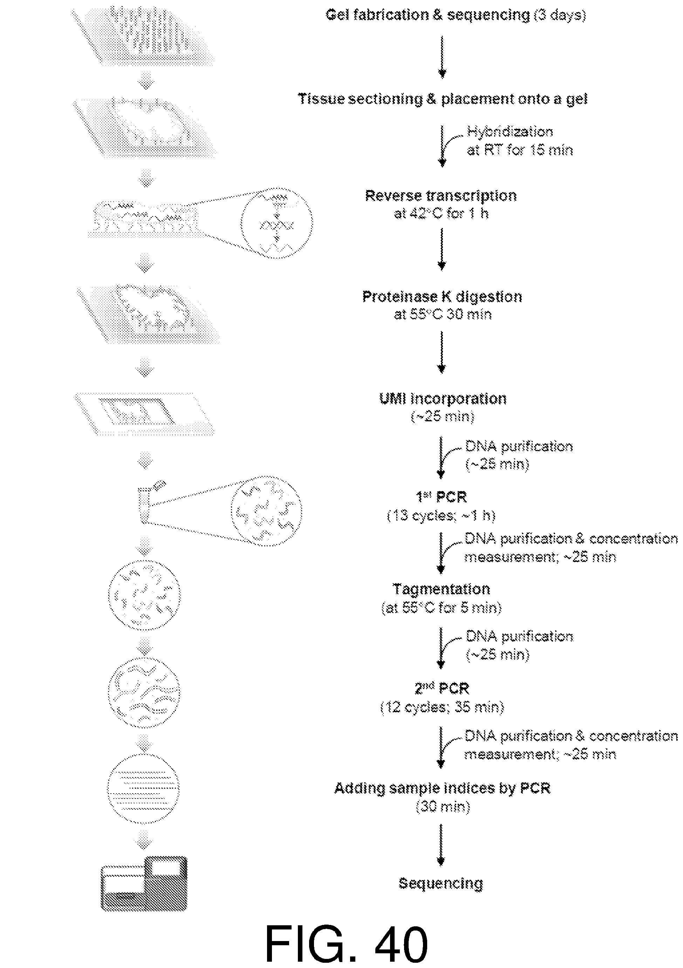

FIG. 40 illustrates a flowchart of the example spatial transcriptomics techniques assay. After polony gel fabrication, the example spatial transcriptomics techniques assay described in Experimental Example 1, from tissue sectioning to sequencing library construction, was performed in 6 hours.

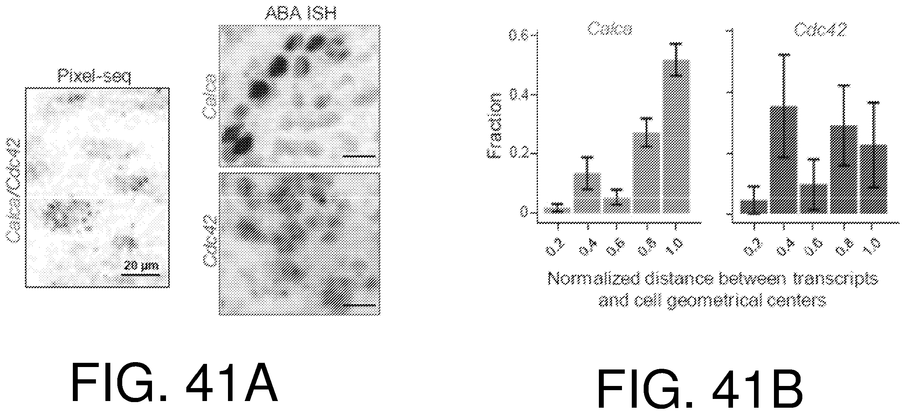

FIGS. 41A and 41B illustrate detection of transcript subcellular distribution by example spatial transcriptomics techniques. FIG. 41A illustrates example spatial transcriptomics techniques-detected subcellular distributions of Calca and Cdc42 (1 pixel = 0.325 µm) compared with the ABA ISH data. FIG. 41B illustrates a statistical analysis of subcellular distributions of Calca and Cdc42. Transcripts are binned by their normalized distances from cell geometrical centers; the centers and borders (normalized to the corresponding radiuses) are set as 0 and 1, respectively. Data represent mean values of 21 (out of 243) and 7 (out of 324) cells with at least 10 transcripts of Calca and Cdc42, respectively; error bars, 95% SEM.

### DETAILED DESCRIPTION

The present invention relates to methods for identifying the location and type of single cells within a tissue sample. The present invention also relates to a polony gel for single cell RNA sequencing.

Various implementations described herein relate to improved techniques for spatial transcriptomics, as well as for achieving enhanced single cell RNA sequencing (scRNA-seq). Spatially barcoded RNA sequencing is emerging as a powerful tool for analyzing cell and tissue organization and function in physiological and pathological conditions. Various methods described herein use arrayed oligonucleotides bearing spatial indices to barcode various analytes (e.g., RNAs, proteins) in tissue sections and do not require cell dissociation. However, their spatial resolution and RNA capturing efficiency have been limited by existing formats of oligo arrays. Various implementations described herein relate to polony-indexed library-sequencing (PIXEL-seq) using "continuous" polony-derived oligo arrays on a crosslinked hydrogels surface for spatial barcoding. Various examples of crosslinked hydrogels (also referred to as "crosslinked PAA gels") include hydrogels that include crosslinked polyacrylamide/bis; polyacrylamide/N,B'-Diallyltartramide (DATA); polyacrylamide/N.N'-(1,2-Dihydroxyethylene)biaacrylamide (DHEBA); and other gels with similar chemical and/or physical characteristics.

Various implementations described herein relate to an enhanced platform for spatial transcriptomics. This platform can be used to generate spatial transcriptomes with higher resolution and higher RNA capture efficiency than existing technologies. In contrast to existing technologies, various implementations of the present disclosure utilize a crosslinked PAA gel. Polonies formed on the surface of a crosslinked PAA gel show a continuous, homogenous DNA distribution, which is highly suited for tissue barcoding applications. In some cases, the crosslinked PAA gel is manufactured prior to primer grafting and template seeding. The crosslinked PAA gel is superior to previous gels used for transcriptomics, such as the gel described in Bentley, D. R. et al., Nature 456, 53-59 (2008) (referred to herein as a "linear PAA gel"), because the crosslinked PAA gel minimizes lateral diffusion of molecules (e.g., mRNA) across its surface. Accordingly, molecules released from a cell of a tissue sample disposed on a surface of the crosslinked PAA gel predictably remain aligned with the cell in a direction that is perpendicular to the surface of the gel.

Dense, continuous polonies are generated on the surface of a base gel (e.g., a crosslinked PAA gel). Primers are attached to the gel. Next, the base gel is seeded with various templates. As used herein, the term "template," and its equivalents, may refer to a sequence of nucleotides or amino acids that includes an index sequence and a capture sequence. Examples of templates include template precursors and/or cleaved templates, which are described further in this disclosure. For instance, template precursors are suspended in a solution that is distributed along a surface of the base gel. The template precursors attach to the primers on the surface of the gel. The template precursors are randomly distributed in the solution and therefore randomly hybridized to the surface of the base gel. In some cases, the template precursors include at least one index sequence (also referred to as a "spatial index") and at least one capture sequence. In various implementations, the base gel is seeded with multiple types of template precursors, each including a unique spatial index and a universal poly-T probe that is configured to capture different messenger RNAs (mRNAs). Other than the poly-T capture sequence, other capture sequences can be used to detect different target sequences in RNAs. Capture sequences can also be used to detect target sequences in DNA tags attached to antibodies, antibody fragments, nanobodies, monobodies, and nucleic acid aptamers, for example, when the target analyte is a protein rather than mRNA. Capture sequences can be added later by ligation and/or polymerase extension methods after cleavage.

Once a base gel has been seeded with template precursors, the template precursors are subjected to amplification to form polonies. In certain examples, the amplification includes isothermal bridge amplification. An individual template precursor seeded on the base gel surface is grown into an individual polony including many copies of the template precursor, attached to the surface of the base gel. Thus, various polonies corresponding to the seeded template precursors are generated across the surface of the base gel. Due to the random distribution of the seeded templates, the polonies are also randomly distributed on the surface of the base gel. Neighboring polonies can connect, such that the surface of the base gel is substantially covered by polonies. In various cases, the template precursors are selectively cleaved and/or digested in order to expose their respective capture sequences. For example, restriction digestion may be performed on double-stranded DNA (dsDNA) polonies to convert the template precursors to templates and expose the poly-T probe for mRNA capturing, cDNA synthesis, and spatial indexing. The base gel with the polonies distributed along a surface may be referred to as a "polony gel."

The polony gels of the present application have several advantages over existing technologies. First, widths of the polonies are significantly smaller than features reported by other techniques, which enhances the spatial resolution of the spatial transcriptome analysis. Second, the polonies on crosslinked gel show a continuous and more homogenous DNA distribution, likely because the polony amplification occurs close to the gel surface with less constraint. Third, the inventors have experimentally shown that a greater number of priming sites for cDNA synthesis are exposed after restriction digestion than in other technologies where the polonies grow within the substrate, thereby providing an enhanced RNA capture ratio, resulting in higher cDNA yield. Additionally, crosslinked gel substrate can constrain the lateral RNA diffusion to preserve the spatial resolution of spatial transcriptomes generated using the polony gels.

In various implementations in which the polonies are randomly distributed, the polonies may be spatially mapped prior to RNA capturing. For example, conventional sequencing techniques (e.g., sequencing-by-synthesis) can be performed to identify the locations of the polonies, as well as the corresponding index sequences within the polonies, on the surface of the crosslinked PAA gel. That is, a map of the polonies along the surface of the crosslinked PAA gel (a "polony map") can be generated.

The polony gel and polony map may be used to identify the type and location of various cells within a tissue sample. In various implementations, a tissue section is disposed on the polony gel. In various implementations, a single cell within the tissue section overlaps multiple polonies. Different RNAs from the single cell are adsorbed to the surface of the overlapping polonies. Because the RNAs is hybridized to the polony gel, the RNAs from the single cell do not, or minimally, diffuse to other sections of the polony gel, which further enhances spatial resolution. The RNAs may be captured by the exposed capture probe on the templates of the polonies.

After RNA capturing, in situ cDNA synthesis can be performed. Resulted cDNAs can be amplified from the gels and generate libraries for subsequent sequencing (e.g., next generation sequencing (NGS)). After decoding the sequencing reads, each RNA may be assigned to a spatial index sequence and mapped to polony map. By aligning the spatial index sequence to the polony gel map, a high-resolution spatial transcriptome can be produced.

Segmenting the cells can be performed using a variety of methods. In some cases, the tissue section on the gels may be stained and pre-scanned to generate a reference image to identify cell boundaries. However, in some implementations, the tissue imaging is unnecessary. For example, the polony map may be cross-referenced to the sequencing reads to identify which polonies overlap individual cells, and the locations of the cells can be estimated based on the locations of the polonies. Accordingly, a high-resolution spatial transcriptome can be generated in accordance with various implementations described herein.

Particular examples will now be described with reference to the accompanying figures. The scope of this disclosure includes individual examples described herein as well as any combination of the examples, unless otherwise specified.

FIG. 1 illustrates an example system 100 for generating a spatial transcriptome 102 of a tissue sample 104 using a polony gel 106. Such systems may be useful in the claimed methods. The polony gel 106 includes a base gel 108 and multiple types of polonies, such as a first polony 110 and a second polony 112. The first polony 110 and the second polony 112 are disposed at and/or on a surface of the base gel 108 with limited or no penetration into the gel itself.

In various cases, the base gel 108 is particularly suited to prevent lateral mRNA diffusion in a direction parallel to the surface. In various implementations, the base gel 108 includes polyacrylamide (PAA) (e.g., crosslinked polyacrylamide/bis; polyacrylamide/N,B'-Diallyltartramide (DATA); polyacrylamide/N.N'-(1,2-Dihydroxyethylene)biaacrylamide (DHEBA)). In various cases, the base gel 108 includes crosslinked PAA. In some implementations, base gel 108 includes at least one of 3-20% (by weight) polyacrylamide (e.g., polyacrylamide/bis, polyacrylamide/DATA, polyacrylamide/DHEBA, or any combination thereof); 0.01-1 % (by weight) ammonium persulfate; or 0.01-1% (by volume) N,N,N',N'-tetramethylethylenediamine (TEMED). According to some examples, the base gel 108 further includes 1-50 mg/ml (0.001- 0.1 % (by weight) N-(5-bromoacetamidylpentyl) acrylamide (BRAPA). The methods of the invention comprise generating a crosslinked polyacrylamide (PAA) gel comprising 3-20% acrylamide/bis, 0.01-1% ammonium persulfate, and 0.01-1% N,N,N',N'-tetramethylethylenediamine (TEMED). The polony gels for single cell RNA sequencing as claimed comprise a hydrogel comprising crosslinked PAA; a first polony on a surface of the hydrogel, the first polony comprising a first template that comprises a first capture sequence and a first index sequence; and a second polony on the surface of the hydrogel, the second polony bordering the first polony and comprising a second template that comprises a second capture sequence and a second index sequence.

In some examples, the base gel 108 is generated by polymerizing acrylamide. For example, the base gel 108 is generated by polymerizing the acrylamide at a temperature greater than or equal to 0° Celsius (C) and less than or equal to 30°C. In some implementations, the base gel 108 is generated at a humidity of 0 to 60% humidity. The acrylamide may be polymerized in an anerobic environment. For instance, the polymerization is performed in an environment that includes less than 1,000 parts per million (ppm) of Oxygen.

The surface of the base gel 108 is a linear (e.g., flat) surface, for example. In some cases, the base gel 108 is shaped like a rectangular prism, although many shapes are appropriate. According to various implementations, the first and second polonies 110 and 112 are disposed in and/or on the linear surface of the base gel 108. Although only two types of polonies are illustrated in FIG. 1, in various examples, the polony gel 106 includes numerous types of polonies, such as 10, 100, 1,000, or 10,000 types of polonies, or more. Although illustrated in a regular pattern in FIG. 1, the first and second polonies 110 and 112 are patterned in an irregular pattern in various implementations. For example, the first and second polonies 110 and 112 may be randomly distributed on a surface of the base gel 108.

In various cases, the first and second polonies 110 and 112 may have widths that are smaller than widths of cells within the tissue sample 104. For example, the first polony 110 may have a narrower width than an example cell in the tissue sample 104 and the second polony 112 may have a narrower width than the example cell. In various implementations, the width of the example first polony 110 and/or the width of the example second polony 112 are in a range of 0.1 micron (µm) to 10 µm, or 0.1 µm to 2 µm..

The first polony 110 includes multiple copies of a first template 114 and the second polony 112 includes multiple copies of a second template 116. For example, the first polony 110 includes greater than or equal to 100 copies of the first template 114 and less than or equal to 1,000,000 copies of the first template 114. Similarly, the second polony 112 includes greater than or equal to 100 copies of the second template 116 and less than or equal to 1,000,000 copies of the second template 116.

According to various implementations, the first template 114 includes a first index sequence and the second template 116 includes a second index sequence. As used herein, the terms "index sequence," "spatial index," "spatial barcode," and their equivalents can refer to a particular sequence that is unique to a template. The index sequence, for example, has a length that is greater than or equal to 10 bases or base pairs (BPs) and that is less than or equal to 50 bases or BP (e.g., 24 BP). The first index sequence is different than the second index sequence. Accordingly, the first template 114 has a different sequence than the second template 116.

In various cases, the first template 114 and the second template 116 include at least one capture sequence that binds a target analyte (e.g., RNA, DNA, protein, or etc.). In various implementations, a capture sequence is nonspecific to a sequence of the analyte that it binds to. For example, the capture sequence is a poly-T probe that attaches to different RNA sequences. In some implementations, a capture sequence is specific to the analyte that it binds to. For instance, the capture sequence is specifically complementary to the sequence of an RNA analyte, such that the capture sequence specifically binds to the RNA. The capture sequence, in some cases, has a length that is greater than or equal to 15 bases or BP and is less than or equal to 500 bases or BP. According to various implementations, the capture sequence is configured to bind mRNA that has diffused from cells in the tissue sample 104. The capture sequence(s), in various cases, is at a 3' end of the first template 114 and a 3' end of the second template 116. Capture sequences for proteins include, for example, antibody binding domains, nanobodies, monobodies, and nucleic acid aptamers.

According to some examples, the first template 114 and the second template 116 further include at least one sequencing primer site. The sequencing primer site(s) is for spatial index sequencing to generate image(s) 126, the length can be 30-50 BP. Examples of the sequencing primer site include GCGGCCGCTAATACGACTCACTATAGGGATC (SEQ ID NO: 1) and/or CGAATTCCATCATTGTTCCCGGGTTCCTCATTCTC (SEQ ID NO: 2). For example, the sequencing primer is disposed between the first index sequence and the capture sequence of the first template 114, and the sequencing primer is disposed between the second index sequence and the capture sequence of the second template 116.

The first template 114 and the second template 116 are bound to the base gel 108 by primers 118. According to various implementations, two different types of primers 118 (also referred to as a "primer pair") are bound to the base gel 108. In various implementations, the first template 114, the second template 116, and the primers 118 include DNA. In some implementations, a 5' end of each primer 118 includes a phosphorothioate (PS) modification and/or an acrydite modification. The 5' end of each type of primer 118 is bound to PAA in the base gel 108. For example, if the primers 118 have a PS modification, the base gel 108 includes BRAPA. In some instances, if the primers 116 have an acrydite modification, the base gel 108 omits BRAPA. The primer pairs 118 include a sequence that is complementary to binding sequence 308 of template and that facilitates attachment of the first and second templates 114 and 116 to the primers 118. The primers 118, for instance, individually have a length of 25-35 bases or BP.

The polony gel 106 may be fabricated by a process that includes fabricating the base gel 108, binding the two types of primer 118 to the base gel 108, attaching template precursors to the bound primer 118, amplifying the template precursors on the base gel 108, and digesting the template precursors to reveal the capture sequences and the sequencing primer site of the first and second templates 114 and 116. The first and second polonies 110 and 112 are a result of the digestion of the amplified template precursors. The template precursors may include the index sequences and the capture sequences. In addition, the template precursors may further include at least one digestion site that specifically binds at least one enzyme (e.g., Taql). In various cases, a digestion site is adjacent to the capture sequence within an example template precursor. For example, the digestion site is connected to a 3' end of the capture sequence within the example template precursor. The template precursors and the first and second templates 114 and 116 may include DNA, in various examples.

According to various implementations, a border of the first polony 110 touches a border of the second polony 112 (i.e., is continuous). For example, an example of the first template 114 in the first polony 110 neighbors an example of the second template 116 in the second polony 112 by a distance in a range of 0.1-1 µm. In some cases, the first polony 110 and second polony 112 overlap, such as an example of the first template 114 is disposed between two examples of the second template 116. According to various implementations, a distance between an example of the first template 114 and a first example of the second template 116 is narrower than a distance between the first example of the second template 116 and a second example of the second template 116. In various cases, the first and second polonies 110 and 112 do not overlap. The first and second polonies 110 112 are densely packed together and substantially continuous on the surface of the base gel 102.

In various examples, at least one first computing device 120 is configured to generate a polony map 122 indicating the positions of the first polonies 110 and the positions of the second polonies 112 on the surface of the polony gel 106. The computing device(s) 120 may include at least one processor and memory storing instructions that, when executed by the processor, cause the processor to perform various operations of the computing device(s) 120.

The polony map 122 can be generated by sequencing the first and second polonies 110 and 112 by any appropriate sequencing method. For example, sequencing-by-synthesis can be performed on the first and second polonies 110 and 112. A sequencing-by-synthesis technique may be performed by adding DNA polymerase and fluorescently tagged deoxyribonucleotide triphosphates (dNTPs) to the polony gel 108. For example, at least one liquid including the DNA polymerase and dNTPs is disposed on the surface of the polony gel 108. The DNA polymerase facilitates a DNA synthesis reaction that generates new DNA molecules that are complementary to the first and second templates 114 and 116 in the polony gel 108. In particular, the new DNA molecules are generated using the fluorescently tagged dNTPs. As each fluorescently tagged dNTPs is incorporated into the new DNA molecules a photon is emitted. In various implementations, the index sequences of the first and second templates 114 and 116 are determined by detecting the photons.

The photons, for example, are detected by at least one camera 124 that is communicatively coupled to the first computing device(s) 120. In various implementations, the base gel 108 is disposed between the camera(s) 124 and the first polonies 110 and second polonies 112. The camera(s) 124 is configured to generate at least one image 126 of the polony gel 106. As used herein, the term "image," and its equivalents, can refer to a two-dimensional (2D) group of pixels and/or a three-dimensional (3D) group of pixels (also referred to as "voxels" in 3D images). In various implementations, each pixel in an image has at least one value. In the case of an RGB image, each pixel has three values respectively corresponding to a red channel, a green channel, and a blue channel. Each pixel of the image(s) captured by the camera(s) 124 depicts an area on the surface of the polony gel 108. In particular, pixels of the image(s) represent photons emitted from the new DNA molecules generated in the sequencing-by-synthesis reaction. In some cases, the camera(s) 124 include and/or are communicatively coupled to a fluorescence microscope, such that the photons from the new DNA molecules travel through an objective of the fluorescence microscope before being detected by at least one array of photosensors in the camera(s) 124. The image(s) 126, for example, include multiple images captured by the camera(s) 124 at different focal lengths, angles, times, or any combination thereof.

The computing device(s) 120 identify the index sequences in the first template 114 and the second template 116 by analyzing the image(s) 126. For example, the computing device(s) 120 identify the sequences of the new DNA generated by the sequencing reaction by identifying the sequence of photons emitted by the dNTPs during the synthesis of the new DNA.

In addition, the computing device(s) 120 identify the locations of the first and second polonies 110 and 112 by analyzing the image(s) 126. For example, the computing device(s) 120 identify a group of first pixels in the image(s) 126 that correspond to the first index sequence of the first template 114. The computing device(s) 120 may determine that the first polony 110 is located in an area of the surface of the polony gel 108 that is depicted by the first pixels. Similarly, the computing device(s) 120 may identify a group of second pixels in the image(s) 126 that correspond to the second index sequence of the second template 116, and may determine that the second polony 112 is located in an area of the surface of the polony gel 108 that is depicted by the second pixels.

The polony map 122 indicates the identities and/or locations of the first and second polonies 110 and 112. For example, the polony map 122 inclues a 2D image with pixels corresponding to areas of the surface of the polony gel 106. The value of each pixel may correspond to the identity of the polony at the corresponding location. For example, the polony map 122 may include first pixels with a first value (e.g., "1") corresponding to the locations of the first polonies 110 along the surface of the polony gel 106, and may include second pixels with a second value (e.g., "2") corresponding to the locations of the second polonies 112 along the surface of the polony gel 106. In some cases, the first polonies 110 are represented by a first color (e.g., an RGB color) and the second polonies 112 are represented by a second color. In various cases, at least one pixel representing a region of the first polonies 110 directly neighbors at least one pixel representing a region of the second polonies 112.

In addition, the polony map 122 may include metadata indicating the index sequence corresponding to each polony on the polony gel 106. For instance, the polony map 122 may indicate that the first index sequence of the first template 114 corresponds to the area on the base gel 108 that is covered by the first polony 110, and may also indicate that the second index sequence of the second template 114 corresponds to the area of the base gel 108 that is covered by the second polony 112.

The polony gel 106 may be packaged into a housing 128. Collectively, the polony gel 106 and the housing 128 may be referred to as a "kit." The housing provides structural support for the polony gel 106 and/or prevents contamination of the polony gel 106 by dust, gasses, fluids, liquids, or other contaminants before it is used for tissue analysis. The housing 128 may include a polymer or other material, such as polystyrene, polypropylene, or the like. In some implementations, the housing 128 includes a tray in which the polony gel 106 is disposed and a peelable lid that encloses the polony gel 106 within the tray. The lid may be removed from the rest of the housing 128 in order to expose the first polonies 110 and the second polonies 112.

The polony map 122 may be used to generate the spatial transcriptome 102 of the tissue sample 104. In various implementations, the tissue sample 104 is disposed on the surface of the polony gel 106. For example, the tissue sample 104 is a cryosectioned portion of a multicellular organism (e.g., an animal, a plant, a fungus, etc.). The tissue sample 104 includes multiple cells. The cells may contain the same or similar DNA. However, the cells may express different RNA. For instance, the tissue sample 104 includes a first cell type that expresses a first RNA and a second cell type that expresses a second RNA. The spatial transcriptome 102 indicates the locations of the first cell type in the tissue sample 104 and the locations of the second cell type in the tissue sample 104, for example.

In various implementations, an analyte (e.g., RNA) from the tissue sample 104 travels from the cells to the polony gel 106. In various implementations, the base gel 108 prevents substantial diffusion by the analyte along the surface of the polony gel 106. Thus, the analyte from an example cell in the tissue sample 104 remains aligned with the example cell in a vertical direction (e.g., a perpendicular to the surface of the polony gel 106). Because the widths of the first polonies 110 and the second polonies 112 are narrower than the cells within the tissue sample 104, the individual cells may overlap with multiple polonies of the polony gel 106.

The analyte from the tissue sample 104 bind to the templates in the polony gel 106. For example, the analyte from an example cell overlapping an example first polony 110 binds to the capture sequence of the first template 114. If the example cell also overlaps an example second polony 112, the RNA from the example cell also binds to the capture sequence of the second template 116.

The spatial transcriptome 102 may be generated by sequencing the polonies and the bound analyte. In various implementations in which the analyte is RNA, reverse transcription is performed to generate cDNAs that are complementary to the first template 114 bound to the RNA and the second template 116 bound to the RNA. For example, a Reverse Transcriptase (RT) binds to an end of the first template 114 where the capture sequence is disposed. The RT facilitates a polymerization reaction wherein the first template 114 is extended by nucleotides that are complementary to the bound RNA. Accordingly, the extended first template 114 includes both the index sequence and a sequence complementary to the RNA.

RNA can be subjected to any form of cDNA generation or sequencing. Reverse Transcriptases (RTs) are enzymes that perform reverse transcription of RNA into a first strand of cDNA. More processive RTs can be used to increase sequence read lengths. In certain examples, the processivity of an RT refers to the ability of an RT to generate a complementary strand of DNA across the full-length of the template RNA. Some RT enzymes (e.g., SuperScript IV (SSIV) achieve this via multiple binding events, whereas others (e.g., MarathonRT), can do so in single binding event. Traditionally, RT included Moloney Murine Leukemia Virus RT (M-MLV RT) and Avian Myeloblastosis Virus RT (AMV RT). RTs have since been developed that are superior for the generation of longer, or full-length, cDNAs, even at lower temperature ranges. For example, the M-MLV gene was mutated to eliminate the endogenous RNase H activity and this modified enzyme was referred to as Superscript^{™} II RT (Gibco-BRL). In certain examples, an RT with high processivity is MarathonRT. For more information regarding RTs with high processivity see US 20210155910 (also published as WO2019005955).

In certain implementations, the RT are thermocycling RT, thereby allowing for amplification of RNA templates in a single reaction.

The RNA sequence bound to the first and second templates 114 can be determined by sequencing the cDNAs. As is understood by one of ordinary skill in the art, adapters can also be used to target particular types of RNA for cDNA generation or to allow for labeling all types of RNA for non-selective cDNA generation. Useful RNA adapters are described in, for example, US2014/0357528. Adapters which provide priming sequences for both amplification and sequencing of fragments for use with the 454 Life Science GS20 sequencing system are described by F. Cheung, et al. in BMC Genomics 2006, 7:272.

Various suitable sequencing techniques can be used to sequence the cDNAs. For example, in certain implementations, sample partition PCR methods may be used. In sample partitioning, numerous methods can be used to divide samples into discrete partitions (e.g., droplets). Exemplary partitioning methods and systems include use of one or more of emulsification, droplet actuation, microfluidics platforms, continuous-flow microfluidics, reagent immobilization, and combinations thereof. In particular implementations, partitioning is performed to divide a sample into a sufficient number of partitions such that each partition contains one or zero nucleic acid molecules. In particular implementations, the number and size of partitions is based on the concentration and volume of the bulk sample.

In particular implementations, amplification can be performed by sample partition dPCR (spdPCR). An example of sample partition dPCR is Droplet Digital PCR. Droplet digital PCR (ddPCR) (e.g., Droplet Digital^{™} PCR (ddPCR^{™}) (Bio-Rad Laboratories, Hercules, CA)) technology uses a combination of microfluidics and surfactant chemistry to divide PCR samples into water-in-oil droplets. Hindson et al., Anal. Chem. 83(22): 8604-8610 (2011). The droplets support PCR amplification of template molecules they contain and use reagents and workflows similar to those used for most standard Taqman probe-based assays.

Following PCR, each droplet is analyzed or read in a flow cytometer to determine the fraction of PCR-positive droplets in the original sample. These data are then analyzed using Poisson statistics to determine the target concentration in the original sample. See Bio-Rad Droplet Digital^{™} (ddPCR^{™}) PCR Technology.

Amplification can be performed. Nucleic acids of a sample (e.g., partitioned nucleic acids) can be amplified by any suitable PCR methodology. Exemplary PCR types include allele-specific PCR, assembly PCR, asymmetric PCR, endpoint PCR, hot-start PCR, in situ PCR, intersequence-specific PCR, inverse PCR, linear after exponential PCR, ligation-mediated PCR, methylation-specific PCR, miniprimer PCR, multiplex ligation-dependent probe amplification, multiplex PCR, nested PCR, overlap-extension PCR, polymerase cycling assembly, qualitative PCR, quantitative PCR, real-time PCR, single-cell PCR, solid-phase PCR, thermal asymmetric interlaced PCR, touchdown PCR, universal fast walking PCR, etc. Ligase chain reaction (LCR) may also be used.

PCR may be performed with a thermostable polymerase, such as Taq DNA polymerase (e.g., wild-type enzyme, a Stoffel fragment, FastStart polymerase, etc.), Pfu DNA polymerase, S-Tbr polymerase, Tth polymerase, Vent polymerase, or a combination thereof, among others.

PCR and LCR are driven by thermal cycling. Alternative amplification reactions, which may be performed isothermally, can also be used. Exemplary isothermal techniques include branched-probe DNA assays, cascade-RCA, helicase-dependent amplification, loop-mediated isothermal amplification (LAMP), nucleic acid based amplification (NASBA), nicking enzyme amplification reaction (NEAR), PAN-AC, Q-beta replicase amplification, rolling circle replication (RCA), self-sustaining sequence replication, strand-displacement amplification, etc.

In examples using sample partitioning, amplification reagents can be added to a sample prior to partitioning, concurrently with partitioning and/or after partitioning has occurred. In particular implementations, all partitions are subjected to amplification conditions (e.g. reagents and thermal cycling), but amplification only occurs in partitions containing target nucleic acids (e.g. nucleic acids containing sequences complementary to primers added to the sample). The template nucleic acid can be the limiting reagent in a partitioned amplification reaction. In particular implementations, a partition contains one or zero target (e.g. template) nucleic acid molecules.

In particular implementations, nucleic acid targets,, primers, and/or probes are immobilized to a surface, for example, a substrate, plate, array, bead, particle, etc. Immobilization of one or more reagents provides (or assists in) one or more of: partitioning of reagents (e.g. target nucleic acids, primers, probes, etc.), controlling the number of reagents per partition, and/or controlling the ratio of one reagent to another in each partition. In particular implementations, assay reagents and/or target nucleic acids are immobilized to a surface while retaining the capability to interact and/or react with other reagents (e.g. reagent dispensed from a microfluidic platform, a droplet microactuator, etc.). In particular implementations, reagents are immobilized on a substrate and droplets or partitioned reagents are brought into contact with the immobilized reagents. Techniques for immobilization of nucleic acids and other reagents to surfaces are well understood by those of ordinary in the art. See, for example, U.S. Patent No. 5,472,881 and Taira et al. Biotechnol. Bioeng. 89(7), 835-8 (2005).

Target sequence detection methods can be utilized to identify sample partitions containing amplified target(s) (i.e., unique sequences). Detection can be based on one or more characteristics of a sample such as a physical, chemical, luminescent, or electrical aspects, which correlate with amplification.

In particular implementations, fluorescence detection methods are used to detect amplified target(s), and/or identification of samples (e.g., partitions) containing amplified target(s). Exemplary fluorescent detection reagents include TaqMan probes, SYBR Green fluorescent probes, molecular beacon probes, scorpion probes, and/or LightUp probes^{®} (LightUp Technologies AB, Huddinge, Sweden). Additional detection reagents and methods are described in, for example, U.S. Patent Nos. 5,945,283; 5,210,015; 5,538,848; and 5,863,736; PCT Publication WO 97/22719; and publications: Gibson et al., Genome Research, 6, 995-1001 (1996); Heid et al., Genome Research, 6, 986-994 (1996); Holland et al., Proc. Natl. Acad. Sci. USA 88, 7276-7280, (1991); Livak et al., Genome Research, 4, 357-362 (1995); Piatek et al., Nat. Biotechnol. 16, 359-63 (1998); Neri et al., Advances in Nucleic Acid and Protein Analysis, 3826, 117-125 (2000); Compton, Nature 350, 91-92 (1991); Thelwell et al., Nucleic Acids Research, 28, 3752-3761 (2000); Tyagi and Kramer, Nat. Biotechnol. 14, 303-308 (1996); Tyagi et al., Nat. Biotechnol. 16, 49-53 (1998); and Sohn et al., Proc. Natl. Acad. Sci. U.S.A. 97, 10687-10690 (2000).

In particular implementations, detection reagents are included with amplification reagents added to a bulk or partitioned sample. In particular implementations, amplification reagents also serve as detection reagents. In particular implementations, detection reagents are added to partitions following amplification. In particular implementations, measurements of the absolute copy number and the relative proportion of target nucleic acids in a sample (e.g. relative to other targets nucleic acids, relative to non-target nucleic acids, relative to total nucleic acids, etc.) can be measured based on the detection of samples (e.g., partitions) containing amplified targets.

In particular implementations, following amplification, samples containing amplified target(s) are sorted from samples not containing amplified targets or from samples containing other amplified target(s). In particular implementations, samples are sorted following amplification based on physical, chemical, and/or optical characteristics of the samples, the nucleic acids therein (e.g. concentration), and/or status of detection reagents. In particular implementations, individual samples are isolated for subsequent manipulation, processing, and/or analysis of the amplified target(s) therein. In particular implementations, samples containing similar characteristics (e.g. same fluorescent labels, similar nucleic acid concentrations, etc.) are grouped (e.g. into packets) for subsequent manipulation, processing, and/or analysis.

Particular implementations utilize NGS. In particular implementations, sequencing with commercially available NGS platforms may be conducted with the following steps. First, DNA sequencing libraries may be generated by clonal amplification by PCR in vitro. Second, the DNA may be sequenced by synthesis, such that the DNA sequence is determined by the addition of nucleotides to the complementary strand rather through chain-termination chemistry. Third, the spatially segregated, amplified DNA templates may be sequenced simultaneously in a massively parallel fashion without the requirement for a physical separation step. While these steps are followed in most NGS platforms, each utilizes a different strategy (see e.g., Anderson, M. W. and Schrijver, I., 2010, Genes, 1: 38-69.). Examples of NGS platforms include Oxford Nanopore Technologies, Roche 454, GS FLX Titanium, Illumina, HiSeq 2000, Genom Analyzer IIX, IIE, IScanSQ, Life Technologies Solid 4, Helicos Biosciences Heliscope, Pacific Biosciences (PacBio) SMART and PacBio HiFi.

According to various examples, at least one sensor 130 is configured to detect detection signals 132 from the polony gel 106. The detection signals 132 are indicative of the analyte from the tissue sample 104 and the templates that the analyte is bound to within the polony gel 106. For example, the detection signal(s) 132 are indicative of the index sequence of an example template and a sequence of the analyte (e.g., RNA) that is bound to the example template. In particular cases, the detection signal(s) 132 are generated by a device and/or reaction used to perform NGS. For example, the detection signal(s) 132 include photons and/or images emitted from one or more sequencing-by-synthesis reactions performed on cDNA that includes sequences indicative of an index sequence of a template and of RNA attached to the template (e.g., a DNA sequence that is complementary to the RNA).

Although FIG. 1 illustrates that the detection signal(s) 132 are emitted from the housing 128, implementations are not so limited. In some cases, analytes (e.g., the cDNA) are extracted from the polony gel 106 (e.g., in solution). These analytes may be disposed in a vial, microfluidic device, or some other type of fluid receptacle. In some implementations, the analytes are mixed with various reagents to perform sequencing. According to various examples, the fluid receptacle is physically placed inside of a device that is configured to perform sequencing of the analytes in the receptacle. The detection signal(s) 132 are emitted from the fluid receptacle and are detected by the sensor(s) 130. The sensor(s) 130 may be part of the device itself.

At least one second computing device 134 may be configured to generate the spatial transcriptome 102 based on the detection signal(s) 132 detected by the sensor(s) 130. The second computing device(s) 134 may include at least one processor and memory storing instructions that, when executed by the at least one processor, cause the second computing device(s) 134 to perform various operations described herein. The second computing device(s) 134, for example, is communicatively coupled to the sensor(s) 130. In particular cases, the second computing device(s) 134 may recognize index sequences indicated in an example detection signal 132 based on the polony map 122. For example, the second computing device(s) 134 may identify the index sequence of the first template A13 based on the example detection signal 132. The second computing device(s) 134 may determine the location of the source of the detection signal by referring to the polony map 122. For example, the polony map 122 may indicate the position of the first polonies 110 that correspond to the first index sequence of the first template 114. In addition, the second computing device(s) 134 may identify the sequence of the analyte bound to the first template 114 based on the example detection signal 132. The second computing device(s) 134 may determine that a particular cell type corresponding to the analyte is overlapping the first polonies 110. The second computing device(s) 134 may indicate, in the spatial transcriptome 102, that the particular cell type is located in a position corresponding to the first polonies 110. In addition, the spatial transcriptome 102 may indicate the RNA expressed by the first cell type. Thus, the spatial transcriptome 102 indicates both the relative position and type of cells within the tissue sample 104.

According to some implementations, the second computing device(s) 134 may be further configured to estimate the cell boundaries of the cells within the tissue sample 104 based on the detection signal(s) 132 and the polony map 122. In various cases, the widths of the polonies on the surface of the polony gel 106 are significantly smaller than the cells of the tissue sample. The detection signal(s) 132 may indicate the index sequences of multiple polonies that overlap each cell within the tissue sample 104. By identifying the positions of multiple polonies overlapping an example cell within the tissue sample 104, the second computing device(s) 134 may estimate that the location of the cell overlaps the multiple polonies. In addition, the second computing device(s) 134 may estimate or otherwise detect an edge or boundary of the example cell by identifying neighboring polonies that are not bound to the mRNA from the example cell. Thus, various implementations described herein can further be used to identify the geometry of the cells within the tissue sample 104 without staining or imaging the cells. In some cases, the second computing device(s) 134 is configured to generate a spatial boundary map of the cells in the tissue sample 104. The spatial boundary map, for example, is a 2D image representing the boundaries of the cells in the tissue sample 104 on the surface of the polony gel 108.

Although FIG. 1 has been described using a nucleotide-based detection mechanism, implementations are not so limited. For example, proteins and their modifications in a tissue can be detected with DNA tagged antibodies, antibody fragments, nanobodies, monobodies, and nucleic acid aptamers, for example, when the target analyte is a protein rather than mRNA. Capture sequences can be added later by ligation and/or polymerase extension methods after cleavage.

FIGS. 2A to 2D illustrate example techniques for generating a spatial transcriptome using polonies. FIG. 2A illustrates an example of polonies of a polony gel 200. In particular, FIG. 2A illustrates first through sixth polonies 202-1 to 202-6 disposed on a surface of a base gel. The first through sixth polonies 202-1 to 202-6 may be generated using various techniques described herein. For example, the surface of the base gel may be seeded with multiple copies of primer pair. A solution including template precursors may be disposed on the surface of the base gel. The template precursors may individual include different index sequences. Individual template precursor molecules may attach to the primer on the surface of the base gel. Then, the attached template precursors may be amplified into the first through sixth polonies 202-1 to 202-6. Digestion can be performed to convert the template precursors into templates. Each of the first through sixth polonies 202-1 to 202-6 may include templates with a different index sequence. For example, the template in the first polony 202-1 may be different than the template in the second polony 202-2. The primers, template precursors, templates, or any combination thereof include DNA.

As shown in FIG. 2A, the first through sixth polonies 202-1 to 202-6 are continuous on the surface of the base gel. In some implementations, a distance between a first template in the first polony 202-1 and a template in the second polony 202-2 is no longer than a distance between the first template and a second template in the first polony 202-1.

FIG. 2B illustrates an example polony map 204 representing the polony gel 200. The polony map 204 includes an image. The image includes multiple pixels, which respectively depict areas on the surface of the polony gel 200. In various implementations, the polony map 204 includes first through sixth pixel sets 206-1 to 206-6 that respectively depict the first through sixth polonies 202-1 to 202-6.

The polony map 204 may be generated by sequencing the first through sixth polonies 202-1 to 202-6. In various implementations, sequencing-by-synthesis is performed on the polony gel 200. For example, at least one solution including DNA polymerase and fluorescently tagged deoxynucleoside triphosphates (dNTPs) is disposed on the surface of the polony gel 200. The DNA polymerase facilitates a synthesis reaction that generates new DNA strands that are at least partially complementary to the index sequences of the templates in the first to sixth polonies 202-1 to 202-6. Each time the DNA polymerase adds a fluorescently tagged dNTP to one of the new DNA strands, the dNTP emits a photon. At least one camera (e.g., including a fluorescent microscope) detects the photons emitted as the DNA strands are synthesized by capturing images of the polony gel 100. At least one computer may determine the sequences of the templates in the first to sixth polonies 202-1 to 202-5 by analyzing the images.

In addition, the computer(s) may segment the images into regions representing the first to sixth polonies 202-1 to 202-6 by analyzing the images. For example, a first region of pixels in the images of the polony gel 100 depicts photons consistent with a first index sequence and a second region of pixels in the images of the polony gel 100 depict photons consistent with a second index sequence. Accordingly, the computer(s) may define the first pixel set 206-1 corresponding to the first region of pixels and may define the second pixel set 206-2 corresponding to the second region of pixels. In various implementations, some of the pixels in the polony map 204 may overlap a boundary between neighboring polonies. Each individual pixel may be defined according to the polony with the highest intensity signal detected. For example, a first signal (e.g., a visual signal) detected from an area may correspond to a first index sequence, and a second signal (e.g., a visual signal) detected from the area may correspond to a second index sequence. If the intensity of the first signal exceeds that of the second signal, then the pixel corresponding to the area is defined as being part of the polony associated with the first index sequence..

In some implementations, the first through sixth pixel sets 206-1 to 206-6 are respectively defined according to different values. For examples, pixels in the first pixel set 206-1 have a first value, pixels in the second pixel set 206-2 have a second value, and so on. In some implementations, the values are colors in an RGB scale. In various implementations, the pixel map 204 further includes metadata that associates each value of the pixel map 204 with the corresponding index sequence. For instance, the metadata indicates that the first pixel set 206-1 is associated with the first index sequence of the first polony 202-1, that the second pixel set 206-2 is associated with the second index sequence of the second polony 202-2, and so on.

FIG. 2C illustrates an image 208 of a tissue sample that includes a first cell 210-1, a second cell 210-2, and a third cell 210-3. The first through third cells 210-1 to 210-3 originate from the same organism, but have different cell types. That is, the first cell 210-1 expresses first mRNA, the second cell 210-2 expresses second mRNA, and the third cell 210-3 expresses third mRNA.

In various implementations, the locations and types of the first to third cells 210-1 to 210-3 are determined using the polony gel 200 and the polony map 204. For instance, the tissue sample is disposed on the surface of the polony gel 200. The mRNA from the first to third cells 210-1 to 210-3 diffuses from the first to third cells 210-1 to 210-3 and onto the polony gel 200. The capture sequences of the templates in the first to sixth polonies 202-1 to 202-5 attach to the mRNA. In various cases, the templates and attached mRNA are sequenced. The locations and types of the first to third cells 210-1 to 210-3 are determined based on the resultant sequences.

FIG. 2D illustrates an example of a spatial transcriptome 212 of the tissue sample illustrated in FIG. 2C. The spatial transcriptome 212 includes an image including a first pixel set 214-1 corresponding to the first cell 210-1, the first and second polonies 202-1 and 202-2, and the first and second pixel sets 206-1 and 206-2 of the polony map 204. The image of the spatial transcriptome 212 further includes a second pixel set 214-2 corresponding to the second cell 210-2, the third and fourth polonies 202-3 and 202-4, and the third and fourth pixel sets 206-3 and 206-4 of the polony map 204. In addition, the image of the spatial transcriptome 212 includes a third pixel set 214-3 corresponding to the third cell 210-3, the fifth and sixth polonies 202-5 and 202-6, and the fifth and sixth pixel sets 206-5 and 206-6 of the polony map 204.

In various implementations, the computer(s) is configured to identify cells that overlap the first to sixth polonies 202-1 to 202-6. In some implementations, the computer(s) generate and/or otherwise identify sequencing data representing sequences of mRNAs from the cells 210-1 to 210-3 and the index sequences of the polonies 202-1 to 202-6 to which the mRNAs are bound. In some cases, cDNAs are generated based on the templates of the polonies 202-1 to 202-6 bound to the mRNAs. For example, the computer(s) identify sequences that reflect unique mRNA molecules output by the first to third cells 210-1 to 210-3 as well as the index sequences of the first to sixth polonies 202-1 to 202-6. The computer(s) identify one or more of the polonies 202-1 to 202-6 overlapping an individual one of the cells 210-1 to 210-3 by identifying one or more of the respective index sequences and the mRNA sequences output by the cells 210-1 to 210-3. The computer defines the areas of the polonies 202-1 to 202-6 on the polony gel 200 as cell areas based on the cells 210-1 to 210-3 whose mRNA sequences are identified with the corresponding index sequences of the polonies 202-1 to 202-6.

For example, the computer(s) may identify the index sequences of the first and second polonies 202-1 and 202-2 with a first mRNA sequence from the first cell 210-1; the index sequences of the third and fourth polonies 202-3 and 202-4 with a second mRNA sequence from the second cell; and the index sequences of the fifth and sixth polonies 202-5 and 202-6 with a third mRNA sequence from the third cell. This may be because the first mRNA from the first cell 210-1 has diffused into the first and second polonies 202-1 and 202-2; the second mRNA from the second cell 210-2 has diffused into the third and fourth polonies 202-3 and 202-4; and the third mRNA from the third cell 210-3 has diffused into the fifth and sixth polonies 202-5 and 202-6. By identifying which index sequences correspond to which mRNA sequences, the computer(s) may identify which cells overlap which polonies of the polony gel 200. Accordingly, the computer(s) may define the pixels corresponding to the first and second pixel sets 206-1 and 206-2 as the area where the first cell 210-1 is located; the pixels corresponding to the third and fourth pixel sets 206-3 and 206-4 as the area where the second cell 210-2 is located; and the pixels corresponding to the fifth and sixth pixel sets 206-5 and 206-6 as the area where the third cell 210-3 is located.

The first pixel set 214-1 represents the area of the first cell 210-1; the second pixel set 214-2 represents the area of the second cell 210-2; and the third pixel set 214-3 represents the area of the third cell 210-3. For instance, the first pixel set 214-1 has a first value corresponding to the first cell 210-1, the second pixel set 214-2 has a second value corresponding to the second cell 210-2, and the third pixel set 214-3 has a third value corresponding to the third cell 210-3. In various implementations, the spatial transcriptome 212 includes metadata that associates the first pixel set 214-1 with the mRNA from the first cell 210-1, associates the second pixel set 214-2 with the mRNA from the second cell 210-2, and associates the third pixel set 214-3 with the mRNA from the third cell 210-3.

In various implementations, multiple cells overlap a single polony. For instance, the first cell 210-1 and the second cell 210-2 both overlap the first polony 210-1. In these circumstances, sequencing data may indicate that the index sequence of the first polony 202-1 is associated with mRNA from the first cell 210-1 and the second cell 210-2. That is, the mRNA from the first cell 210-1 and the second cell 210-2 may bind to the same template of the first polony 202-1. The pixels associated with the first polony 202-1 will be assigned to either the first cell 210-1 or the second cell 210-2, depending on transcription similarity (also referred to as "transcript similarity") of its neighboring pixels. Transcription similarity is defined as the similarity between signals associated with neighboring areas on the polony map, wherein the signals correspond to the RNA sequences attached to templates in the neighboring areas. These signals, for instance, may correspond to the cDNA sequences that are indicative of the index sequences and the RNA sequences attached to the templates.

In some cases, the border of the cells overlapping a single polony are defined based on a reference image. The reference image indicates the structural boundaries of the first to third cells 210-1 to 210-3 in the tissue sample. For example, the first to third cells 210-1 to 210-3 are stained and imaged to generate the reference image. In some instances, the reference image is segmented to identify the boundaries of the first to third cells 210-1 to 210-3. The computer(s) labels the segmented first to third cells 210-1 to 210-3 based on the sequencing data. For instance, the computer(s) compares the polony map 204 to the reference image to identify that two neighboring cells overlap the first polony 202-1. The computer(s) labels the segmented cells based on the sequencing data.

In various implementations, however, the computer(s) labels the cells in the tissue sample without using a reference. That is, in some examples, the cells are accurately segmented without directly imaging the cells. In various cases, the computer(s) implement a volume- distance-based segmentation technique (also referred to as "V-seg") in order to segment the cells based on the sequencing data. Because the transcript level and profile tend to be constant in a cell body, transcripts found in neighboring polonies (or neighboring indices) with closer UMI densities and higher similarity are presumed to be more likely from the same cell. In various implementations, the computer(s) map the mRNA from the cells to the polonies in the polony map 204. The computer(s) construct a nearest neighbor network from the map with calculated edge weights (referred to as "volume distances") based on the spatial distance, the UMI densities, and the transcript similarity of two neighboring indices. In various cases, the boundaries of the cells are identified by segmenting the network. For instance, the computer(s) segment the network using a graph-based community detection with a fast greedy algorithm.

The spatial transcriptome 212 represents the identity and relative location of the cells in the tissue sample. Notably, the index sequences can be associated with the mRNA sequences using a sequencing technique that does not require imaging the tissue sample, as long as they are sequenced using a non-imaging method. Accordingly, various techniques described herein can be used to estimate an image of the cells 210-1 to 210-3 without conventional microscopy or direct imaging of the cells 210-1 to 210-3.

FIGS. 3A to CB illustrate examples of various constituents used to generate polony gels and to identify spatial transcriptomes. FIG. 3A illustrates a template precursor 300 to be attached to a primer 302 during polony gel fabrication. The primer 302 is seeded, attached, or otherwise disposed on a base gel 304. The primer 302, for example, includes DNA. In various cases, the primer 302 includes a modification 306 that binds the primer 302 to the base gel 304. For example, the modification 306 is a PS modification or an acrydite modification. In various instances, the modification 306 is on a 5' end of the primer 302. The primer 302 is attached to a surface of the base gel 304 via the modification 306. In various implementations, the base gel 304 is a crosslinked PAA gel. In various cases, the base gel 304 includes 3-20% acrylamide/bis, 0.01-1% ammonium persulfate, and 0.01-1% N,N,N',N'-TEMED. In some implementations in which the primer 302 is a PS modified primer, the base gel 304 includes 1-50 mg/ml BRAPA.

The template precursor 300 includes multiple constituent sequences, such as at least a binding sequence 308, an index sequence 310, a sequencing primer 312, a capture sequence 314, a cleavage site 316, and an amplification sequence 318. In various implementations, the template precursor 300 includes DNA.

The binding sequence 308 binds to the primer 302. In various implementations, the binding sequence 308 is disposed on a 5' end of the template precursor 300 and binds to the 3' end of the primer 302. For example, the binding sequence 308 includes a polony bridge primer forward sequence.

The index sequence 310 acts as a barcode for identifying the template precursor 300. In some examples, the index sequence 310 is in a range of 10-50 bases. For instance, the index sequence 310 has a length of 20-40 bases, such as 24 bases.

The sequencing primer 312 facilitates sequencing of the index sequence 310. In some examples, the sequencing primer 312 includes 25-50 bases. Sequencing by synthesis, for example, may be used to sequence the index sequence 310 and to generate the image 126 used to form polony map 122.

The capture sequence 314 binds an RNA of a tissue sample. In some implementations, the capture sequence 314 is nonspecific to the RNA. For instance, the capture sequence 314 is a poly-T sequence. In some cases, the capture sequence 314 is specific to the RNA. For example, the capture sequence 314 is complementary to at least a subset of the sequence of nucleotides in the RNA. That is, the capture sequence 314, in some cases, specifically binds the RNA. The capture sequence 314 is in a range of 15-30 BPs.

The cleavage site 316 includes bases that specifically bind an enzyme. The enzyme facilitates a digestion reaction that cleaves the template precursor 300 at the second cleavage site 316. In some cases, the enzyme is Taql, Xmal, BsaWI, BstBI, Dpnll, Xbal, and BspEl. In various implementations, cleavage of the template precursor 300 at the second cleavage site 316 exposes the capture sequence 314.

The amplification sequence 318 facilitates amplification efficiency of the template precursor 300 once the template precursor 300 is bound to the base gel 304.

FIG. 3B illustrates an example of a cleaved template 320 that captures an RNA 322. The template 322 includes at least some of the constituents of the template precursor 300, such as the first binding sequence 308, the index sequence 310, the sequencing primer 312, and the capture sequence 314.

In various implementations, the RNA 322 diffuses from a cell of a tissue sample. The RNA 322 binds to the capture sequence 314 of the cleaved template 320. After the RNA 322 is bound to the cleaved template 312, reverse transcription is performed on the RNA 322 bound to the cleaved template 320. For example, reverse transcriptase extends the cleaved template 320 to include a complementary to the RNA 322. The resultant cDNA is indicative of both the index sequence 310 and the sequence of the RNA 322. For instance, the cDNA includes the index sequence 310 and a sequence that is complementary to at least a portion of the RNA 322 (e.g., the portion of the RNA 322 that is not bound to the capture sequence 314). The cDNA is sequenced. In various implementations, the sequence of the RNA 322 and/or the index sequence 310 can be identified based on the sequence of the cDNA.

In various implementations, if the location of a polony including the template 320 is known, the location of the RNA 322 can be known based on the resultant sequencing data. In various implementations, the cell that is the source of the RNA 322 can be located based on the location of the polony.

FIGS. 4 to 6 illustrate example processes in accordance with various implementations of the present disclosure. Although FIGS. 4 to 6 illustrate specific orders of steps within these processes, implementations are not limited to the orders illustrated in FIGS. 4 to 6.

FIG. 4 illustrates an example process 400 for synthesizing a polony gel. The process 400 may be performed by an entity such as a machine (e.g., a microfluidic device that includes at least one pump and at least one reservoir containing at least one reagent).

At 402, the entity generates a base gel. In various implementations, the base gel is generated by polymerizing acrylamide. In some cases, the base gel is generated by polymerizing acrylamide/bis in the presence of ammonium persulfate, TEMED, BRAPA, or any combination thereof. In various cases, the acrylamide is polymerized in an anaerobic environment, such as in the presence of less than 1,000 ppm of oxygen. According to some examples, the polymerization is performed at a temperature of 0-30° C and/or at a humidity of 0-60%. The resultant base gel, according to various implementations, is a crosslinked PAA gel. The crosslinked PAA gel, for example, includes 3-20% acrylamide/bis, 0.01-1% ammonium persulfate, and 0.01-1% TEMED. The methods of the invention utilise a crosslinked polyacrylamide (PAA) gel comprising 3-20% acrylamide/bis, 0.01-1% ammonium persulfate, and 0.01-1% N,N,N',N'-tetramethylethylenediamine (TEMED). The polony gels for single cell RNA seqeuncing as claimed comprise a hydrogel comprising crosslinked PAA; a first polony on a surface of the hydrogel, the first polony comprising a first template that comprises a first capture sequence and a first index sequence; and a second polony on the surface of the hydrogel, the second polony bordering the first polony and comprising a second template that comprises a second capture sequence and a second index sequence.

At 404, the entity attaches primers to the base gel. In various implementations, the primers include DNA. In some cases, the primers include a modification that facilitates binding the primers to the base gel. The modification, for example, is a PS modification or an acrydite modification. In various implementations in which the base gel includes BRAPA, the modification is a PS modification. In various implementations, the primers are attached when a solution including the primers is disposed on a surface of the base gel.

At 406, the entity attaches template precursors to the primers. In various implementations, the template precursors include index sequences and capture sequences. The index sequence of an example template precursor (e.g., each template precursor) includes an individual barcode represented by multiple nucleotides. For example, the index sequences of the template precursors include 10-50 BP. In various implementations, the capture sequences are nonspecific (e.g., a poly-T probe) or specific (e.g., a sequence that specifically binds a target RNA). According to some cases, the template precursors further include sequencing primers that can be used to sequence the index sequence in order to generate one or more images (e.g., image 126) indicative of polonies that are grown on the surface of the base gel.

At 408, the entity generates polonies by amplifying the template precursors. According to various cases, each polony includes 10-1,000,000 copies of an example template precursor. The resultant polonies have widths that are significantly narrower than the width of a cell. For example, an example polony has a width of 0.1 to one µm.

At 410, the entity performs digestion to expose capture sequences and the sequencing primer site in the template precursors. For example, the template precursors include digestion sites that are adjacent to the capture sequences. For instance, an enzyme is introduced to the polonies and specifically binds an example digestion site in an example template precursor. The enzyme cleaves the template precursor, leaving a fragment of the template precursor bound to the primer. The capture sequence is disposed at a 3' end of the cleaved template.

Optionally, the same or different entity (e.g., a computer) generates a polony map of the polony gel. In some cases, the cleaved templates are sequenced using sequencing-by-synthesis. At least one camera obtains images of the polony gel as new DNA is synthesized using fluorescently-tagged nucleotides. Based on the images, the entity identifies the locations of the polonies on the polony gel. In addition, the entity identifies the index sequences of the templates in the polonies. The polony map indicates the locations and index sequences of the polonies on the polony gel.

FIG. 5 illustrates an example process 500 for generating a spatial transcriptome using a polony gel. The process 500 is performed by an entity, which may include a computer and/or processor.

At 502, the entity identifies a polony map. In various implementations, the polony map represents the locations and/or identities of polonies distributed on the surface of a polony gel. The polonies are continuously distributed on the surface of the polony gel, in various implementations. In some cases, the polony map includes an image that includes groups of pixels whose values indicate the shapes and locations of polonies on the surface of the polony gel. In various examples, the polony map indicates index sequences of templates that are included in the polonies.

At 504, the entity introduces a polony gel to a tissue sample. The tissue sample includes multiple cells. In various cases, the tissue sample is a cryosectioned portion of a multicellular organism. The tissue sample is disposed on the surface of the polony gel. Accordingly, RNA from the cells of the tissue sample diffuse from the cells onto the polonies. In various implementations, the templates include capture sequences that bind to the RNA from the cells.

At 506, the entity performs in situ cDNA synthesis on the RNAs of the tissue sample and indices of the polony gel. In various implementations, the sequences of the generated cDNA indicate the index sequences in the templates and/or the sequences of the RNA from the cells.

At 508, the entity sequences the cDNAs. In various implementations, the entity identifies the sequences of nucleotides in the resultant cDNAs. Various sequencing techniques can be used to identify the sequences of the cDNAs. The entity generates sequencing data that indicates the sequences of the cDNAs.

At 510, the entity generates a spatial transcriptome by comparing the polony map to the sequenced cDNAs. In various implementations, the entity identifies index sequences indicated by the cDNAs. In addition, the entity identifies RNA sequences indicated by the cDNAs. The entity, according to some examples, identifies the cells that overlap the polonies based on the sequencing data. In various cases, the entity generates a spatial transcriptome based on the polony map and the sequencing data. The spatial transcriptome, for instance, indicates the locations and/or identities of cells within the tissue sample. According to various implementations, the entity segments the cells based on the sequencing data and the polony map.

FIG. 6 illustrates an example process 600 for segmenting cells based on sequencing data representing mRNA from the cells bound to templates of a polony gel. The process 600 is performed by an entity, which may include a computer and/or processor.

At 602, the entity identifies a polony map. In various implementations, the polony map includes an image representing the areas of individual polonies distributed on the surface of a polony gel. In some cases, the polony map includes additional data (e.g., metadata) indicating index sequences associated with the respective polonies. For example, the polonies are defined by multiple copies of the same template (e.g., a cleaved template). Each template may include a respective index sequence. The polony map indicates the location and/or index sequence of at least one polony (e.g., each polony) of the polony gel, according to various cases.

At 604, the entity identifies sequencing data. The sequencing data may represent the sequences of cDNA generated by exposing the polony gel to a tissue sample. In some implementations, a cryosectioned portion of a tissue of a multicellular organism is placed on the surface of the polony gel. Various mRNA from the cells of the tissue sample diffuse onto the surface of the polony gel, where the polonies are disposed. Capture sequences of the templates of the polonies bind the mRNA. Subsequently, cDNA is generated using the mRNA captured on the templates. In various cases, the cDNA sequences are indicative of the index sequences and the mRNA, such that the sequencing data indicates what mRNA sequences are bound to what templates of the polony gel. The sequencing data is generated by sequencing the cDNA sequences.

At 606, the entity generates a network based on the polony map and the sequencing data. In various implementations, the entity identifies index sequences in the polony map and the sequencing data. The network is an undirected spatial network of the identified index sequences as nodes. In some implementations, the entity generates a nearest neighbor network. According to some implementations, the entity determines edge weights of the network using the spatial distance and transcript similarity (e.g., mRNA sequence similarity) between pairs of neighboring index sequence nodes.

At 608, the entity identifies boundaries of the cells by segmenting the network. For example, the entity aggregates transcripts (e.g., mRNA sequences) into representations of single cells in the tissue sample using a segmentation technique. According to various implementations, the entity uses graph-based detection to segment the representations of the cells. For example, the entity uses fast greedy segmentation. In some cases, the entity generates a cell-gene matrix based on the results of the segmentation. In various implementations, the entity generates a cell map based on the matrix. For example, the cell map is an image whose pixels indicate the areas and boundaries of cells in the tissue sample as they are distributed on the surface of the polony gel. According to some implementations, the entity generates a spatial transcriptome based on the matrix. The spatial transcriptome, for instance, indicates the locations (e.g., areas and boundaries of cells in the tissue sample as they are distributed on the surface of the polony gel) as well as the identities of the cells. For example, the spatial transcriptome indicates the types of individual cells in the tissue sample based on the sequences of the mRNA that is released from the cells and used to generate the sequencing data.

FIG. 7 illustrates an example of a system 700 for performing various functions described herein. The system 700 includes any of memory 704, processor(s) 706, removable storage 708, non-removable storage 710, input device(s) 712, output device(s) 714, and transceiver(s) 716. The system 700 may be configured to perform various methods and functions disclosed herein.

The memory 704 may include component(s) 718. The component(s) 718 may include at least one of instruction(s), program(s), database(s), software, operating system(s), etc. In some implementations, the component(s) 718 include instructions that are executed by processor(s) 706 and/or other components of the system 700.

In some implementations, the processor(s) 706 include a central processing unit (CPU), a graphics processing unit (GPU), or both CPU and GPU, or other processing unit or component known in the art.

The system 700 may also include additional data storage devices (removable and/or non-removable) such as, for example, magnetic disks, optical disks, or tape. Such additional storage is illustrated in FIG. 7 by removable storage 708 and non-removable storage 710. Tangible computer-readable media can include volatile and non-volatile, removable and non-removable media implemented in any method or technology for storage of information, such as computer readable instructions, data structures, program modules, or other data. The memory 704, the removable storage 708, and the non-removable storage 710 are all examples of computer-readable storage media. Computer-readable storage media include, but are not limited to, Random Access Memory (RAM), Read-Only Memory (ROM), Electrically Erasable Programmable Read-Only Memory (EEPROM), flash memory, or other memory technology, Compact Disk Read-Only Memory (CD-ROM), Digital Versatile Discs (DVDs), Content-Addressable Memory (CAM), or other optical storage, magnetic cassettes, magnetic tape, magnetic disk storage or other magnetic storage devices, or any other medium which can be used to store the desired information and which can be accessed by the system 700. Any such tangible computer-readable media can be part of the system 700.

The system 700 may be configured to communicate over a telecommunications network using any common wireless (e.g., BLUETOOTH; WI-FI; ZIGBEE; 3^{rd} Generation Partnership Project (3GPP) network, such as Long Term Evolution (LTE) and/or New Radio (NR); etc.) and/or wired (e.g., Ethernet, universal serial bus (USB), etc.) network access technology. Moreover, the system 700 may be configured to run any compatible device Operating System (OS), including but not limited to, Microsoft Windows Mobile, Google Android, Apple iOS, Linux Mobile, as well as any other common mobile device OS.

The system 700 also can include input device(s) 712, such as a keypad, one or more buttons, a cursor control, a touch-sensitive display, voice input device, camera, etc., and output device(s) 714 such as a display, speakers, printers, etc. For instance, the input device(s) 712 may include a camera (e.g., including a fluorescent microscope) configured to capture images of a polony gel during sequencing, and the processor 706 may generate a polony map based on the images. In some implementations, the input device(s) 712 include some other type of sensor configured to detect signals indicative of the sequences of cDNA generated based on analytes from a tissue sample binding to polonies of a polony gel. In some cases, the output device(s) 714 include a display configured to visually output at least one of a polony map, sequencing data, or a spatial transcriptome.

As illustrated in FIG. 7, the system 700 also includes one or more wired or wireless transceiver(s) 716. For example, the transceiver(s) 716 can include a network interface card (NIC), a network adapter, a Local Area Network (LAN) adapter, or a physical, virtual, or logical address to connect to various network components, for example. To increase throughput when exchanging wireless data, the transceiver(s) 716 can utilize multiple-input/multiple-output (MIMO) technology. The transceiver(s) 716 can comprise any sort of wireless transceivers capable of engaging in wireless (e.g., radio frequency (RF)) communication. The transceiver(s) 716 can also include other wireless modems, such as a modem for engaging in WI-FI, WiMAX, BLUETOOTH, infrared communication, and the like. The transceiver(s) 716 may include transmitter(s), receiver(s), or both. Such systems may be useful in the methods of the invention.

FIG. 8 illustrates a table comparing example spatial transcriptomics techniques to other reported methods. Compared with other oligo arrays used by other reported methods (P.L. Stahl et al. (2016); S.G. Rodriques et al. (2019); S. Vickovic et al. (2019)), the feature size in various implementations of the present disclosure decreases by 3 to 200 folds (FIG. 13). The decreased feature size offers the sufficient resolution for single cell segmentation for mammalian tissues.

FIG. 9 illustrates an example of an anticipated benefit of PAA-gel on in-situ cDNA synthesis. FIG. 9 illustrates that PAA gels may efficiently capture RNA and slow down RNA diffusion. Gels on other types of substrates, in contrast, have relatively fast diffusion rates in solution. FIG. 10 illustrates an example structural difference of a linear PAA-gel and a crosslinked PAA-gel. Various results described herein suggest that the PAA substrate can constrain the RNA diffusion to preserve the spatial resolution (FIG. 9). Additionally, the crosslinked PAA gave a 17.6-fold higher cDNA yield than the linear PAA, probably due to a higher oligo accessibility in the crosslinked gel (FIG. 10).

FIG. 11 illustrates an example of a spatial distribution of cDNA read density per polony (1 pixel = 0.325 * 0.325 um). FIG. 11 also illustrates a close visualization of cDNA density distribution showing a subcellular resolution (right panel). Guided by the predetermined spatial index map, sequencing reads were constructed into a digital spatial transcriptome image at subcellular resolution (FIG. 11). Each segmented cell was estimated having 50 clustered indices.

### EXPERIMENTAL EXAMPLES

Particular techniques for spatial barcoding, which reliably achieve the resolution and transcript capture efficiency of the dissociative scRNA-seq, will now be described. These techniques use "continuous" polony gels with evenly distributed, ultrahigh-density oligos of ≤ 1-µm feature sizes and/or an efficient segmentation algorithm to precisely aggregate mapped transcripts into single cells. Experimental Example 1 describes second techniques for generating polony gels as well as additional analysis of the polony gels. For example, in Experimental Example 1, spatial, single-cell analysis as demonstrated by mapping the mouse main olfactory bulb (MOB) without scRNA-seq data-guided annotation. In addition, a three-dimensional (3D) atlas of the parabrachial nucleus (PBN), a critical brain region for relaying sensory information to forebrain structures, was further generated. Notably, the PBN is difficult to analyze with dissociative or other spatial transcriptomics assays. The data from Experimental Example 1 revealed subnucleus-specific distributions of previously known and unknown PBN neurons. Beyond mapping the brain structure, Experimental Example 1 also an analysis of neuropathic pain-regulated gene expression in the PBN demonstrates a wide array of region and cell-type specific changes. Experimental Example 1 further provides an analysis of cell-cell communication and glial transcriptomic dynamics suggested an unexpected protective mechanism in the homeostatic adult brain adapted to the pain condition. Together, these examples underpin the importance of the non-dissociative scRNA-seq for studying structural and functional heterogeneity in complex tissues. Experimental Example 2 describes techniques for generating polony gels and generating libraries using the polony gels.

### EXPERIMENTAL EXAMPLE 1

This Experimental Example described herein demonstrate non-dissociative scRNA-seq by translating the resolution and sensitivity of the spatial transcriptomics techniques according to various implementations of this disclosure into the single-cell assay on brain tissues. In various implementations, the success of this Experimental Example can be attributed to the synthesis of continuous polony gels. In addition, the success of these Experimental Examples can be attributed to the positioning of transcript reads to single cells with high accuracy. Further, the techniques described herein can be used to generate quick, affordable spatial transcriptomes. After gel fabrication, the assay from tissue sectioning to sequencing library construction takes about 6 hours with an affordable protocol for implementation in standard-equipped laboratories (FIG. 40 and Supplementary Table 7). To make the assay widely accessible, the gel fabrication is scalable and compatible with commercial sequencing platforms. Like other spatial barcoding assays (e.g., Liu, Y. et al., Cell 183, 1665-81 (2020); Vickovic, S. et al. SM-Omics: An automated platform for high-throughput spatial multi-omics, Preprint at https://www.biorxiv.org/content/10.1101/2020.10.14.338418v1 (2021)), these techniques can also be applicable to protein detection with DNA-tagged antibodies and possibly small-molecule analytes via affinity reagent innovation (Kang, S. et al., J. Am. Chem. Soc. 141, 10948-52 (2019)).

In this Experimental Example, various subcellular transcript distributions in brain cells (FIG. 41) were observed. The ≤ 1-µm spatial resolution of various techniques described herein can reveal subcellular heterogeneity, such as protein localization, interaction, and modification.

Pain, a multidimensional experience, involves sensory, affective, and cognitive components in the periphery and brain. So far, the single-cell transcriptomics of pain-induced changes has been limited to sensory tissues, such as dorsal root ganglion (Kupari, J. et al., Nat. Commun. 12, 1510 (2021)), but cells and responses in other components in pain processing are largely unknown. Despite the importance in elucidating pain mechanisms and developing new analgesics, research in this field has been hampered by the lack of suitable tools. In this regard, the techniques described herein addresses long-standing needs. The single-cell PBN atlas and the unveiled pain-regulated changes in the transcriptome provide a basis for future mechanistic studies on the PBN's roles in affective motivational and sensory discriminative pathways of pain and other processes. Although limited samples were analyzed, the unusual heterogeneity revealed by example spatial transcriptomics techniques highlights the necessity to analyze more anatomical positions at different time points to develop a complete view of the structural and functional landscape.

### Fabrication of continuous polony gels with high resolution and RNA capture efficiency

A critical need to achieve non-dissociative scRNA-seq is to maximize the resolution and sensitivity of tissue transcript capturing on an oligo array. Arrays made from the commercial flowcells were optimized for DNA sequencing but not necessarily for tissue mapping. Their specialized fabrication methods restrict the innovation by other developers. In this Experimental Example, polony-derived oligo arrays were synthesized on a crosslinked polyacrylamide (PAA) gel surface (hereafter named "polony gel") particularly designed for spatial barcoding applications. By screening gel fabrication conditions, it was experimentally determined that polonies on specific crosslinked gel surfaces show relatively fast size expansion to form a continuous, homogenous DNA distribution with minimal feature-to-feature gaps, which is distinct from those amplified in other oligo arrays (e.g., sequencing flowcells using a linear PAA gel) that show a discrete, peakshape distribution (FIG. 12b (left) and FIG. 17a).

The continuous polonies described herein are ideally suited for tissue mapping by minimizing the bias caused by unevenly distributed capturing probes. Additionally, these polonies appear to be easily accessible to restriction digestion; 93.6% of double-stranded DNAs were digested by Taql to expose 3' poly-T for transcript capturing (FIG. 17b). Although the polonies are connected, they rarely interpenetrate due to a polony exclusion effect (Aach, J. & Church, G. M., J. Theor. Biol. 228, 31-46 (2004)), so their borders can be delineated by the sequencing (FIG. 12b (middle)). Because polonies show variable sizes and shapes, to maximize the feature resolution, a base-calling pipeline was developed that determines the major index species in each image pixel (0.325 × 0.325 µm2) to construct a pixel-level spatial index map (also referred to as a "polony map") (FIG. 12b (right) and FIG. 13).

The gels in this Experimental Example were fabricated with an adaptable protocol using off-the-shelf reagents, materials, and equipment. Gels with scalable sizes (e.g., 6 × 30 mm²) were cast on a coverslip and assembled into a flowcell for polony amplification and sequencing, similar to previous work described in Gu, L. et al., Nature 30 515, 554-57 (2014). The polony amplification was highly efficient, yielding an average of 20,337 template copies per polony (or feature) after amplification cycles (FIG. 12c and FIG. 19), which is a 9-fold increase from a method described in Bentley, D. R. et al., Nature 456, 53-59 (2008) using a linear PAA gel. This process produced an oligo density of 1.74 × 104 molecules/µm², which is close to that spotted on a glass substrate (Stahl, P. L. et al., Science 353, 78-82 (2016)). Polonies were mapped by sequencing 24-bp spatial indices using sequencing-by-synthesis chemistry (described in Bentley, D. R. et al., Nature 456, 53-59 (2008)) with an epifluorescence microscope equipped with a fluidic system. With the imaging setup, gels with 0.5 to 0.8 million polonies per mm² passing filter were reliably fabricated, which is close to that of a nonpatterned HiSeq flowcell. Using the techniques described herein, continuous polonies reached higher densities with clear boundaries (FIG. 20) and sequencing them required improved imaging resolution.

In addition, the polony gels were analyzed using mouse olfactory bulb-isocortex sections. To quickly assess the resolution and sensitivity of capturing global transcripts, spatially indexed cDNAs synthesized on the gel were imaged. The protocol included attaching 10-µm thick frozen sections to dried gels for hybridizing mRNAs and cDNA synthesis. Even without tissue fixation, Cy5- labelled cDNAs in a mouse olfactory bulb-isocortex section showed clear single-cell resolution (FIG. 12D). The diffusion was measured by registering a nuclear stained mouse MOB image to the synthesized cDNA signals; a scaling factor was determined to be 1.028 ± 0.016 (FIG. 21). The results demonstrated that the porous gel structure can constrain transcript lateral diffusion to preserve single-cell or even subcellular resolution.

FIG. 19 illustrates an example of an anticipated benefit of PAA-gel on in-situ cDNA synthesis. FIG. 19 illustrates that PAA gels may efficiently capture RNA and slow down RNA diffusion. Gels on other types of substrates, in contrast, have relatively fast diffusion rates in solution. FIG. 20 illustrates an example structural difference of a linear PAA-gel and a crosslinked PAA-gel. Various results described herein suggest that the PAA substrate can constrain the RNA diffusion to preserve the spatial resolution (FIG. 19).

Next, cDNA yields on polony gels and linear PAA gels (fabricated in accordance with methods described in Bentley, D. R. et al., Nature 456, 53-59 (2008)) were compared. A 17.6-fold higher Cy5 signal was detected on polony gels (FIG. 22), likely due to the higher probe density and accessibility on the crosslinked gel surface.

### Demonstration of the non-dissociative scRNA-seq on the MOB

As a proof-of-principle, the mouse MOB was assayed with a typical layered structure of the brain previously used for testing spatial transcriptomics technologies (Stahl, P. L. et al., Science 353, 78-82 (2016); Vickovic, S. et al., Nat. Methods 16, 987-90 (2019); Stickels, R. R. et al., Nat. Biotechnol. 39, 313-19 (2021); Chen, A. et al. Large field of view-spatially resolved transcriptomics at nanoscale resolution, Preprint at https://www.biorxiv.org/content/10.1101/2021.01.17.427004v2 (2021)). To assess reproducibility, 10-µm coronal sections were attached to different gels to map the global transcriptome. For example, 83% of raw reads were mapped to the gels to obtain 82.5 million unique molecular identifiers (UMIs) located to a 13-mm² MOB region with read densities from 1 to 678 UMIs per spatial index. UMI density maps showed a continuous, multi-layered MOB structure like the Cy5-labelled cDNA image (FIG. 13a). About 23,000 unique genes were detected with over 10 UMIs in at least one of three replicates; the data showed high correlation (R ≥ 0.968; FIG. 23a). To demonstrate the improvements of the techniques described herein, the results were compared with the most recent mouse MOB data from other spatial transcriptomics assays (Visium (Lebrigand, K. et al., The spatial landscape of gene expression isoforms in tissue sections. Preprint at https://www.biorxiv.org/content/10.1101/2020.08.24.252296v1 (2020)), Slide-seqV2 (Stickels, R. R. et al., Nat. Biotechnol. 39, 313-19 (2021)), HDST (Vickovic, S. et al., Nat. Methods 16, 987-90 (2019)), and Stereo-seq (Chen, A. et al. Large field of view-spatially resolved transcriptomics at nanoscale resolution, Preprint at https://www.biorxiv.org/content/10.1101/2021.01.17.427004v2 (2021))). With a sequencing depth of 80%, example spatial transcriptomics techniques detected a mean of 47, 977, and 19,975 UMIs per bin of 2 × 2, 10 × 10, and 50 × 50 µm², respectively, in the whole MOB area (FIG. 23b). Significant higher UMIs were found within specific layers; for example, a mean of 83, 1,695, and 37,037 UMIs per bin of 2 × 2, 10 × 10, and 50 × 50 µm², respectively, in a mitral cell layer (MCL) with densely populated cell bodies. At the resolutions comparable to array feature sizes of other assays, even without considering feature-to-feature gaps, the techniques described herein detected ≥ 6.4, 23.4, 2.1, and 1.3-fold more UMIs than Stereo-seq, HDST, Slide-seqV2, and Visium, respectively. Detected MOB layer-specific gene expression patterns agree with in situ hybridization (ISH) data from the Allen Mouse Brain Atlas (AMBA) (FIG. 23c).

OLD FIG. 23 illustrates an example of a spatial distribution of cDNA read density per polony (1 pixel = 0.325 * 0.325 um). FIG. 23 also illustrates a close visualization of cDNA density distribution showing a subcellular resolution (right panel).

In this Experimental Example, the efficacy of a novel cell segmentation technique was also demonstrated. The high-quality transcript map was used to segment mapped transcripts into single cells. Simulations demonstrated that ≤ 1 µm polonies offer a sufficient resolution to delineate mammalian cell boundaries; for example, a simulation shows that, if an array is covered by a seqFISH-mapped mouse cortex region with an average cell diameter of 15.9 ± 4 µm (Eng, C. H. L. et al., 45 Nature 568, 235-239 (2019)), the percentage of features contacting only one cell increases from 50.3 to 96.3% when the feature diameter decreases from 10 to 1 µm (FIG. 24). The transcript segmentation could be guided by a "reference" image, a stained cellular structure image of the same tissue (Cho, C. S. et al., Cell 184, 3559-3572 (2021)). However, it is challenging to apply it to brain cells which are morphologically and densely intertwined. Additionally, the reference image and example spatial transcriptomics techniques data have to be acquired in different assay steps, imposing a further challenge on aligning the data. Thus, a volume- distance-based segmentation technique ("V-seg") was developed and implemented in R (FIG. 13b and FIG. 25). Because the transcript level and profile tend to be constant in a cell body (Padovan-Merhar, O. et al., Mol. Cell58, 339-352 (2015)), transcripts found in neighboring indices with closer UMI densities and higher similarity are more likely from the same cell.

The segmentation technique used in this Experimental Example includes constructing a nearest neighbor network from mapped transcripts with calculated edge weights, termed volume distances, based on the spatial distance, the UMI densities, and the transcript similarity of two neighboring indices. The network is segmented via a graph-based community detection with a fast greedy algorithm. The technique was tested to be computationally efficient; 2,000 cells can be processed per minute.

In the case of the MOB in FIG. 13, the edge threshold was the weight < 3 and the spatial transcript map was divided to images of 800 × 800 pixels (1 pixel= 0.325 µm) for individual processing before stitching them together. The segmentation technique described in in this Experimental Example was also performed using the PBN data. In the case of the PBN 4, the segmentation was performed in two steps to separately process aggregated Calca cells and other cell types. Step 1: the edge threshold was the weight < 4 and the transcript map was divided to images of 600 × 600 pixels. Segmented cells were clustered by Seurat to separate Calca cells. Step 2: Other cells were re- segmented using the edge threshold of the weight < 4 and the image size of 800 × 800 pixels.

The MOB provided a rigorous test for the segmentation since it includes highly diverse structures, densities, and morphologies of cells surrounded by neuropil which also contains mRNAs for local protein synthesis. Cryosectioning creates different cutting sites even for similar cells residing in different orientations and tissue depths, resulting in different amounts of RNAs accessible to the gel. In the mapped MOB section, the segmentation technique was used to segment 86% (70.8 million) mapped transcripts into 23,351 detected cells; 22,830 with UMIs ≥ 256 were selected for cell classification. Unsupervised clustering (Hao, Y. et al., Cell 184, 3573-3587 (2020)) recapitulated layer-specific distributions of classical neuronal and non-neuronal cell types (FIG. 13c). To validate the unsupervised cell classification, it was compared with that guided with a dissociative scRNA-seq dataset (Tepe, B. et al., Cell Rep. 25, 2689-2703 (2018)). The unsupervised and supervised results showed a high agreement (FIG. 13d (top two) and FIG. 26). By comparison, the random segmentation using a regular bin (10 × 10 µm²) comparable to V-seg-detected cell sizes (equal to squares of (10.3 ± 3.9)² µm²) correctly identified major cell types with the scRNA-seq data-guided annotation, but largely failed with the unsupervised classification (FIG. 13d (bottom two)). The improved segmentation by V-seg was confirmed by the consistency between the output cell boundaries and the UMI density and marker gene distributions (FIG. 13e).

It is possible that V-seg aggregates transcripts from multiple cells or divides one cell into multiple factions. To assess the under- or over-segmentation, average diameters of segmented cells were measured to be 8.9 ± 3.5 to 14.5 ± 4.8 µm (FIG. 13f (top)), reasonable sizes for cell bodies in the MOB (Pinching, A. J. & Powell, T. P. S., J. Cell Sci. 9, 305-45 (1971)). Segmented cells showed cell-type specific UMI densities (FIG. 13f (bottom)), consistent with the layer-specific UMI density distributions (FIG. 13a). As expected, UMI counts increase with cell sizes, for example, the means of periglomerular type 1 (PGC-1; 10.9 ± 4.6 µm) and mitral/tufted cells (M/TCs; 14.5 ± 4.8 µm) are 3,346 and 6,458 UMls/cell, respectively (FIG. 13g). The relative abundances of major cell types detected by example spatial transcriptomics techniques and dissociative scRNA-seq; the rankings were similar but with differences, e.g., the most abundant cells, granule cells (GCs), detected by example spatial transcriptomics techniques ranked second by scRNA-seq (FIG. 13h). This discrepancy is reasonable because example spatial transcriptomics techniques analyzed only a section of the MOB and during tissue dissociation different cell types have different capture rates.

### 3D cell atlas of the mouse PBN

In addition, the polony gels described in this Experimental Example was used to construct a 3D cell atlas of the mouse PBN. The PBN is difficult to analyze without single-cell resolution. The PBN located in the pons was selected for analysis because it has important functions for relaying sensory information from the periphery to the forebrain, responding to internal and external stimuli, as well as maintaining homeostasis(Palmiter, R. D., Trends Neurosci. 41, 280-293 (2018)). Previous studies using unique genetic markers located neurons within the PBN that transmit distinct signals related to thermal sensation (Norris, A. J. et al, eLife 10, e60779 (2021)), pain (Huang, T. W. et al., Nature 565, 86-90 (2019)), and appetite, visceral malaise, and threat detection (Campos, C. A. et al., Nature 555, 617-22 (2018)). However, the identity of most cells in the PBN and their spatial organization were unknown.

PBN coronal sections were analyzed in the middle (Bregma, -5.35 mm) with the largest cross section. With a sequencing depth of 88%, each section generated 21 ± 4.5 million UMIs located to a 3 × 3 mm² region centered on the PBN surrounded by the cerebellar cortex, trigeminal motor (V) and principal sensory nuclei, locus ceruleus, and cuneiform nucleus (FIG. 14a). The UMI density map was used to chart PBN subregions such as the lateral (PBNI) and medial (PBNm) divided by the superior cerebellar peduncle (scp), a large fiber tract showing distinctly fewer UMIs. Mapped transcripts were aggregated into 15,618 ± 1,093 cells per section. Unsupervised clustering by Seurat defined distinctive marker genes (FIG. 14b); markers were compared to the consensus in mousebrain.org (Zeisel, A. et al., Cell 174, 999-14 (2018)) to identify 21 neuronal and non-neuronal cell types (FIG. 14c and Supplementary Table 1). Further subclustering of neurons identified 18 subtypes (FIG. 14d and Supplementary Table 2).

To assess the robustness of the clustering, the spatial patterns of clustered cells were compared to the AMBA anatomic reference (Extended Data FIGS.11,12). Most of the clustered cells exhibit region-specific distributions correlated to anatomical structures of the PBN and surrounding regions (Extended Data FIGS.13,14). For example, 81.0% and 53.2% of clustered Calca/Nts+ and Tac1+ neuron subtypes were found in different subregions of the PBNI (FIG. 14e), consistent with previous reports (Campos, C. A. et al., Nature 555, 617-22 (2018); Barik, A. et al., Neuron 100, 1491-03 (2018)). The Calca+ neurons in the PBN and the trigeminal region were separated by differentially expressed markers (e.g., Sncg); the latter were correctly segmented from intertwined trigeminal motor neurons (Sncg/Uchl1+) in the same region. Additionally, two previously unknown PBN neurons were identified: the Resp18/Ctxn2+ subtype in the PBNI's dorsal and ventral subnuclei, the Resp18/Sst+ in the central subnucleus, and both were also in the PBNm. Their locations overlap with areas involved in taste-guided behavior (Jarvie, B. C. et al., Nat. Commun. 12, 224 (2021)). Some non- neuronal cells also show region specificity; for example, the most abundant astrocyte subtype, Astro1, was enriched in the PBN and the neighboring pontine central gray region (Extended Data FIGS.12a,13).

To study the 3D heterogeneity, the anterior, middle, and posterior sections of the same PBN sample (Bregma, -5.20, -5.35, and -5.50 mm, respectively) were analyzed. Distinct changes along the rostral-caudal extent of the PBN were observed for distributions of several neuropeptide-expressing genes (FIG. 31), implying transcriptomic and anatomical heterogeneity. The two known neuron subtypes in the same subnucleus, Calca/Nts+ and Tac1+, were selected as a focus of the analysis. The clustering, as well as the marker gene distributions, revealed their 3D organization: the Tac1+ cells are densely populated in the anterior position and surround the Calca/Nts+ in the middle position, and both are mixed in the posterior position (FIG. 14f).

Because the distance between cells affects their communication, direct cell contacts were measured in the PBN atlas. Adjacent cells were quantified using a pair cross-correlation function (PCCF) statistic (Philimonenko, A. A. et al., J. Struct. Biol. 132, 201-10 (2000)) to compare detected cell contacts (or colocalization) between the same or different subtypes with the probability of the random colocalization. The high colocalization between the same cell types agrees with the observed cell aggregations; for example, the Purkinje (Pcp2/Pvalb+) and Bergmann (Timp4/Aldoc+) cells in the cerebellum and CGRP-expressing neurons (Calca/Nts+) in the PBN (FIG. 14g and FIG. 30). High neuron-neuron contacts were found for the Calca/Nts+ and Tac1+ in the PBNI and the Calca/Sncg+ and Sncg/Uchl1+ in the trigeminal. Typically, cells showing region-specific distributions were found with preferential contacts with specific neurons or non-neuronal cells.

### Cell type- and subnucleus-specific mRNA changes in response to chronic pain

Cell type- and subnucleus-specific mRNA changes in response to chronic pain were analyzed in this Experimental Example. The polony gels were used to detect changes in gene expression in response to stimuli. The precise analysis of activity-triggered adaptations in specific cells requires comparison of functionally identical cells (e.g., the same type of cells in identical brain regions with similar connectivity) from different animals. To demonstrate the transcriptomic and anatomical accuracy of example spatial transcriptomics techniques for this application, chronic pain-regulated changes in the PBN were analyzed. The PBN is known to be a major hub to receive, process, and relay nociceptive signals (Palmiter, R. D., Trends Neurosci. 41, 280-293 (2018); Sun, L. et al., Nat. Commun. 11, 5974 (2020)). As part of adaptations to neuropathic pain, PBN cells are likely to mount complex transcriptional responses (Yap, E. L. & Greenberg, M. E., Neuron 100, 330-48 (2018)). However, such changes, as well as many others in different brain regions, are yet to be unveiled. Coronal PBN sections were assayed from animals that received either a sham operation or partial sciatic nerve ligation (SNL)-induced neuropathic pain (30th day post-surgery).

To facilitate comparing cells in identical anatomical sites, the PBN was divided into four subregions (two PBNI and two PBNm) and the trigeminal for the comparison (FIG. 15a). To minimize variations caused by individual heterogeneity and the sectioning of brain samples, two middle sections with the highest cluster similarity (FIG. 15b, Extended Data FIG.16,17) were compared. Unsupervised clustering of 32,377 cells pooled from the two sections identified 16 neuronal and 12 non-neuronal subtypes (Supplementary Table 3). A differential abundance analysis (Zhao, J. et al., Proc. Natl. Acad. Sci. USA. 118, e2100293118 (2021)) of the sham and SNL mouse data detected a remarkable imbalance of cell distributions (FIG. 15c), which is corroborated by changed levels and spatial patterns of individual genes (Extended Data FIGS.18,19,20). Differential gene-expression analysis found 487 genes in neurons and 181 in non-neurons with altered expression including 16 encoding secreted proteins (P < 0.05; FIG. 15d and Supplementary Tables 4,5); for example, 1.23 to 1.85-fold decreases (P < 0.05) of Apoe in glial cells, in contrast to the upregulation in other injury and disease models (Pfrieger, F. W. & Ungerer, N., Prog. Lipid Res. 50, 357-71 (2011)), implying its multifaceted role in inflammation and pain modulation in the PBN. These genes in neuronal clusters were predicted to be differentially involved in neuron development, stress responses, inflammation, etc. (FIG. 15e).

An analysis of gene regulation in specific cell types within subnuclei was performed. Of particular interest was how expression of different neuropeptides respond to the pain condition. Thus, transcriptional changes of peptide precursor genes within the chosen PBN sections. Calca, the gene encoding CGRP was slightly upregulated (1.55-fold, P = 6.07 × 10⁻⁶) in motor neurons in the trigeminal (Neu5), but not significantly in the PBN (Neu6) (FIGS. 15f and 4g). Scg2 and Cck were downregulated by 2.54- and 2.82-fold (P < 0.001), respectively, with regional specificity: Scg2 decreased across the PBN, but Cck changed mainly in the subregion 2 populated by Resp18/Ctxn2+ neurons. Notably, Penk, encoding an opioid precursor, showed decreased expression in subregions 1 and 3: 2.13- and 3.03-fold, respectively (P < 0.001), but a 3.07-fold increase in the subregion 2 (Neu1 and Neu2; P < 0.05). These examples along with all the other changes provide valuable clues enabling functional experiments in the future.

### Cell-cell communication coordinated by transcriptional dynamic of microglia and astrocytes

Cell-cell communication coordinated by transcriptional dynamics of microglia and astrocytes was further analyzed. Given the reference and pain-induced transcriptome maps, this Experimental Example was used to analyze how gene regulation affects local cell- cell communication. To quantitatively compare the cell-signaling networks, signaling likelihoods for each cell as "sender" or "receiver" were computed using ligand and receptor transcript levels and spatial distances from other senders and receivers (FIG. 37a) (Cang, Z. X. & Nie, Q., Nat. Commun. 11, 2084 (2020)). Because transcripts are mainly detected in cell bodies and it is difficult to analyze the long-distance communication mediated by cell projections such as axons, neurons and non-neuronal cells were treated equally in this analysis. The comparison of signaling likelihoods between the sham and SNL mouse datasets indicates that the major changes in the PBN region were associated with microglia and astrocytes (FIG. 37b and Supplementary Table 6), which are known to coordinate neuronal development and homeostasis (Vainchtein, I. D. & Molofsky, A. V., Trends in Neurosci. 43, 144-54 (2020)). A subcluster-level analysis of signaling between microglia (M), astrocytes (AS1-AS5), and major PBN neurons (Neu1-Neu4 and Neu6-Neu8) revealed subcluster-specific increases or decreases in microglial and astrocyte signaling (FIG. 16a). A detailed comparison of the contributions by individual ligand-receptor(s) pairs found that the top contributors were neuropeptides, cytokines, glycoproteins, lipoproteins, and their receptors (FIG. 16b). Furthermore, to understand the signaling heterogeneity, cells of a sender subcluster were profiling for the contribution by each ligand. Interestingly, the senders showed a bimodal (e.g., Mif in Neu2 and Apoe in AS1) or unimodal distribution (e.g., Spp1 in Neu3 and C1qb in M), and the pain-responsive and non-responsive subpopulations appeared to be separated in the bimodal distribution where the responsive cells had higher signaling likelihoods than the non-responsive (FIG. 16c).

Given the importance of microglia and astrocytes for signaling, their heterogeneity at the transcriptome level was further investigated. To date, microglial heterogeneity associated with physiologic roles such as supporting synaptic development and remodeling in the homeostatic adult brain has not been confirmed by dissociative scRNA-seq (Li, Q. Y. et al., Neuron 101, 207-23 (2019)), because microglial gene regulation is environmentally sensitive and can be easily disrupted by tissue dissociation (Gosselin, D. et al., Science 356, eaal3222 (2017)). Here, 584 cells with microglial markers (e.g., C1qa and C1qb) were re-clustered into two subtypes, M1 and M1* (FIG. 16d (left) and Extended Data FIG. 22a). Despite their similarity, M1* was annotated with specific immune response-regulating marker genes (e.g., Mif and Sod1) involved in an interleukin-12 (II-12)-mediated signaling pathway (P = 6.68 × 10-5) and neutrophil mediated immunity (P = 5.22 × 10-4) (Extended Data FIG. 22b). M1* is different from an activated neuroinflammatory state induced by lipopolysaccharide43 due to the lack of three marker genes, II1a, Tnf, and C1q. Considering a strong association between their marker genes in immune regulation, M1* could represent a transition state to the activated microglia. Under the pain condition, M1* decreased from 33.6% to 22.0% of the microglial population (FIG. 16d (right)). Given the different ligand and receptor profiles of M1 and M1*, the decrease of M1* is associated with the changed communication in the signaling networks. Microglia showed a relatively even spatial distribution (FIG. 16e) likely due to their high motility in the tissue.

Transcriptomic and signaling heterogeneity of astrocytes identified from the initial clustering were correlated. 4,471 cells were re-clustered into eight subtypes annotated with marker genes (FIG. 16f and FIG. 39a); most of the subclusters are connected, suggesting a continuum of transcriptomic states. As expected, some subtypes had region-specific distributions (FIG. 39b). In the clustering outcome, the pain-induced major changes were found in the subtypes 2 and 3, which was confirmed by the differential abundance analysis (FIG. 16g (left)). To understand the transcriptomic changes, the pseudo-temporal ordering of all subtypes was analyzed (FIG. 16g (right)). Projection of the whole-cell population along a pseudotime trajectory revealed three separated groups, A1, A2, and Pan (FIG. 16h), which can be correlated to three astrocyte states with specific physiological roles (Liddelow, S. A. et al., Nature 541, 481-87

(2017)). A1 and A2 astrocytes with differentially expressed Sparc and Sparcl1 are known to have destructive and protective roles, respectively, in maintaining homeostasis; thus, the increase of A1 is often associated with neuroinflammation (Liddelow, S. A. et al., Nature 541, 481-87 (2017)). Here, a significant decrease of A1, mainly contributed by the subtypes 2 and 3, was found for the SNL condition (P = 1.20 × 10⁻⁷), suggesting that pain adaptation might involve an unknown neuronal protection mechanism. Remarkably, the comparison of astrocyte spatial distributions found that the A1 decrease was mostly in the PBN region, but the other two states had no obvious changes (FIG. 16i). These results, together with the analysis of neuron-glia communication, provide important evidence of the region-specific glial transcriptomic dynamics supporting local neuronal activities.

### Methods

In Experimental Example 1, fabrication of continuous polony gels with high resolution and RNA capture efficiency was performed. 370-base pair (bp) DNA templates bearing a 24-bp random sequence were synthesized by Integrated DNA Technologies (IDT). They were PCR amplified (Taq 2× master mix; New England Biolabs (NEB) M0270) with bridge PCR primers (BA(+) and BA(-); Supplementary Table 8) for 15 cycles and size selected by 2% agar gel. Purified DNAs were quantified by Qubit 4 fluorometer (Thermo Fisher Scientific), diluted to 1 nM, and aliquoted for storage at 20°C. The template structure used in this Experimental Example was AATGATACGGCGACCACCGAGATCTACACTGCGGCCGCTAATACGACTCACTATAGGGAT CTNNNNNNNNNNNNNNNNNNNNNNAGAGAATGAGGAACCCGGGAACAATGATGGAATTTT TTTTTTTTTTTTTTTTCGAACCACCGAGGTTGCCGGACTAGCGCAAGTACTTGTCCATTCCT GAAGAAATATTATATTTATACAACTTACCCATAGAATCCTATTTACTAGGAAAGGAAAAGCCT CCTATTTATAAAAATTGGATAGAGCTTTCTCAACAACAGTGGAATATCAATGATAGAACAATT GCCGATTTATTAGATGGGGTCTTAATAATACCATCGATCTCGTATGCCGTCTTCTGCTTG (SEQ ID NO: 3), wherein the sequence of the BA(+) primer is AATGATACGGCGACCACCGAGATCTACAC (SEQ ID NO:4); The sequence of the Taql digestion site is TCGA; the sequence of the spatial index is NNNNNNNNNNNNNNNNNNNNNNNN (SEQ ID NO: 5); the sequencing priming site is CCACCGAGGTTGCCGGACTAGCGCAAG (SEQ ID NO: 6); and the sequence of the BA(-) primer is TCTCGTATGCCGTCTTCTGCTTG (SEQ ID NO: 7).

Crosslinked PAA gel casting was performed in an anaerobic chamber (Coy Lab) as previously described (Gu, L. et al., Nature 30 515, 554-57 (2014)). A gel casting solution (8% acrylamide/bis-acrylamide (w/v, 19:1; Sigma-Aldrich A9099 and M7279), 16 mg/mL N-(5-bromoacetamidylpentyl) acrylamide (Carbosynth FB166698), 0.01% (w/v) ammonium persulfate (freshly dissolved; Sigma-Aldrich A3678), and 0.015% (v/v) N,N,N', N'-tetramethylethylenediamine (Thermo Fisher Scientific 15524-010)) was mixed in the chamber immediately before the casting. For example, to cast a gel of 8 × 40 mm² (a center region of 6 × 30 mm² to be sequenced), a 20 µL casting solution was applied to the surface of a Bind-silane (GE healthcare 17-1330-01)-coated round coverslip (Warner Instrument 64-1696). To form a gel layer of < 5-µm thickness, a repel-silane (Cytiva 17133201)-coated rectangular top glass piece (8 × 40 mm², 1-mm thick) was placed on top of the solution and tightly pressed it against the coverslip to form a uniform liquid layer between the coverslip and the top glass. The gel was polymerized at room temperature (R.T.) for 90 min before transferred from the chamber to a PCR workstation (AirClean 600). After removing the top glass, the gel-coated coverslip was assembled into a FCS2 flow cell (Bioptechs) with a 0.2-mm-thick gasket to form a channel of 6 × 35 mm² with a volume of 42 µL.

Primer grafting and template hybridization was performed using the crosslinked PAA gel. The flow cell was rinsed with 2 mL milli-Q water and then 500 µL grafting buffer (10 mM potassium phosphate buffer, pH 7). Primer grafting was performed by incubating 25 µM each 5' phosphorothioate-modified primers (PS-BA(+) and PS-BA(-), IDT; Supplementary Table 8) in the grafting buffer at 50°C for 1 hour. The flow cell was washed with 500 µL hybridization buffer (5 × SSC, 0.05% Tween-20; Invitrogen AM9763 and Sigma-Aldrich P9416). 12 µL 1 nM DNA templates were denatured in 12 µL freshly prepared 0.2 N sodium hydroxide (Sigma-Aldrich 72068) at R.T. for 5 min and then neutralized with 12 µL 200 mM Tris-HCl, pH 7. The mixture was diluted in 964 µL ice-cold hybridization buffer to a final concentration of 12 pM. 500 µL diluted templates were injected into the flow cell, incubated at 75°C for 2 min, and air cooled to 40°C in 15 min. Before polony amplification, the flow cell was washed with 500 µL preamplification wash buffer (2 M Betaine (Sigma- Aldrich B2629) and 20 mM Tris-HCl, pH 8.8).

Polony amplification was performed with computer-controlled P625 pumps (Instech Laboratories) and a heating/cooling block (CPAC Ultraflat CPAC HT 2-TEC, Inheco). Gelanchored first- strand templates were synthesized by pumping 150 µL Taq DNA polymerase mixture (50 U/mL Taq DNA polymerase (NEB M0267) and 200 µM each dNTPs (GenScript C01582)) in a polony amplification buffer (2 M Betaine (Sigma-Aldrich B2629), 20 mM Tris-HCl (pH 8.8), 10 mM ammonium sulfate (Sigma-Aldrich A4418), 2 mM magnesium sulfate (Sigma-Aldrich M2773), 0.1% (v/v) Triton-X-100 (Sigma-Aldrich T8787), and 1.3% (v/v) DMSO (Sigma-Aldrich D8418)) at 74°C for 5 min. The flow cell temperature decreased to 60 °C for isothermal bridge amplification. The amplification was performed by repeating 35 cycles of: i) Denaturation: pump 500 µL deionized formamide (Emdmillipore 4670) at a flow rate of 3 mL/min and stop for 1 min; ii) Annealing: pump 500 µL polony amplification buffer at 3 mL/min and stop for 2 sec; iii) Extension: pump 500 µL Bst DNA polymerase mixture (80 U/mL Bst DNA polymerase (NEB M0275) and 200 µM each dNTPs) in the polony amplification buffer at 3 mL/min and stop for 2 min.

To generate single-stranded DNA (ssDNA) for polony sequencing and expose 3' poly-T probe for transcript capturing, the polonies were digested. The flow cell was washed with 1× CutSmart buffer (NEB) and then added with 3 × 100 µL 2,000 U/mL Taql (NEB R0149)in 1× CutSmart buffer and incubated at 60°C for 3 × 10 min. Digested polony gels were washed with 500 µL elution buffer 1 (1 × SSC and 70% formamide (v/v)) before sequencing or stored in 100% formamide.

Polony sequencing was performed with an Illumina HiSeq SBS kit v4. Images were acquired using a Nikon Ti-E automated inverted fluorescence microscope equipped with a perfect focus system, a Nikon CFI60 Plan Fluor 40×/1.3-NA oil immersion objective, a linear encoded motorized stage (Nikon Ti-S-ER), and an Andor iXon Ultra 888 EMCCD camera (16-bit dynamic range, 1,024 × 1,024 array with 13 µm pixels). A four-channel imaging setup comprised two laser lines (Laser Quantum GEM 532 nm (500 mW) and Melles Griot 85-RCA-400 660 nm (400 mWS)) and two filter cubes with an emission filter (610/60-730/60 or 555/40-685/20; Chroma Technology) and a 532/660 dichroic mirror (Chroma Technology). Sequencing regents were injected to the flow cell by a fluidic system including a multi- position microelectric valve (Valco Instruments EMH2CA) and a multi-channel syringe drive pump (Kloehn V6 12K). The sequencing was automated by building an application in Java 1.6 using jSerialComm (http://fazecast.github.io/jSerialComm/) to control the fluidic system and Micro-Manager v1.4.22 (https://www.micro-manager.org/) to acquire images from selected stage positions. The sequencing was performed with reagents provided in the HiSeq SBS kit following a standard HiSeq sequencing protocol. Each sequencing cycle included i) pre-cleavage wash with a cleavage buffer, ii) dye and protection group removal by a cleavage mix, iii) post-cleavage wash with a high salt buffer, iv) a pre-incorporation wash with an incorporation buffer, v) incorporation with an incorporation mix, and vi) imaging acquisition in a scan mix.

All of the animal experiments described within this Experimental Example were conducted according to US National Institutes of Health guidelines for animal research and approved by the Institutional Animal Care and Use Committee (IACUC) at the University of Washington. Male C57Bl/6J mice (Jackson Laboratory 000664) ranging from 3-4 months of age, were used for all studies. Animals (i.e., mice) were housed in temperature- and humidity-controlled facilities with 12-h light/dark cycles with ad libitum access to standard chow diet (LabDiet 5053).

Nerve ligation surgery was performed on some of the mice. Sciatic nerve surgeries were performed as previously described in Seltzer, Z. et al., Pain 43, 205-18 (1990). Mice were anesthetized with 2% isoflurane at a flow rate of 1 L/min and a 1-cm long incision was made in the proximal one third of the lateral thigh. The sciatic nerve was exteriorized with forceps inserted under the nerve. A silk suture was then passed through approximately 1/2 of the nerve bundle, before being tightly ligated and crushed. For sham mice, the sciatic nerve was exteriorized using forceps and then returned to its normal position. The skin was then closed with sutures. All mice were euthanized for tissue extraction exactly 30 days post-nerve ligation surgery.

To prepare tissues, mice were anesthetized with Beuthansia (0.2 mL, i.p.; Merck) and decapitated. The brain was rapidly dissected, frozen on crushed dry ice, and stored at -80°C until sectioning.

In this Experimental Example, sections were prepared and used for transcript capturing and cDNA synthesis. After polony sequencing, the gel-attached coverslip was dissembled from the flow cell and washed with milli-Q water and 3 × 80 µL wash buffer 1 (0.1 × SSC and 0.4 × Maxima H Minus RT buffer (Thermo Fisher, EP0753)). The gel was dried in the PCR workstation before use. For transcript capturing, depending on tissue sizes, single or multiple tissue sections were placed on the same gel (e.g., 6 × 30 mm²). Assaying multiple tissues on the same gel can reduce assay biases and reagent costs. Specifically, frozen tissues were equilibrated at -20°C in a Cryostat NX70 (Thermo Scientific) for 15 minutes. A tissue block was mounted onto a holder with O.C.T. (Fisher Healthcare 4585) and sliced to 10-µm sections. Immediately after transferring tissue sections to a dried gel surface, a tissue hybridization buffer (6 × SSC and 2 U/µL RNAseOUT (Thermo Fisher 10777019) was gently applied to immerse the sections and then the gel was incubated at R.T. for 15 min. For example, 40 µL hybridization buffer was added to a 4 × 4 mm² section. After the hybridization, the buffer was removed by pipetting and the coverslip was reassembled into the FCS2 flow cell. cDNAs were synthesized by adding 100 µL reverse transcription (RT) mixture (5 µL Maxima H- Reverse Transcriptase (Thermo Fisher EP0753), 20 µL 5× Maxima RT buffer, 20 µL 20% Ficoll PM-400 (Sigma-Aldrich, F4375), 10 µL 10 mM each dNTPs, 5 µL 50 µM template switch oligo (Qiagen 339414YCO0076714), 2.5 µL RNAseOUT (40 U/µL), and 37.5 µL H2O) and incubating the flow cell at 42°C for 1 h. A Cy5-dCTP-labeled cDNA assay was performed at the same condition except replacing the dNTPs with 500 µM each dATP/dGTP/dTTP, 12.5 µM of dCTP, 25 µM Cy5-dCTP (PerkinElmer NEL577001EA).

Tissue cleanup was performed. After the cDNA synthesis, 100 µL proteinase K digestion solution (10 µL proteinase K (Qiagen 19131) in 90 µL Qiagen PKD buffer (Qiagen 1034963)) was added to the flow cell and incubated at 55°C for 30 min. To remove digested proteins, genomic DNAs, and others, the gel was washed with 3 × 500 µL elution buffer 2 (2 × SSC and 0.1% SDS) and 3 × 500 µL wash buffer 2 (0.1 × SSC).

Sequencing libraries were constructed. Recovering spatially indexed cDNAs from the gel and introducing 15 unique molecular identifiers (UMIs) into cDNAs were achieved by a two-step primer extension, which was performed in a slide chamber assembled with i) a coverslip and ii) a 70 × 26 × 2 mm silicone pad punched with a rectangular hole (8 × 8, or 8 × 20, or 8 × 50 mm² for different gel sizes) via plasma-activated bonding, and iii) a plate sealing film (BioRad MSB1001) cut to the silicone pad size and punched with a small reagent hole. Typically, a 6 × 6 mm² gel was put into the slide chamber, sealed with the sealing film, and added with 150 µL UMI incorporation mixt (75 µL 2× Q5 Ultrall master mix (NEB M0544), 0.75 µL 20 mg/mL BSA (NEB B9000), 0.75 µL Q5 HotStart polymerase (NEB M0493), 6 µL each 10 µM two primers (TSO and UMI; Supplementary Table 8), and 61.5 µL H2O). The chamber was placed into a DNA Engine Slide Chamber Alpha unit (Bio-Rad) to perform two cycles of thermal cycling including denaturing at 95°C for 0.5 min, annealing at 65°C for 0.5 min, and extension at 72°C for 0.5 min. In the first cycle, 2nd-strand cDNAs were synthesized; in the second cycle, UMIs were introduced to 3rd-strand cDNAs by the primer annealing to the 2nd-strand cDNAs released from the gel. After the UMI incorporation, the reaction mixture was transferred to a 1.5 mL tube and purified by Ampure XP beads (Beckman Coulter A63987) with 0.6× bead-to-sample ratio. Eluted cDNAs were further amplified to obtain 5-10 ng DNA per sample; for example, we used 12 PCR cycles for a MOB section. Typically, 50 µL cDNA amplification mix comprised 25 µL 2× Q5 Ultrall master mix (NEB); 2 µL each 10 µM TSO and TruSeq sequencing primers (Supplementary Table 8), and 21 µL purified DNA. After the amplification, 1 ng DNA was used for sequencing library construction with a Nextera XT kit (Illumina FC-131-1024) and TruSeq (LibP5; Supplementary Table 8) and Nextera index primers following the manufacture's protocol.

HiPlex FISH was also performed. 10-µm-thick cryosectioned brain tissues were mounted on Superfrost Plus slides (Fisher Scientific). RNAScope HiPlex assay (ACDBio) was performed following the manufacturer's protocol. Briefly, slides were fixed in 4% paraformaldehyde, dehydrated in 50%, 70%, and 100% ethanol, then treated with Protease IV. Probes (e.g., Penk, Pdyn, Calca, and Tac1) were hybridized at 40 C̊ for 2 h then amplified for detection. Cell nuclei were stained with DAPI then fluorescent images were acquired using a Keyence BZ-X700 microscope. Images were registered using HiPlex image registration software (ACDBio) and minimally processed in Fiji (ImageJ) to enhance brightness and contrast for optimal representation of the data.

To assess template diffusion in the RNA capturing, a mouse MOB section was placed on a dried gel pre-soaked with a nucleus staining buffer (0.1 × SSC, 2.5 × SYTOX Green, 0.4 × RT buffer) and SYTOX-stained nuclei were immediately imaged with a FITC channel (Ex488 nm/Em520 nm). Cy5-labelled cDNAs were synthesized on the same tissue and then imaged with a Cy5 channel (Ex640 nm/Em665 nm). The nucleus image served a reference for analyzing template diffusion in the Cy5 image. 10 regions (250 × 250 pixels) were randomly selected from the two images to determine a transformation matrix and a scaling factor using a MATLAB builtin function, imregister().

Base calling of polony sequencing was performed. Raw sequencing tiff images were processed to extract intensities by Dlight, a custom-built suite in MATLAB. All images were registered to the merged images of the Cy3 and Cy5 channels from the first sequencing cycle using imregcorr(). Next, a polony reference map was generated from images of the first eight sequencing cycles, termed template cycles. Polonies were identified by searching local signal thresholds (> median + 2 × standard deviation) in all template cycle images and then finding polony centroids with an optimal chastity value. A PhiX control library (Illumina FC-110- 3001) was used to optimize Dlight parameters. Intensity values of polony centroids and image pixels were analyzed by a 3Dec base-caller (Wang, B. et al., Sci. Rep. 7, 41348 (2017)) allowing the correction of the signal crosstalk between adjacent polonies. To find polony boundaries, unassigned image pixels were compared with spatial index-assigned polony centroids within a distance less than 5 pixels using a pipeline described in FIG. 18. These pixels were assigned with adjacent assigned spatial indices with the highest signal correlation coefficient above 0.7. A final spatial index map was constructed by combining all sequenced gel positions into a single image.

Transcript mapping was performed. After cDNA library sequencing, FASTQ files were processed to map transcripts to the spatial index map. Spatial index and UMI sequences were first extracted by Flexbar49. The index sequences were mapped back to the spatial index map by Bowtie50 allowing up to 2 mismatches. Paired- end reads of mapped indices were aligned to mouse transcriptome (GRCm38) using STARv2.7.051 with a default setting. Sequencing reads with the same transcriptome mapping locus, UMI, and spatial index were collapsed to unique records for subsequent analysis.

Transcript segmentation was performed using a method, volume-distance-based segmentation of mapped transcripts (V-seg), which was implemented in R and developed to aggregate feature-level UMIs to single cells. V-seg first uses mapped transcript data to construct a rNN-network (nearest neighbors within a defined radius, r) by connecting spatial indices as nodes (FIG. 25a). The network was constructed using a cutoff edge distance of 2 µm to ensure every index was connected to at least one different index and also decrease edge or connection redundancy (FIG. 25b). The edge weight was calculated by considering the spatial distance, the UMI densities and transcript similarity between two connected spatial indices using an equation in FIG. 25c. Next, the edge-weighted network was taken as an input for the graph- based segmentation with a Fast Greedy algorithm using an iGraph R package. Because the Fast Greedy algorithm is sensitive to the network size, to maximize a modularity score, it is necessary to optimize segmentation parameters for different tissues.

For the MOB clustering, 22,830 cells were analyzed. SCTransform normalization was applied in Seurat using 3,000 highly variable genes. After Principal Component Analysis (PCA), 18 PCs were used in the UMAP visualization, and the clustering graph was generated with a resolution of 0.8. For the clustering outcome shown in FIG. 14c, 63,808 cells from four PBN sections were analyzed with 22 PCs and graphed with a resolution of 0.3. For the clustering outcome shown in FIG. 14d, 31,505 neurons from FIG. 14c were analyzed with 12 PCs and graphed with a resolution of 0.65. For the clustering in FIG. 15b, 15,833 neurons from two PBN sections were analyzed with 18 PCs and graphed with a resolution of 0.7, and 16,544 nonneuronal cells were analyzed with 22 PCs and graphed with a resolution of 0.4. For the clustering outcome in FIG. 16d, 584 microglia from the two PBN sections were analyzed with 14 PCs and graphed with a resolution of 0.4. For the clustering outcome in FIG. 16f, 4,471 astrocytes were analyzed with PCs and graphed with a resolution of 0.4. Marker genes for each cluster were identified by Seurat using a Wilcoxon test and then compared with the consensus in a published mouse brain cell type atlas (Zeisel, A. et al., Cell 174, 999-14 (2018)).

scRNA-seq-guided annotation was performed. Segmented MOB cells were annotated with a published MOB scRNA-seq dataset (GSE121891) 25 as a reference to predict cell type compositions using scvi-tools52. The top 3,000 variable genes were selected for the model training. Raw gene counts for training and testing datasets were scaled to 104. The training and prediction were run at max_epoches = 50. Cell types of segmented cells were determined by the predicted cell types with the highest ratios.

Differential abundance analysis was performed. The clustering results in FIG. 15b were analyzed by DA-seq (Zhao, J. et al., Proc. Natl. Acad. Sci. USA 118, e2100293118 (2021)) to identify differences between cell distributions in the UMAP space for the Sham and SNL conditions. Coordinates of the UMAP clustering outcomes and Sham and SNL-condition labels were maintained for the DA-seq analysis using a score vector of k = seq (50, 500, 50).

Spatial pattern gene analysis was performed. To identify mRNA distributions showing different spatial patterns in the Sham and SNL conditions, the cell-gene matrix and cell spatial coordinates were processed by Giotto (Dries, R. et al. Genome Biol. 22, 78 (2021)). The analysis used a default setting (e.g., a minimum detected genes of 64 per cell and a scale factor of 6,400) to find spatially patterned genes by a "ranking" method. The top 200 spatially patterned genes were chosen for a GO enrichment analysis (Kuleshov, M. V. et al., Nucleic Acids Res. 44, W90-97 (2016)).

Cell contacts (or colocalization) between the same or different cell types were quantitatively compared using a pairwise cross-correlation function (PCCF) statistic which was previously applied to analyze polony colocalization (Gu, L. et al., Nature 30 515, 554-57 (2014)). Here, to analyze cell colocalization, PCCF was defined as the ratio of a detected number of colocalization events to a random colocalization possibility. The random colocalization was simulated by a simplified model, where irregular cells were represented as round shapes with original sizes. After randomizing original cell positions, touched and overlapped cells were counted; 100 simulations were performed to calculate the average colocalization count as the random colocalization possibility. In FIG. 14g, the PCCF was computed for all annotated cell clusters to generate the heatmap.

The pseudotime analysis of the astrocyte clusters in FIG. 16g was performed by Slingshot (Street, K. et al., BMC Genomics 19, 477 (2018)) with parameters of stretch = 2 and thresh = 0.001. The P-value of the pseudotime values for the Sham and SNL conditions was calculated with a Kolmogorov-Smirnov test.

Cell-cell communication was evaluated by spatial profiling of signaling likelihoods of individual cells as senders and receivers using SpaOTsc with default parameters (Cang, Z. X. & Nie, Q., Nat. Commun. 11, 2084 (2020)). For cluster-to-cluster communication, signaling likelihoods higher than 0.003 are shown in FIG. 16a. In FIG. 16b, a Kolmogorov-Smirnov test was used to compare cluster-level signaling likelihoods for paired ligand and receptor(s) genes between the Sham and SNL conditions.

### EXPERIMENTAL EXAMPLE 2

Spatial transcriptomics techniques using polony gel for spatial barcoding will be described with respect to Experimental Example 2. As shown in FIG. 12A, this example utilizes DNA templates, each bearing two polony bridge primers, two spatial barcode sequencing primer sites, a spatial index barcode, a poly(T) probe, and two restriction digestion sites. In certain examples, the DNA template includes 150 - 1500 base pair (bp). In certain examples, the DNA templates include 350 - 390 (e.g., 370) bp. The index can be random or synthetically designed and will generally include 10 - 50 (e.g., 24 random) bp. Exemplary restriction digestion sites include Taql, BsaWI, BstBI, Dpnll, Xbal, and BspEl.

The templates can be synthesized and disposed on the surface of a crosslinked PAA gel surface, and then amplified into polonies. Spatial indices of the polonies, and their coordinates, can be determined by polony sequencing. After restriction digestion to expose a 3' capturing sequence, biological samples (e.g., cryosectioned tissues) can be placed on the gel to perform in situ reverse transcription. Spatially barcoded cDNAs can be amplified and analyzed using next-generation sequencing.

FIG. 12B illustrates a comparison of 3D intensity profiles of "continuous" (on a crosslinked PAA gel) and standard "discrete" polonies (on a semifluidic linear PAA gel) stained by SYBR Green. Polony amplification was performed with the same template concentration (15 pM) and cycle number (35). The borders of continuous polonies are delineated by sequencing and their spatial locations are determined by a pixel-level base calling algorithm. By screening PAA gel fabrication conditions, it was found that oligo arrays derived from polonies formed on a crosslinked PAA gel surface are suitable for tissue barcoding applications. In these Experimental Examples, it was confirmed that widely used DNA polonies grown in porous substrates such as the linear PAA gel (D.R. Bentley et al. (2008)) show typical discrete features with the highest intensities at polony centers and the lowest at the borders (FIG.12B). In next-generation sequencing, polony peaks facilitate the registration of fluorescence signals, but might cause RNA capturing efficiency bias across polonies and inside a polony. In various implementations described herein, by changing the gel casting condition, continuous polonies were formed on the crosslinked PAA surface (FIG. 12B). These polonies show relatively fast size expansion and a more homogenous DNA distribution than discrete polonies, likely because the clustering occurs close to the gel surface with less constraint on the amplification reaction. Although continuous polonies are connected, they rarely interpenetrate, and their borders can be delineated by sequencing (FIG. 12B).

FIG. 17B illustrates an example evaluation of restriction site cleavage efficiency of continuous polony DNAs on a crosslinked PAA gel. Part a of FIG. 17B illustrates detection of noncleaved polonies by a Cy5-probe. Control DNA polonies were produced by USER enzyme digestion to create single-stranded DNA. The Taql-treated polonies were digested under 60 degrees Celsius for one hour. Then, the Cy5 was hybridized for visualization. FIG. 17B illustrates an assessment of the fraction of cleaved DNA by image intensity analysis. 8 field of views (1024x1024 pixels) were included. Notably, compared with discrete polonies grown inside of PAA gels, continuous polonies can be efficiently cleaved by restriction digestion; in this Experimental Example, 93.6% of double-stranded polony DNAs were digested to expose a priming site for cDNA synthesis by the Taql digestion (FIG. 17B). Given the above advantages, continuous polonies are better suited than the polonies generated using a linear PAA gel for preparing highquality oligo gels for RNA capturing. These previous polonies are discrete and formed on semifluidic linear PAA gels.

In this Experimental Example, to manufacture oligo gels with spatially defined indices, 370-base pair (bp) templates were amplified bearing 24-bp spatial indices randomly seeded on a gel with a customizable size (e.g., 6 × 30 mm²) casted on a coverslip and then assembled in a flowcell, similarly as reported in L. Gu et al. (2014). Spatial indices were sequenced by a sequencing-by-synthesis chemistry (D.R. Bentley et al. (2008)). FIG. 18 illustrates an example pixel-level reconstruction of a spatial index map. Part a of FIG. 18 illustrates a pipeline of pixel-level reconstruction. Briefly, the pixel-level intensity was extracted from sequenced images for primary base calling using a MATLAB script. The spiked-in 6mers were checked within the barcode sequences and the unmatched ones were discarded. The remaining sequences were allocated to their coordinates. Those unassigned pixels were directed to the position by neighborpixel correlation (distance = 5 pixels). Part b illustrates an example of a raw image of four-color sequencing. Part c illustrates a spatial heatmap of correlation-coefficient between a center pixel and its neighbors. Part d illustrates an example density plot of correlation-coefficient. In this example, a cutoff of 0.7 was chosen based on the distribution. Part e illustrates a final reconstruction of a pixel-level map of spatial indices. In various examples, raw sequencing images were converted to a pixel-level spatial index map (FIG. 12B) by a base-calling pipeline which determines the major index species in each image pixel (0.325 × 0.325 µm²) (FIG. 18).

To assess the resolution of the arrays, feature densities and sizes were measured. 0.5-0.8 million unique indices (or features) were identified per mm², close to the polony density of anon-patterned HiSeq flowcell using a linear PAA gel.

FIG. 8 illustrates a table comparing example spatial transcriptomics techniques to other reported methods. Compared with other oligo arrays used by other reported methods (P.L. Stahl et al. (2016); S.G. Rodriques et al. (2019); S. Vickovic et al. (2019)), the feature size in various implementations of the present disclosure decreases by 3 to 200 folds (FIG. 8). The decreased feature size offers the sufficient resolution for single cell segmentation for mammalian tissues.

FIG. 9 illustrates an example of an anticipated benefit of PAA-gel on in-situ cDNA synthesis. FIG. 9 illustrates that PAA gels may efficiently capture RNA and slow down RNA diffusion. Gels on other types of substrates, in contrast, have relatively fast diffusion rates in solution. FIG. 10 illustrates an example structural difference of a linear PAA-gel and a crosslinked PAA-gel. Various results described herein suggest that the PAA substrate can constrain the RNA diffusion to preserve the spatial resolution (FIG. 9). Additionally, the crosslinked PAA gave a 17.6-fold higher cDNA yield than the linear PAA, probably due to a higher oligo accessibility in the crosslinked gel (FIG. 10).

FIG. 11 illustrates an example of a spatial distribution of cDNA read density per polony (1 pixel = 0.325 * 0.325 um). FIG. 11 also illustrates a close visualization of cDNA density distribution showing a subcellular resolution (right panel). Guided by the predetermined spatial index map, sequencing reads were constructed into a digital spatial transcriptome image at subcellular resolution. Each segmented cell was estimated having 50 clustered indices.

FIG. 24 illustrates an example estimation of the impact of feature size on a spatial transcriptome. Part a of FIG. 24 shows a published seqFISH+ data (pixel size = 0.103 um) that was used to simulate a simple spatial-indexed transcriptome experiment. The simulation adopts a bead-shaped spatial index with 5% variation in radius through a Gaussian distribution. On a 2D plane, a series of simulated beads with different diameters (10 - 1 um) are progressively packed and linked to transcripts within its radius based on the coordinates. The simulation was run 10 times using the raw dataset (average cell diameter: 15.9 um) and the modified dataset (the average cell diameter was intentionally decreased to 7.95 um). Part b of FIG. 24 illustrates a fraction of features that can be confidently assigned to a single cell. Cutoff = 0.7, 70% of the mRNAs from a single feature belongs to the same cell. In various implementations, a computational simulation suggests that, in an array covered by a tissue section with the average cell diameter of 15.9 ± 4 µm, the percentage of features contacting only one cell increases from 50.3 to 96.3% if the feature diameter decreases from 10 to 1 µm (FIG. 24).

The RNA capture efficiency correlates to the density of array oligos and their accessibility to tissue RNAs. The crosslinked PAA gel was reported with a 10 to 30-fold higher oligo capturing efficiency than solid surface substrates such as glass slides (N. E. Broude et al., NUCLEIC ACIDS RES. 29, e92 (2001)). Compared with the linear PAA gel for polony amplification (D.R. Bentley et al. (2008)), the crosslinked gel described herein provides a better mechanical strength and stability for tissue mapping assays.

FIG. 12C illustrates an example of a realtime, quantitative comparison of DNA copies within polonies amplified in the crosslinked and linear PAA gels. FIG. 19 illustrates an example comparison of polony intensities in crosslinked and linear PAA gels. Part a of FIG. 19 illustrates examples of SYBR green-stained images of polonies amplified in a crosslinked PAA gel. Part b of FIG. 19 illustrates examples of SYBR green stained polonies in a linear PAA gel. This disclosure demonstrates that the crosslinked gel supports highly efficient polony amplification, yielding average 20,337 template copies per feature after 35 amplification cycles (FIG. 12C and FIG. 19), a 9-fold increase from that of a reported method using a linear PAA gel (R.D. Mitra et al. (1999)), equivalent to an oligo density of 1.74 × 104 molecules/µm² which is close to that of total oligos spotted on a glass substrate (P.L. Stahl et al. (2016)) (FIG. 13). In various examples, continuous polony arrays have over 80% substrate surface covered with oligos. A higher coverage can be achieved by increasing the template concentration and a higher resolution sequencing image setup.

In various implementations, after exposing a 3' oligo(dT) sequence by the Taql digestion, the resolution and capturing efficiency of global messenger RNA (mRNA) detection was tested. Cryosectioned tissues without fixation or other pretreatments were tested, with a goal of avoiding adverse effects of fixatives on cDNA synthesis and simplifying the protocol. In an example protocol, 10-µm frozen sections were attached to dried arrays and then reagents for cDNA synthesis were added.

FIG. 12D illustrates an example of detection of spatially index cDNAs in a mouse olfactory bulb section by the reverse transcription incorporation of Cy5-labeled dCTP. Labeled cDNAs show the single-cell and subcellular resolution. Fluorescence imaging of Cy5-labelled cDNAs in a mouse olfactory bulb-isocortex section achieved clear single-cell resolution (FIG. 12D), implying minimal lateral diffusion. Furthermore, cDNAs within single cells showed subcellular signal variations (FIG. 12D), likely due to polony-associated fluctuations of oligo density distribution. The impact of the diffusion was further assessed by registering images of a nuclear stained olfactory bulb section on a dried array as the reference and the synthesized cDNAs. FIG. 21 illustrates an example of diffusion estimation of in-situ synthesized cDNA in PAA-gels. FIG. 21 shows the prescan nuclei of mouse main olfactory bulb (SYTOX) and cy5-dCTP incorporated cDNA, a zoom-in visualization of in-situ cDNA, which can be merged to nuclei-stained images by image registration, and an estimate of scaling factors as indicators of diffusion through an example region. A scaling factor was determined to be 1.028 ± 0.016 (FIG. 21).

FIG. 22 illustrates an example of Cy5-dCTP incorporated in-situ cDNA synthesis on different gels. Part a shows an example visualization of mouse accessory olfactory bulb Cy5-dCTP incorporated in-situ cDNA on crosslinked PAA gels and linear PAA gels. Part b of FIG. 22 illustrates an example image analysis to compare cDNA synthesis efficiency between two different gels. Various results described herein suggest that the PAA substrate can constrain the RNA diffusion to preserve the spatial resolution. Additionally, the crosslinked PAA gave a 17.6-fold higher cDNA yield than the linear PAA (FIG. 22), probably due to a higher oligo accessibility in the crosslinked gel.

As a proof-of-principle for subcellular spatial transcriptome mapping, mouse accessory olfactory bulb sections were analyzed by PIXEL-seq. FIGS. 15 to 18 illustrate examples of a spatial gene expression analysis of a mouse accessory OLB. FIG. 13 illustrates an example of total unique molecular identifier (UMI) distribution per feature. 10-um bins were generated by grouping 33 pixels. A segmented cell was manually curated with a nucleus-stained image as a reference. FIG. 13 illustrates an example of UMAP clustering of binned features from a mouse accessory OLB section with colors indicating different cell types.

In these examples, a polony-oligo gel with a sequenced feature density of 0.5 million per mm² was attached to the tissue section and the spatially indexed library was analyzed by an Illumina HiSeq 4000 system. Guided by the predetermined spatial index map, sequencing reads were constructed into a digital spatial transcriptome image at subcellular resolution. Each segmented cell was estimated having 50 clustered indices. The median UMIs per bin (10 µm) was 1,000, while DBSCAN segmented cell was assigned with more UMIs (median = 1,800). Among the RNAs recovered, 70% were protein coding genes and 2% were ribosomal RNAs (rRNAs). An unsupervised spatial pattern analysis by Seurat and the relocated clustering by UMAP show layered spatial gene expression patterns as expected.

To further evaluate the performance of PIXEL-seq, a main OLB of the mouse brain was analyzed. The main OLB sections have relatively dense neuronal cell bodies from distinct neuron types presented in multiple morphological layers (S. Vickovic et al. (2019)). Various experiments were performed to determine whether the data captured in the Experimental Example could show the expected layers and other histological features. Three replicate sections were analyzed and the performance of example spatial transcriptomics techniques was tested by two steps: i) unsupervised spatial pattern detection with high-resolution feature sizes, and ii) generating high-resolution spatial expression patterns of individual genes.

FIG. 13 illustrates an example spatial distribution of cDNA densities (1 pixel = 0.325 * 0.325 um²), confirms high resolution and sensitivity of mRNA detection of the methods described in this Experimental Example, and illustrates an example of the total unique genes detected in a triplicate assay. FIG. 23A illustrates a correlation analysis of gene expression among three biological replicates. FIG. 23B illustrates an example of total UMI distribution per 16-um feature (42 pixels). FIGS. 2, 23C, and 26 illustrate UMAP clustering of binned features from a mouse OLB section with colors indicating different cell types, illustrate an example of a spatial distribution of UMAP grouped cell types, and illustrate that two detected genes show spatial pattern expression that is consistent with Allen Brain Atlas in situ hybridization (ISH) data. 23,000 unique genes are confidently detected in at least one replicate (reads count > 10). 78.3% of the genes were mapped to protein coding regions. A high correlation between each replicate indicates the robustness of example spatial transcriptomics techniques (FIG. 23A). With the saturating sequencing depth 70.6 ± 8.4%, UMIs were determined per binned feature (16 µm, median=1,175; FIGS. 2, 23C, and 26). Next, the unsupervised analysis by Seurat correctly identified layer-specific expression signatures (FIGS. 2, 23C, and 26). In total, 200 genes with spatially varied expression were identified. The found layer-specific genes (e.g. Penk and Doc2g) of these experiments agree to their ISH images from the Allen Brain Atlas (FIGS. 2, 23C, and 26).

Although example spatial transcriptomics techniques was demonstrated for poly(T) oligocaptured global transcriptome profiling, it is applicable to other spatial barcoding-based omics assays. Restriction digested polony-oligos can be added with any defined 3' adapter sequences (e.g., random or gene specific primers) by polymerase extension or DNA ligation for both global and targeted transcriptome profiling. Like a previous method (M. Stoeckius et al., NATURE METHODS 14, 865-+ (2017)), example spatial transcriptomics techniques can be applied to spatial proteomics and simultaneous multi-omics (e.g., RNA and protein) assays by using DNA-barcoded affinity reagents, such as antibodies, nanobodies, monobodies, and nucleic acid aptamers. Together, continuous polony-oligo gels represent a new generation oligo array which achieves one of the highest feature densities and homogenous oligo distribution, providing an ideal platform for spatial molecular barcoding in structured tissues.

In Experimental Example 2, various methods were performed to generate sample spatial transcriptomes. For example, spatial template DNA sequences were synthesized by Integrated DNA Technologies. The template was PCR amplified (Taq 2x master mix, NEB M0270; Bridge primers; 15 cycles) and size selected by 2% agar gel. The template sequence was 
AATGATACGGCGACCACCGAGATCTACACTGCGGCCGCTAATACGACTCACTATAGGGAT
CTNNNNNNNNNNNNNNNNNNNNNNAGAGAATGAGGAACCCGGGAACAATGATGGAATTTT
TTTTTTTTTTTTTTTTCGAACCACCGAGGTTGCCGGACTAGCGCAAGTACTTGTCCATTCCT
GAAGAAATATTATATTTATACAACTTACCCATAGAATCCTATTTACTAGGAAAGGAAAAGCCT
CCTATTTATAAAAATTGGATAGAGCTTTCTCAACAACAGTGGAATATCAATGATAGAACAATT
GCCGATTTATTAGATGGGGTCTTAATAATACCATCGATCTCGTATGCCGTCTTCTGCTTG
 (SEQ ID NO: 8). Within the template sequence, the polony bridge primer forward was AATGATACGGCGACCACCGAGATCTACAC (SEQ ID NO: 9), the spatial barcode forward sequencing primer was CGGCCGCTAATACGACTCACTATAGGGATC (SEQ ID NO: 10), the spatial barcode was NNNNNNnnnNNNNNNnnnNNNNNN (SEQ ID NO: 11), wherein N represents a random base and n represents spike sequences, the spatial barcode sequencing primer was GAGAATGAGGAACCCGGGAACAATGATGGAA (SEQ ID NO: 12), the Xmal cleavage site to remove polyT primer for ligation was CCCGGG, the polyT primer for mRNA capture was TTTTTTTTTTTTTTTTTTTT (SEQ ID NO: 13), the Taql cleavage sites to expose the polyT primer end were TCGA, the probe site to determine cleavage efficiency was CCACCGAGGTTGCCGGACTAGCGCAAG (SEQ ID NO: 14), and the polony bridge primer reverse was ATCTCGTATGCCGTCTTCTGCTTG (SEQ ID NO: 15). Primer BA(+): AATGATACGGCGACCACCGAGATCTACAC (SEQ ID NO: 16). Primer BA(-): CAAGCAGAAGACGGCATACGAGAT (SEQ ID NO: 17).

Crosslinked PAA gels were manufactured and primed for polony amplification. Two pairs of amplification primers were tested for the polony amplification. For gel anchoring, one pair have an acrydite modification and the other have a phosphorothioate (PS) modification.

The crosslinked acrylamide/Bis gel mixture compounds vary depending on what primer pairs are used. If PS modified primers are used, the gel mixture contains 3-20% acrylamide/bis (acrylamide, Sigma A9099; N, N'-methylenebisacrylamide (Bis), Sigma M7279), 1-50 mg/ml N-(5-bromoacetamidylpentyl) acrylamide (Carbosynth FB166698), The ratio between acrylamide and bis ranges from 30:1 to 1:1. 0.01-1% ammonium persulfate (APS) (Sigma A9164), and 0.01-1% N,N,N',N'-tetramethylethylenediamine (TEMED) (Invitrogen 15524-010). If acrydite modified primers are used, the gel mixture contains 3-20% acrylamide/bis, 0.01-1% ammonium persulfate (APS), and 0.01-1% N,N,N',N'-tetramethylethylenediamine (TEMED), as well as 10-1000 µmol primer each, and 0.1-100nm template. All compounds were mixed inside an oxygen-free anaerobic chamber (oxygen is less than 1000 ppm). 30 µL gel mixture was dropped on the surface of a Bind silane (GE healthcare 17-1330-01) coated round coverslip (Warner Instrument 64-1693). Then a repel-silane (Cytiva 17133201) coated rectangular top glass piece (40mm × 6mm) was placed on the gel mixture to form a top glass/gel mixture/coverslip sandwich. 0.1MPa pressure was applied to the sandwich to generate a thin film of gel. The PAA gel film was polymerized at room temperature within 90 minutes -18 hours. Then the top glass was removed and the coverslip (with PAA gel attached) was assembled into a FCS2 flow cell (Bioptechs).

If PS modified primers are used, primers were grafted and templates were hybridized. The flow cell was rinsed thoroughly with milli-Q water and 10 mM potassium phosphate buffer pH 7, respectively. The primer grafting was performed by incubating a primer mixture containing 0.1-500 µM each PS group modified primer diluted in 10 mM potassium phosphate buffer pH 7 at room temperature for 1 hour. The flow cell was washed with hybridization buffer (5 × SSC with 0.05% tween, Invitrogen AM9763 and Sigma P9416). 30 µL 1 nM of DNA library was denatured in 30 µL fresh prepared 0.2 N sodium hydroxide (NaOH) (Sigma 72068). After incubating the mixture at room temperature for 5 min, 30 µL of 200 mM Tris-HCL pH 7 (Sigma 93362) was added to neutralize the mixture. The mixture was next diluted in cold hybridization buffer to the final concentration of 1-100 pM. The template mixture was injected into the flow cell at 76°C and then air cooled to 40°C. After DNA template hybridization, the flow cell was incubated with 500 µl of Taq DNA polymerase mixture containing 100-500 units/ml Taq DNA Polymerase (NEB M0267) and 10 mM each dNTP (GenScript C01582,) at 74°C for 5 min. After incubation, the temperature was dropped to 60°C.

Polony amplification was performed on the template-seeded gels. The assembled flow cell was heated to 60°C for polony amplification. The polonies were isothermally amplified by flowing in order 1) deionized formamide (Invitrogen AM9342,) for denaturation; 2) amplification buffer (2 M betain (Sigma B2629), 20 mM Tris-HCL (pH 8.8) (Sigma 93362), 10 mM ammonium sulfate (Sigma A4418), 2 mM magnesium sulfate (Sigma M2773), 0.1% (v/v) Triton-X-100 (Sigma T8787), and 1.3% (v/v) DMSO (Sigma d8418) for washing; and 3) enzyme mix containing 10-500 units/ml Bst DNA polymerase (NEB M0275) and 25 mM each dNTP (GenScript C01582) in the amplification buffer for extension. In each of these experiments, a total of 22-35 amplification cycles were run.

Digestion was performed using Taql enzyme. To generate single stranded DNA (ssDNA) for polony sequencing as well as exposing polyT ends for reverse transcription, the polony array was digested with 500-2000 unit/ml Taql enzyme (NEB R0149) diluted in 1X CutSmart^{®} Buffer (from New England BioLabs Inc. of Ipswitch, MA) at 60°C for 1 hour.

Polonies were identified and sequenced. Single stranded polonies were hybridized with a sequencing primer (2 µM) in the hybridization buffer. The sequencing was performed at 60°C with Illumina HiSeq SBS Kit v4. A sequencing cycle includes 1) a pre-cleavage wash with the cleavage buffer, 2) a dye and protection group cleavage with the cleavage mix, 3) a post-cleavage wash with the high salt buffer, 4) a pre-incorporation wash with the incorporation buffer, 5) the incorporation with the incorporation mix, and 6) imaging acquisition in the scan mix. A MFCS^{™}-EZ + ESS^{™} fluidic system (Fluigent) was used to control the sequencing fluidics.

Images were acquired using a Nikon Ti-E automated inverted fluorescence microscope equipped with a Perfect Focus System (PFS), a Nikon CFI60 Plan Fluor 40×/1.3-NA Oil Immersion Objective Len, a linear encoded motorized stage (Nikon Ti-S-ER), and an Andor iXon Ultra 888 EMCCD camera (16-bit dynamic range, 1024×1024 array with 13 µm pixels). A Laser Quantum GEM 500mW 532nm and a Melles Griot 85-RCA-400 660nm 400mWS were used for the Hiseq kit four color imaging. To automate sequencing fluidics and image acquisition, an application was built in Java 1.6 using jSerialComm (http://fazecast.github.io/jSerialComm/) to control the fluidic system and Micro-Manager v1.4.22 (https://www.micro-manager.org/) to acquire images from selected stage positions. The application consists of a simple Java form for user interaction, as well as a text file parser to read in tab-delimited instructions, and classes to send and receive information through the COM ports connected to the pump and valve systems. The form facilitates the coordination between the imaging and the fluidic systems based on instructions listed in a user-selected text file.

Gene-specific capturing ends or poly-T ends were generated. For the whole transcriptome analysis, a template with a polyT sequence adjacent to the Taql site was used. To adapt the primer to the barcode for capturing specific mRNA in situ, a 58-bp primer was synthesized from IDT, bearing 8 random base as UMI. The capturing primer (10 µM) was annealed to polony DNA in the hybridization buffer at 85°C for 6 min followed by decreasing the temperature to 40°C and washing off unbound primers with amplification buffer. The extension was run with Bst polymerase at 60.5°C for 5 min. After 3 times extension, the elution buffer (1x SSC, 70% formamide) was applied to create single stranded capturing end with a volume of 2 ml. The prepared flow cells can be stored in formamide until used. Gene Specific capturing primer: -GeneSpecificPrimer-NNNNNNNNTCGATCGATTCCATCATTGTTCCCGG (SEQ ID NO: 18).

A library was constructed from tissue sections. A sequenced polony array was pre-coated with nuclei staining buffer (0.1x SSC, 2.5x SYTOX^{®} Green, from Thermo Fisher Scientific of Maltham, MA, or 2.5x TO-PRO^{®}-3 iodide from Thermo Fischer Scientific of Maltham, MA, 0.4x RT buffer). Frozen tissue was placed at -20 °C in a Cryostat for 15 minutes. Tissue was then mounted onto a cutting block with OCT and sliced at 10 µm thickness. Tissue sections were then transferred on the surface of the sequenced polony array attached on a coverslip. The coverslip was then removed from the cryostat and reassembled back to a FCS2 flow cell. The nuclei patterns were then imaged. The tissue hybridization buffer (6xSSC, 2u/ul RNAseout) was gently injected into the flow cell, allowing 10min for the RNA hybridization. The in situ first stand cDNA synthesis was performed within the flow cell in the RT solution for 15 minutes at 37 °C and 45 minutes at 42 °C. RT solution: (29 µL H2O; 13 µL Maxima 5x RT Buffer (Thermofisher, EP0751); 13 µL 20% Ficoll PM-400 (Sigma, F4375); 6.5 µL 10 mM dNTPs; 2 µL RNaseOUT (Thermofisher 10777019); 1.75 µL 50 µM Template Switch Oligo (Qiagen 339414YCO0076714); 3.25 µL Maxima H- RTase (Thermofisher, EP0751)).

The tissue was cleaned by injecting 72 µl proteinase k digestion solution (63ul Qiagen pkd buffer, 9ul proteinase k) into the flow cell. The solution and the tissue were incubated at 55 °C for 30 minutes. 2ml of elution buffer was then applied to ensure the removal of genomic DNA and other contaminants.

To efficiently recover the spatially barcoded cDNA, PCR amplification was performed through transferring the tissue-attached polony array into a lab designed PCR container fitting to DNA Engine Slide Chambers^{™}Alpha units (Bio-Rad). After 2-cycle PCR, the product was purified by Ampure XP beads (Beckman Coulter) with 0.6x beads to sample ratio. The recovered product was then serviced as the template to perform further amplification in order to obtain 5-10ng cDNA. Normally, it will require 11 cycle for a 4mm² tissue section. cDNA PCR mix (14 µL H2O; 25 µL of 2x Q5 master mix (NEB); 1 µL of 10 µM Truseq PCR handle primer (IDT); 1 µL of 10 µM TSO PCR primer (IDT); 7ul eluted DNA)

Library construction. The cDNA was measured by Qubit 4 (Thermo Fisher) to determine the concentration. The library was constructed using Illumina Nextra kit following manufacture's protocol.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; 119% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventor expects skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

Variants of the sequences disclosed and referenced herein are also included. Variants of the nucleic acid sequences disclosed herein include sequences with at least 70% sequence identity, 80% sequence identity, 85% sequence, 90% sequence identity, 95% sequence identity, 96% sequence identity, 97% sequence identity, 98% sequence identity, or 99% sequence identity to the protein, nucleic acid, or gene sequences disclosed herein.

"% sequence identity" refers to a relationship between two or more sequences, as determined by comparing the sequences. In the art, "identity" also means the degree of sequence relatedness between protein, nucleic acid, or gene sequences as determined by the match between strings of such sequences. "Identity" (often referred to as "similarity") can be readily calculated by known methods, including those described in: Computational Molecular Biology (Lesk, A. M., ed.) Oxford University Press, NY (1988); Biocomputing: Informatics and Genome Projects (Smith, D. W., ed.) Academic Press, NY (1994); Computer Analysis of Sequence Data, Part I (Griffin, A. M., and Griffin, H. G., eds.) Humana Press, NJ (1994); Sequence Analysis in Molecular Biology (Von Heijne, G., ed.) Academic Press (1987); and Sequence Analysis Primer (Gribskov, M. and Devereux, J., eds.) Oxford University Press, NY (1992). Preferred methods to determine identity are designed to give the best match between the sequences tested. Methods to determine identity and similarity are codified in publicly available computer programs. Sequence alignments and percent identity calculations may be performed using the Megalign program of the LASERGENE bioinformatics computing suite (DNASTAR, Inc., Madison, Wisconsin). Multiple alignment of the sequences can also be performed using the Clustal method of alignment (Higgins and Sharp CABIOS, 5, 151-153 (1989) with default parameters (GAP PENALTY=10, GAP LENGTH PENALTY=10). Relevant programs also include the GCG suite of programs (Wisconsin Package Version 9.0, Genetics Computer Group (GCG), Madison, Wisconsin); BLASTP, BLASTN, BLASTX (Altschul, et al., J. Mol. Biol. 215:403-410 (1990); DNASTAR (DNASTAR, Inc., Madison, Wisconsin); and the FASTA program incorporating the Smith-Waterman algorithm (Pearson, Comput. Methods Genome Res., [Proc. Int. Symp.] (1994), Meeting Date 1992, 111-20. Editor(s): Suhai, Sandor. Publisher: Plenum, New York, N.Y.. Within the context of this disclosure it will be understood that where sequence analysis software is used for analysis, the results of the analysis are based on the "default values" of the program referenced. As used herein "default values" will mean any set of values or parameters, which originally load with the software when first initialized.

Variants also include nucleic acid molecules that hybridizes under stringent hybridization conditions to a sequence disclosed herein and provide the same function as the reference sequence. Exemplary stringent hybridization conditions include an overnight incubation at 42 °C in a solution including 50% formamide, 5XSSC (750 mM NaCl, 75 mM trisodium citrate), 50 mM sodium phosphate (pH 7.6), 5XDenhardt's solution, 10% dextran sulfate, and 20 µg/ml denatured, sheared salmon sperm DNA, followed by washing the filters in 0.1XSSC at 50 °C. Changes in the stringency of hybridization and signal detection are primarily accomplished through the manipulation of formamide concentration (lower percentages of formamide result in lowered stringency); salt conditions, or temperature. For example, moderately high stringency conditions include an overnight incubation at 37°C in a solution including 6XSSPE (20XSSPE=3M NaCl; 0.2M NaH2PO4; 0.02M EDTA, pH 7.4), 0.5% SDS, 30% formamide, 100 µg/ml salmon sperm blocking DNA; followed by washes at 50 °C with 1XSSPE, 0.1 % SDS. In addition, to achieve even lower stringency, washes performed following stringent hybridization can be done at higher salt concentrations (e.g. 5XSSC). Variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Unless otherwise indicated, the practice of the present disclosure can employ conventional techniques of immunology, molecular biology, microbiology, cell biology and recombinant DNA. These methods are described in the following publications. See, e.g., Sambrook, et al. Molecular Cloning: A Laboratory Manual, 2nd Edition (1989); F. M. Ausubel, et al. eds., Current Protocols in Molecular Biology, (1987); the series Methods IN Enzymology (Academic Press, Inc.); M. MacPherson, et al., PCR: A Practical Approach, IRL Press at Oxford University Press (1991); MacPherson et al., eds. PCR 2: Practical Approach, (1995); Harlow and Lane, eds. Antibodies, A Laboratory Manual, (1988); and R. I. Freshney, ed. Animal Cell Culture (1987).

## Claims

1. A method for identifying the location and type of single cells within a tissue sample, the method comprising:
generating a crosslinked polyacrylamide (PAA) gel comprising 3-20% acrylamide/bis, 0.01-1% ammonium persulfate, and 0.01-1% N,N,N',N'-tetramethylethylenediamine (TEMED);
generating a polony gel by generating a first polony and a second polony on a surface of the crosslinked PAA gel, the first polony comprising a first template, the second polony comprising a second template, the first polony bordering the second polony on the surface of the crosslinked PAA gel;
generating a polony map by sequencing the first template in the first polony and sequencing the second template in the second polony;
capturing, by the first template and the second template, RNA from a cell of a tissue sample, the cell overlapping the first polony and the second polony;
generating cDNA based on the captured RNA, the first template, and the second template;
generating a sequencing library by sequencing the cDNA; and
identifying the location of the cell on the polony gel by comparing the sequencing library to the polony map.

2. The method of claim 1, wherein the crosslinked PAA gel further comprises 1-50 mg/ml N-(5-bromoacetamidylpentyl) acrylamide (BRAPA).

3. The method of any one of claims 1 or 2, wherein generating the polony gel comprises:
attaching a pair of primers to the surface of the crosslinked PAA gel;
attaching a template precursor to the pair of primers, the template precursor comprising an index sequence, a capture sequence, and at least one digestion site;
generating the first polony by generating copies of the template precursor on the surface of the crosslinked PAA gel; and
generating the first template by exposing a copy of the template precursor to an enzyme that specifically binds to the at least one digestion site.

4. The method of any preceding claim, wherein generating the polony map comprises:
identifying a first region on the surface of the polony gel corresponding to a location of the first polony by performing sequencing on the first template;
identifying a second region on the surface of the polony gel corresponding to a location of the second polony by performing sequencing on the second template; and
assigning a first value to first pixels of the polony map corresponding to the first region;
assigning a second value to second pixels of the polony map corresponding to the second region; and
generating metadata associating the first value to a first index sequence in the first template and the second value to a second index sequence in the second template.

5. The method of any preceding claim, wherein generating the cDNA comprises performing reverse transcription of the captured RNA.

6. The method of any preceding claim, wherein identifying the location of the cell on the polony gel comprises:
identifying a first sequence of the cDNA;
determining, by comparing the first sequence to metadata of the polony map, that the first sequence of the cDNA corresponds to a first index sequence of the first template;
identifying a second sequence of the cDNA; and
determining, by comparing the second sequence to metadata of the polony map, that the second sequence of the cDNA corresponds to a second index sequence of the second template.

7. The method of any preceding claim, further comprising:
generating a spatial transcriptome by assigning pixels associated with the first region and the second region to a value corresponding to the cell.

8. A polony gel for single cell RNA sequencing, the polony gel comprising:
a hydrogel comprising crosslinked PAA;
a first polony on a surface of the hydrogel, the first polony comprising a first template that comprises a first capture sequence and a first index sequence; and
a second polony on the surface of the hydrogel, the second polony bordering the first polony and comprising a second template that comprises a second capture sequence and a second index sequence.

9. The polony gel of claim 8, wherein the hydrogel comprises 3-20% acrylamide/bis, 0.01-1% ammonium persulfate, and 0.01-1% N,N,N',N'-tetramethylethylenediamine (TEMED).

10. The polony gel of any one of claims 8 or 9, further comprising:
primers attaching the first template and the second template to the surface of the hydrogel, wherein the hydrogel further comprises 1-50 mg/ml N-(5-bromoacetamidylpentyl) acrylamide (BRAPA).

11. The polony gel of claim 10, wherein the primers comprise a phosphorothioate (PS) modification and/or an acrydite modification.

12. The polony gel of any one of claims 8-11, wherein the first polony comprises 100-1,000,000 copies of the first template; and
wherein the second polony comprises 100-1,000,000 copies of the second template.

13. The polony gel of any one of claims 8-12, wherein the first capture sequence specifically binds to a first RNA sequence; and
wherein the second capture sequence specifically binds to a second RNA sequence.

14. The polony gel of any one of claims 8-13, wherein a width of the first polony is in a range of 0.1-1 µm; and
wherein a width of the second polony is in a range of 0.1-1 µm.

15. The polony gel of any one of claims 8-14, wherein a distance between the first template and the second template is in a range of 0.1-1 µm.

## Patentansprüche

1. Verfahren zur Identifizierung der Position und des Typs einzelner Zellen innerhalb einer Gewebeprobe, umfassend:
Herstellen eines vernetzten Polyacrylamid-(PAA-)Gels, das 3-20 % Acrylamid/Bisacrylamid, 0,01-1 % Ammoniumpersulfat und 0,01-1 % N,N,N',N'-Tetramethylethylendiamin (TEMED) umfasst;
Herstellen eines Polony-Gels durch Erzeugen eines ersten Polonys und eines zweiten Polonys auf einer Oberfläche des vernetzten PAA-Gels, wobei der erste Polony eine erste Matrize umfasst, der zweite Polony eine zweite Matrize umfasst, und der erste Polony an den zweiten Polony angrenzt;
Erzeugen einer Polony-Karte durch Sequenzieren der ersten Matrize im ersten Polony und Sequenzieren der zweiten Matrize im zweiten Polony;
Erfassen - durch die erste und die zweite Matrize - von RNA aus einer Zelle einer Gewebeprobe, wobei die Zelle den ersten und den zweiten Polony überlappt;
Erzeugen von cDNA auf Grundlage der erfassten RNA, der ersten Matrize und der zweiten Matrize;
Erzeugen einer Sequenzierbibliothek durch Sequenzieren der cDNA; und
Bestimmen der Position der Zelle auf dem Polony-Gel durch Vergleichen der Sequenzierbibliothek mit der Polony-Karte.

2. Das Verfahren nach Anspruch 1, wobei das vernetzte PAA-Gel ferner 1-50 mg/ml N-(5-Bromoacetamidylpentyl)acrylamid (BRAPA) umfasst.

3. Das Verfahren nach einem der Ansprüche 1 oder 2, wobei das Herstellen des Polony-Gels Folgendes umfasst:
Anbringen eines Primerpaars an der Oberfläche des vernetzten PAA-Gels;
Ankoppeln eines Matrizen-Vorläufers an das Primerpaar, wobei der Matrizen-Vorläufer eine Indexsequenz, eine Fangsequenz und mindestens eine Spaltstelle umfasst;
Erzeugen des ersten Polonys durch Erzeugen von Kopien des Matrizen-Vorläufers auf der Oberfläche des vernetzten PAA-Gels; und
Erzeugen der ersten Matrize durch Aussetzen einer Kopie des Matrizen-Vorläufers einem Enzym, das spezifisch an die mindestens eine Spaltstelle bindet.

4. Das Verfahren nach einem der vorstehenden Ansprüche, wobei das Erzeugen der Polony-Karte Folgendes umfasst:
Identifizieren einer ersten Region auf der Oberfläche des Polony-Gels, die einer Position des ersten Polonys entspricht, durch Sequenzieren der ersten Matrize;
Identifizieren einer zweiten Region auf der Oberfläche des Polony-Gels, die einer Position des zweiten Polonys entspricht, durch Sequenzieren der zweiten Matrize;
Zuweisen eines ersten Wertes zu ersten Pixeln der Polony-Karte, die der ersten Region entsprechen;
Zuweisen eines zweiten Wertes zu zweiten Pixeln der Polony-Karte, die der zweiten Region entsprechen; und
Erzeugen von Metadaten, die den ersten Wert einer ersten Indexsequenz in der ersten Matrize und den zweiten Wert einer zweiten Indexsequenz in der zweiten Matrize zuordnen.

5. Das Verfahren nach einem der vorstehenden Ansprüche, wobei das Erzeugen der cDNA das Durchführen einer Reverse Transkription der erfassten RNA umfasst.

6. Das Verfahren nach einem der vorstehenden Ansprüche, wobei das Bestimmen der Position der Zelle auf dem Polony-Gel Folgendes umfasst:
Identifizieren einer ersten Sequenz der cDNA;
Bestimmen - durch Vergleichen der ersten Sequenz mit den Metadaten der Polony-Karte -, dass die erste Sequenz der cDNA einer ersten Indexsequenz der ersten Matrize entspricht;
Identifizieren einer zweiten Sequenz der cDNA; und
Bestimmen - durch Vergleichen der zweiten Sequenz mit den Metadaten der Polony-Karte -, dass die zweite Sequenz der cDNA einer zweiten Indexsequenz der zweiten Matrize entspricht.

7. Das Verfahren nach einem der vorstehenden Ansprüche, ferner umfassend: Erzeugen eines räumlichen Transkriptoms, indem Pixel, die mit der ersten Region und der zweiten Region assoziiert sind, einem der Zelle entsprechenden Wert zugeordnet werden.

8. Polony-Gel für die Einzelzell-RNA-Sequenzierung, umfassend:
ein Hydrogel, das vernetztes PAA umfasst;
einen ersten Polony auf einer Oberfläche des Hydrogels, wobei der erste Polony eine erste Matrize umfasst, die eine erste Fangsequenz und eine erste Indexsequenz umfasst; und
einen zweiten Polony auf der Oberfläche des Hydrogels, der an den ersten Polony angrenzt und eine zweite Matrize umfasst, die eine zweite Fangsequenz und eine zweite Indexsequenz umfasst.

9. Das Polony-Gel nach Anspruch 8, wobei das Hydrogel 3-20 % Acrylamid/Bisacrylamid, 0,01-1 % Ammoniumpersulfat und 0,01-1 % N,N,N',N'-Tetramethylethylendiamin (TEMED) umfasst.

10. Das Polony-Gel nach einem der Ansprüche 8 oder 9, ferner umfassend:
Primer, die die erste Matrize und die zweite Matrize an der Oberfläche des Hydrogels fixieren, wobei das Hydrogel ferner 1-50 mg/ml N-(5-Bromoacetamidylpentyl)acrylamid (BRAPA) umfasst.

11. Das Polony-Gel nach Anspruch 10, wobei die Primer eine Phosphorothioat- (PS-) Modifikation und/oder eine Acrydite-Modifikation umfassen.

12. Das Polony-Gel nach einem der Ansprüche 8-11, wobei der erste Polony 100-1.000.000 Kopien der ersten Matrize umfasst; und
wobei der zweite Polony 100-1.000.000 Kopien der zweiten Matrize umfasst.

13. Das Polony-Gel nach einem der Ansprüche 8-12, wobei die erste Fangsequenz spezifisch an eine erste RNA-Sequenz bindet; und
wobei die zweite Fangsequenz spezifisch an eine zweite RNA-Sequenz bindet.

14. Das Polony-Gel nach einem der Ansprüche 8-13, wobei eine Breite des ersten Polonys im Bereich von 0,1-1 µm liegt; und
wobei eine Breite des zweiten Polonys im Bereich von 0,1-1 µm liegt.

15. Das Polony-Gel nach einem der Ansprüche 8-14, wobei ein Abstand zwischen der ersten Matrize und der zweiten Matrize im Bereich von 0,1-1 µm liegt.

## Revendications

1. Procédé d'identification de l'emplacement et du type de cellules uniques au sein d'un échantillon tissulaire, le procédé comprenant :
générer un gel de polyacrylamide (PAA) réticulé comprenant de 3 à 20 % d'acrylamide/bisacrylamide, de 0,01 à 1 % de persulfate d'ammonium, et de 0,01 à 1 % de N,N,N',N'-tétraméthyléthylènediamine (TEMED) ;
générer un gel de polonies en générant un premier polony et un second polony à la surface du gel de PAA réticulé, le premier polony comprenant une première matrice, le second polony comprenant une seconde matrice, le premier polony étant contigu au second polony à ladite surface ;
générer une carte de polonies en séquençant la première matrice dans le premier polony et en séquençant la seconde matrice dans le second polony ;
capturer, par la première matrice et la seconde matrice, de l'ARN provenant d'une cellule d'un échantillon tissulaire, ladite cellule chevauchant le premier polony et le second polony ;
générer un ADNc à partir de l'ARN capturé, de la première matrice et de la seconde matrice ;
générer une bibliothèque de séquençage en séquençant l'ADNc ; et
identifier l'emplacement de la cellule sur le gel de polonies en comparant la bibliothèque de séquençage à la carte de polonies.

2. Le procédé selon la revendication 1, dans lequel le gel de PAA réticulé comprend en outre de 1 à 50 mg/ml de N-(5-bromoacétamidylpentyl) acrylamide (BRAPA).

3. Le procédé selon l'une quelconque des revendications 1 ou 2, dans lequel la génération du gel de polonies comprend :
fixer une paire d'amorces à la surface du gel de PAA réticulé ;
fixer un précurseur de matrice à ladite paire d'amorces, ledit précurseur de matrice comprenant une séquence d'index, une séquence de capture et au moins un site de digestion ;
générer le premier polony en générant des copies du précurseur de matrice à la surface du gel de PAA réticulé ; et
générer la première matrice en exposant une copie du précurseur de matrice à une enzyme qui se lie spécifiquement au(x) site(s) de digestion.

4. Le procédé selon l'une quelconque des revendications précédentes, dans lequel la génération de la carte de polonies comprend :
identifier une première région à la surface du gel de polonies correspondant à l'emplacement du premier polony en réalisant le séquençage de la première matrice ;
identifier une seconde région à la surface du gel de polonies correspondant à l'emplacement du second polony en réalisant le séquençage de la seconde matrice ;
attribuer une première valeur à des premiers pixels de la carte de polonies correspondant à la première région ;
attribuer une seconde valeur à des seconds pixels de la carte de polonies correspondant à la seconde région ; et
générer des métadonnées associant la première valeur à une première séquence d'index dans la première matrice et la seconde valeur à une seconde séquence d'index dans la seconde matrice.

5. Le procédé selon l'une quelconque des revendications précédentes, dans lequel la génération de l'ADNc comprend l'exécution d'une transcription inverse de l'ARN capturé.

6. Le procédé selon l'une quelconque des revendications précédentes, dans lequel l'identification de l'emplacement de la cellule sur le gel de polonies comprend :
identifier une première séquence de l'ADNc ;
déterminer, en comparant la première séquence aux métadonnées de la carte de polonies,
que la première séquence de l'ADNc correspond à une première séquence d'index de la première matrice ;
identifier une seconde séquence de l'ADNc ; et
déterminer, en comparant la seconde séquence aux métadonnées de la carte de polonies, que la seconde séquence de l'ADNc correspond à une seconde séquence d'index de la seconde matrice.

7. Le procédé selon l'une quelconque des revendications précédentes, comprenant en outre :
générer un transcriptome spatial en attribuant aux pixels associés à la première région et à la seconde région une valeur correspondant à la cellule.

8. Gel de polonies pour le séquençage d'ARN de cellules uniques, le gel de polonies comprenant :
un hydrogel comprenant un PAA réticulé ;
un premier polony à la surface de l'hydrogel, le premier polony comprenant une première matrice qui comprend une première séquence de capture et une première séquence d'index ; et
un second polony à ladite surface, contigu au premier polony, comprenant une seconde matrice qui comprend une seconde séquence de capture et une seconde séquence d'index.

9. Le gel de polonies selon la revendication 8, dans lequel l'hydrogel comprend de 3 à 20 % d'acrylamide/bisacrylamide, de 0,01 à 1 % de persulfate d'ammonium et de 0,01 à 1 % de N,N,N',N'-tétraméthyléthylènediamine (TEMED).

10. Le gel de polonies selon l'une quelconque des revendications 8 ou 9, comprenant en outre :
des amorces fixant la première matrice et la seconde matrice à la surface de l'hydrogel,
l'hydrogel comprenant en outre de 1 à 50 mg/ml de N-(5-bromoacétamidylpentyl) acrylamide (BRAPA).

11. Le gel de polonies selon la revendication 10, dans lequel les amorces comprennent une modification phosphorothioate (PS) et/ou une modification acrydite.

12. Le gel de polonies selon l'une quelconque des revendications 8 à 11, dans lequel le premier polony comprend de 100 à 1 000 000 copies de la première matrice ; et
dans lequel le second polony comprend de 100 à 1 000 000 copies de la seconde matrice.

13. Le gel de polonies selon l'une quelconque des revendications 8 à 12, dans lequel la première séquence de capture se lie spécifiquement à une première séquence d'ARN ; et dans lequel la seconde séquence de capture se lie spécifiquement à une seconde séquence d'ARN.

14. Le gel de polonies selon l'une quelconque des revendications 8 à 13, dans lequel une largeur du premier polony est comprise dans une plage de 0,1 à 1 µm ; et
dans lequel une largeur du second polony est comprise dans une plage de 0,1 à 1 µm.

15. Le gel de polonies selon l'une quelconque des revendications 8 à 14, dans lequel une distance entre la première matrice et la seconde matrice est comprise dans une plage de 0,1 à 1 µm.
